# EUROPEAN PATENT SPECIFICATION

(11) **EP 2 747 763 B1**
(45) Date of publication and mention of the grant of the patent: **30.12.2020**
(21) Application number: 12762405.4
(22) Date of filing: 23.08.2012
(51) Int. Cl.: A61K 31/555, A61K 31/519, A61P 27/00, A61P 41/00

(54) **(BACTERIO)CHLOROPHYLL PHOTOSENSITIZERS FOR TREATMENT OF EYE DISEASES AND DISORDERS**
(BAKTERIO-)CHLOROPHYLL-PHOTOSENSIBILISATOREN ZUR BEHANDLUNG VON AUGENERKRANKUNGEN UND -LEIDEN
PHOTOSENSIBILISATEURS (BACTÉRIO)CHLOROPHYLLIENS DESTINÉS AU TRAITEMENT DE TROUBLES ET DE MALADIES OCULAIRES

(30) Priority: 23.08.2011 US 201161526414 P
(43) Date of publication of application: 02.07.2014
(73) Proprietor: Yeda Research and Development Co. Ltd., 76100 Rehovot (IL)
(72) Inventor: SCHERZ, Avigdor, 76329 Rechovot (IL); SALOMON, Yoram, 76287 Rehovot (IL); MARCOVICH, Arie, 76100 Rehovot (IL); BRANDIS, Alexander, 76100 Rehovot (IL); WAGNER, Daniel, 76100 Rehovot (IL)
(74) Representative: Kalhammer, Georg
(86) International application number: PCT/IL2012/050325
(87) International publication number: WO 2013/027222

(56) References cited:
- WO-A2-03/028628
- WO-A2-2005/120573
- CIULLA THOMAS A ET AL: "MV-6401, a potent photosensitizer in experimental animal models: a review of this agent and the current state of photosensitizing agents for the treatment of exudative age-related macular degeneration", DRUGS OF THE FUTURE, vol. 30, no. 10, October 2005 (2005-10), pages 1031-1037, XP002686645, ISSN: 0377-8282
- YOON ET AL: "Photodynamic Therapy with Verteporfin for Corneal Neovascularization", AMERICAN JOURNAL OF OPHTHALMOLOGY, OPHTHALMIC PUBL, CHICAGO, IL, US, vol. 144, no. 3, 29 August 2007 (2007-08-29), pages 390-395.e1, XP022219333, ISSN: 0002-9394, DOI: 10.1016/J.AJO.2007.05.028
- GOHTO YUKO ET AL: "Photodynamic effect of a new photosensitizer ATx-S10 on corneal neovascularization", EXPERIMENTAL EYE RESEARCH, vol. 67, no. 3, September 1998 (1998-09), pages 313-322, XP002686646, ISSN: 0014-4835
- TENTEN VAN Y ET AL: "A PRELIMINARY STUDY ON THE PREVENTION OF POSTERIOR CAPSULE OPACIFICATION BY PHOTODYNAMIC THERAPY WITH BACTERIOCHLORIN A IN RABBITS", OPHTHALMIC RESEARCH, S. KARSER AG. BASEL, CH, vol. 34, no. 3, 1 May 2002 (2002-05-01), pages 113-118, XP008030724, ISSN: 0030-3747, DOI: 10.1159/000063653

## Description

### TECHNICAL FIELD

The present invention is in the fields of ophthalmology and photodynamic therapy (PDT) and relates to photodynamic therapy of diseases, disorders and conditions associated with corneal or scleral anomaly, using photosensitizers, water-soluble particularly chlorophyll and bacteriochlorophyll compounds.

### DEFINITIONS AND ABBREVIATIONS

**Bchl a:** bacteriochlorophyll a: pentacyclic 7,8,17,18-tetrahydroporphyrin with a 5^{th} isocyclic ring, a central Mg atom, a phytyl or geranylgeranyl group at position 17³, a COOCH₃ group at position 13², an H atom at position 13², methyl groups at positions 2, 7, 12, 18, an acetyl group at position 3, and an ethyl group at position 8, herein compound **1**; **Bphe:** bacteriopheophytin a (Bchl in which the central Mg is replaced by two H atoms); **Bpheid:** bacteriopheophorbide a (the C-17²-free carboxylic acid derived from Bphe without the central metal atom); **Chl:** chlorophyll; **Rhodobacteriochlorin:** tetracyclic 7,8,17,18-tetrahydroporphyrin having a -CH₂CH₂COOH group at position 17, a -COOH at position 13, methyl groups at positions 2, 7, 12, 18, ethyl group at position 8 and vivyl as position 3; **Pd-Bpheid:** Pd-bacteriopheophorbide a; **WST11:** palladium 3¹-oxo-15-methoxycarbonyl methyl-rhodobacteriochlorin 13¹-(2-sulfoethyl) amide dipotassium salt; **ROS:** reactive oxygen species; **NIR:** near infrared; **RF:** riboflavin.

IUPAC numbering of the bacteriochlorophyll derivatives is used throughout the specification.

### BACKGROUND ART

Treatment with riboflavin (RF) followed by ultraviolet A (UVA, 370 nm) illumination, results in cornea stiffening and sclera stiffening presumably because of collagen crosslinking (CXL). This has increasingly been used for halting the progression of keratoconus and post refractive laser surgery corneal ectasia (Wollensak et al., 2003(a); Hafezi et al., 2007; Raiskup-Wolf et al., 2008). However, there are several drawbacks to this treatment: (1) The prolonged time of RF treatment (30 min); (2) The prolonged eye exposure to UVA irradiation (30 min), and finally (3) Toxicity to keratocytes (Wollensak et al., 2004(a); Wollensak et al., 2004(b); Wollensak, 2010(a)) and corneal endothelial cells (Wollensak et al., 2003(c); Spoerl et al. 2007), making treatment of corneas thinner than 400 microns problematic (Hafezi et al., 2009; Wollensak, 2010(b)). Hence, there is a need for a safer treatment that can stiffen the cornea with a lesser risk to the patient (Avila and Navia, 2010; WO/2008/052081). One possibility is to use photosensitizers that inflict cornea stiffening upon illumination at the near infra red (NIR) using bacteriochlorophyll derivatives as photosensitizers.

Myopia, also termed nearsightedness is a refractive defect of the eye in which collimated light produces the image focus in front of the retina when accommodation is relaxed. The global prevalence of myopia has been estimated from 800 million to 2.3 billion. In some countries, such as China, India and Malaysia, up to 41% of the adult population is myopic to -1dpt and 80% to - 0.5dpt. Myopia has been related with stretching of the collagenous sclera. Elongation of the globe occurs in the posterior segment of the globe and involves the sclera. Such globe elongation causes myopic progression in predisposed myopic children and adolescents. It usually slows down and stops during the third decade of life, when maturation of body tissues occurs with natural stiffening. This stiffening is related to glycation mediated cross linking.

At present, there is no effective treatment to stop myopic progression and reduce visual loss caused by degenerative myopia. Surgical solutions to arrest myopic progression by applying reinforcement belts around the eye, and suturing them to the sclera, were reported. These surgical solutions were controversial and technically challenging, and did not gain popularity. The critical age of intervention is during childhood or early adolescence. Thus, a simpler approach to stiffen the sclera should be applied.

Since progression of myopia is associated with elongation of the posterior segment of the eye and subsequent stretching of the sclera and chorioretinal tissues, stiffening of the sclera by collagen crosslinking, is expected to retard/stop the progression of the disease and related disorders such as macular stretching and atrophy or bleeding and visual loss. Wollensak and Spoerl reported the use of RF/UVA treatment to achieve such crosslinking and strengthening in human and porcine sclera in vitro. The crosslinking stiffening was demonstrated in vivo on rabbits, and was shown to last several months. This treatment can be applied to arrest myopic progression.

However, such treatment is subjected to the UVA risks which might be hazardous. In addition, the tissue penetration of UV radiation is limited. Illumination of the sclera with UV requires external approach and necessitates surgical exposure. There is therefore, a need for alternative photosensitizers that can induce collagen crosslinking with a safer and better penetrating wavelength at the red of near infrared (NIR).

A non hazardous light with deeper tissue penetration, like NIR in bacteriochlorophyll (BChl) based PDT has been shown to provide efficient and safe anti cancer treatments in oncology and age related macular degeneration in the eye (AMD) (US 7,947,672, WO 2005/120573).

Application of novel water soluble chlorophyll (Chl) and bacteriochlorophyll (BChl) derivatives as sensitizers in PDT has been reported by the present inventors in recent years (US 7,947,672; WO 2005/120573; Ashur et al. 2009; Mazor et al. 2005; Brandis et al., 2005) and by others (Moore et al., 2009; Bourges et al., 2006; Berdugo et al. 2008). Upon NIR illumination these water soluble derivatives generate O₂⁻ and ●OH radicals (Ashur et al. 2009; Mazor et al. 2005; Brandis et al., 2005; Vakrat-Haglili et al., 2005) and have been used so far in vascular-targeted photodynamic therapy (VTP) of cancers in preclinical (Mazor et al. 2005) and advanced clinical trials of prostate cancer therapy (currently in Phase III) (Trachtenberg J et al. 2007; Lepor H. 2008; Moore et al., 2009), following i.v. administration to the treated patients. The effective generation of oxygen radicals as precursors of protein crosslinking (Liu et al., 2004), and the clinical experience established with water soluble Bchls and Chls derivatives, maks these sensitizers potential candidates for application in therapy that is mediated by collagen crosslinking, particularly corneal and scleral stiffening upon NIR illumination after topical application.

In WO 2003/028628 chlorophyll or bacteriochlorophyll compounds for treating corneal vascularization are described. In American Journal of Ophthalmology, 2007, 144, 390-395, Yoon et al. refer to Verteporfin for treating corneal vasculazation, and Gohto et al. disclose that the chlorin derivative ATX-S10 can be used for treating corneal vascularization (see Experimental Eye Research, 1998, 67, 313-322).

In the scientific article published in Drugs of the Future, 2005, 30, 1031-1037, Ciulla et al. refer to chlorophyll or bacteriochlorophyll compounds, which are used for age-related macular degeneration. The use of bacteriochlorin A for treating capsule opacification is described in van Tenten et al., Ophthalmic Research, 2002, 34, 113-118.

### SUMMARY OF THE INVENTION

It has now been found in accordance with the present invention that the water soluble (bacterio)chlorophyll derivatives described in US 7,947,672 and WO 2005/120573 enhance, upon NIR illumination, stiffening of the cornea and sclera of rabbit eye after topical application. Exemplifying results are disclosed herein for *ex vivo* and *in vivo* treatment of rabbit eyes with certain water soluble sulfonated bacteriochlorophyll derivatives. Treatment of corneas and sclera of rabbit eyes with these photosensitizers appeared safe and increased significantly the biomechanical strength of the cornea and the sclera.

The present invention thus relates to a chlorophyll or bacteriochlorophyll derivative or a pharmaceutically acceptable salt thereof for use in treating diseases, disorders and conditions associated with corneal or scleral anomaly selected from corneal thinning or scleral weakening by photodynamic therapy (PDT), wherein the chlorophyll or bacteriochlorophyll derivative has the formula II as defined in the claims.

In a main aspect, the present invention provides ophthalmic compositions comprising a pharmaceutically acceptable carrier and a chlorophyll or bacteriochlorophyll derivative as defined in the claims for use in treating diseases, disorders and conditions associated with corneal or scleral anomaly selected from corneal thinning and scleral weakening by photodynamic therapy (PDT).

The disorders associated with thinning of the cornea include keratoconus, raised intraocular pressure and corneal ectasia caused by trauma, and disorders associated with eye globe elongation include myopia and macular stretching.

### BRIEF DESCRIPTION OF DRAWINGS

**Fig. 1** is a photograph of a corneal strip clamped at a distance of 6 mm between the jaws of a microcomputer-controlled biomaterial tester.
**Fig. 2** is a photograph of a central corneal disc, 8 mm in diameter, mounted on a glass slide that was placed obliquely in a quartz cuvette for fluorescence spectroscopy measurements after WST11/NIR and RF/UVA treatment.
**Fig. 3** are optical absorption spectra (755 nm) of WST11 accumulated in de-epithelialized corneas of rabbit exposed to a solution of WST11 in saline for 10, 20 and 30 minutes.
**Figs. 4A-4H** are fluorescence microscope pictures (**Figs. 4A, 4B****,** **4E, 4F**) and graphs related thereto (**Figs. 4C, 4D****,** **4G, 4H**), showing fluorescence intensity versus penetration depth into de-epithelialized rabbit corneas that were exposed *ex-vivo* to WST11 in saline for 10 minutes (**4A** and **4C**) and 30 minutes (**4B** and **4D**), and to a solution of WST11 and dextran-500 for 10 minutes (**4E** and **4G**), and 30 minutes (**4F** and **4H**). Fluorescence of central corneal sagittal slices (12 µm) was detected at 760 nm upon excitation at 740 nm.
**Fig. 5** are optical absorption spectra of WST11 from corneas *ex-vivo* treated with WST11 solution for 20 minutes prior to illumonation at 755 nm, 10 mW/cm², for the specified time durations.
**Fig. 6** is an electron spin resonance (ESR) spectrum of α-(4-pyridyl *N*-oxide)-*N-tert-*butylnitrone (4-POBN, 65mM) in solution containing ethanol (8%) and WST11 (black) or RF (light blue), following NIR or UVA irradiation, respectively. The black arrows shows the quartet formed upon trapping sinlget oxygen by 4-POBN, and the stars shows the double-triplet formed upon trapping hydroxyl and superoxide radicals.
**Fig. 7** are ESR spectra of 4-POBN obtained form *ex-vivo* WST11/NIR (black) or RF/UVA (light blue) treated corneas, following immerssion in a 4-POBN (65 mM) ethanol- (8%) solution.
**Figs. 8A-8B** are graphs presenting ex vivo stress-strain measurements values of corneal stiffness in ultimate stress units (**8A**) and Young's modulus (**8B**) of corneas after 30-min. incubation with WST11 followed by 30-min. NIR illumination (n=10), or incubation with RF-D solution followed by UVA irradiation (RF-D/UVA).
**Figs. 9A-9B** are graphs presenting *in vivo* stress-strain measurements values of corneal stiffness in ultimate stress units (**9A**) and Young's modulus (**9B**), 1 month after treating rabbit corneas with WST11 (2.5 mg/ml) for 10, 20, or 30 minutes, followed by 30-min NIR illumination (755 nm, 10 mW/cm²), or after RF/UVA treatment.
**Figs. 10A-10B** are graphs presenting *in vivo* stress-strain measurements values of corneal stiffness in ultimate stress units (**10A**) and Young's modulus (**10B**), 1 month after treating rabbit corneas either with WST11 (2.5 mg/ml) is saline without dextran (WST11), or WSTllwith dextran 500 (WST-D) for 20 minutes, followed by 30-min NIR illumination (755 nm, 10 mW/cm²), or after treatment with RF with or without dextran, followed by 30-min UVA irradiation. Control-untreated eyes.
**Figs. 11A-11C** are histological sections of rabbit corneas stained with hematoxylin-eosin two days (**11A-11C**) or 1 week (**11D**) post *in vivo* WST11/NIR illumonation treament (×20 magnification). **d11A:** control; **11B:** cornea treated with WST11 in saline (WST11-S/NIR protocol); **11C:** cornea treated with WST11 and dextran (WST11-D/NIR); **11D:** 1 week afetr WST11-D/NIR treatment.
**Figs. 12A-12B** are histological sections of rabbit corneas stained for apoptosis 1 day after *in vivo* treament with WST11 in saline followed by NIR illumination. **12A-** Control, untreated cornes; **12B** WST11-S/NIR treated cornea.
**Fig. 13** presents fluorescence spectra of rabbit corneas treated with WST11 and NIR irradiation (WST11/NIR), or treated with riboflavin and UVA irradiation (RF/UVA).
**Fig. 14** is a fluorescence microscope picture of rabbit sclera showing penetration of WST11.
**Figs. 15A-15B** are graphs presenting ex vivo stress-strain measurements values of upper equatorial sclera stiffness in ultimate stress units (**15A**) and Young's modulus (**15B**) of corneas after 30-min incubation with WST11 followed by 30-min direct NIR illumination (755 nm, 10 mW/cm²) onto the posterior treated sclera. Control - untreated lower equatorial sclera.
**Figs. 16A-16B** are graphs presenting ex vivo stress-strain measurements values of upper equatorial sclera stiffness in ultimate stress units (**15A**) and Young's modulus (**15B**) of corneas after 20-min incubation with WST11 followed by 30-min NIR illumination (755 nm, 10 mW/cm²) through the anterior cornea. Control - untreated lower equatorial sclera.
**Figs. 17A-17B** are schematic illustration of the apparatus used for ex-vivo drug delivery to the sclera and external or direct illumination (**17A**) and illumination through the anterior cornea (**17B**).
**Figs. 18A-18C** are photographs of the three mirror fundus lens apparatus used for NIR illumination of the posterior treated sclera of a rabbit eye by illuminating the anterior cornea.

### MODES FOR CARRYING OUT THE INVENTION

It is has been found by the present inventors that certain water soluble chlorophyll and bacteriochlorophyll derivatives penetrate sclera and de-epithelialized cornea fairly fast and in a time dependent manner, and upon sensitization by the appropriate irradiation induce consistent and significant stiffening of the cornea and the sclera both *ex vivo* and *in vivo.* The fact that these photosensitizers were photochemically active was reflected in their continuous bleaching and spectra modification into their oxidized form during illumination.

Thus, it is a main object of the present invention to provide pharmaceutical compositions comprising a chlorophyll or bacteriochlorophyll photosensitizer for use in minimally invasive photodynamic therapy (PDT) of diseases, disorders and conditions associated with corneal or scleral anomaly. Particularly, the pharmaceutical composition provided by the invention is for treatment of corneal thinning and/or sclera stretching.

The photosensitizer useful for the purpose of the invention is a water soluble (bacterio)chlorophyll derivative of the formula II: 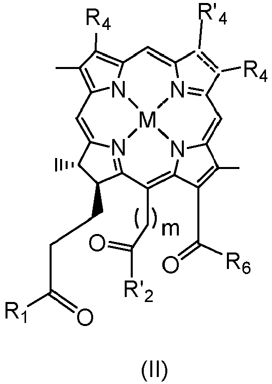 wherein
M represents 2H or an atom selected from the group consisting of Mg, Pd, Pt, Zn, In, Gd and Yb;
R₁, R'₂ and R₆ each independently is Y-R₈, -NR₉R'₉ or -N⁺R₉R'₉R"₉A⁻;
Y is O or S;
R₄ is -CH=CR₉R'₉, -CH=CR₉Hal, -CH=CH-CH₂-NR₉R'₉, -CH=CH-CH₂- N⁺R₉R'₉R"₉ A⁻, -CHO, -CH=NR₉, -CH=N⁺R₉R'₉A⁻, -CH₂-OR₉, -CH₂-SR₉, -CH₂-Hal, -CH₂-R₉, -CH₂-NR₉R'₉, -CH₂-N⁺R₉R'₉R"₉A⁻, -CH₂-CH₂R₉, -CH₂-CH₂Hal, -CH₂-CH₂OR₉, -CH₂-CH₂SR₉, -CH₂-CH₂-NR₉R'₉, -CH₂-CH₂-N⁺R₉R'₉R"₉A⁻, -COCH₃, C(CH₃)=CR₉R'₉, -C(CH₃)=CR₉Hal, -C(CH₃)=NR₉, -CH(CH₃)=N⁺R₉R'₉A⁻, -CH(CH₃)-Hal, -CH(CH₃)-OR₉, -CH(CH₃)-SR₉, -CH(CH₃)-NR₉R'₉, - CH(CH₃)-N⁺R₉R'₉R"₉A⁻, or -C≡CR₉;
R'₄ is methyl or formyl;
R₈, R₉, R'₉ and R"₉ each independently is:
   (a) H;
   (b) C₁-C₂₅ hydrocarbyl;
   (c) C₁-C₂₅ hydrocarbyl substituted by one or more functional groups selected from the group consisting of halogen, nitro, oxo, OR, SR, epoxy, epithio, -CONRR', -COR, COOR", - OSO₃R, -SO₃R", -SO₂R, -NHSO₂R, - SO₂NRR', -NRR', =N-OR, =N-NRR', -C(=NR)-NRR', - NR-NRR', -(R)N-C(=NR)-NRR', O←NR-, >C=NR, -(CH₂)ₙ-NR-COR', -(CH₂)ₙ-CO-NRR', -O-(CH₂)ₙ-OR, -O-(CH₂)ₙ-O-(CH₂)ₙ-R, -PRR', -OPO₃RR', -PO₂HR and -PO₃R"R", wherein n is an integer of 1 to 10 and R and R' each independently is H, hydrocarbyl or heterocyclyl, or R and R' together with the N atom to which they are attached form a 3-7 membered saturated ring optionally containing a further heteroatom selected from O, S and N, wherein the further N atom may be substituted, and R" is H, a cation, hydrocarbyl or heterocyclyl;
   (d) C₁-C₂₅ hydrocarbyl substituted by one or more functional groups selected from the group consisting of positively charged groups, negatively charged groups, basic groups that are converted to positively charged groups under physiological conditions, and acidic groups that are converted to negatively charged groups under physiological conditions;
   (e) C₁-C₂₅ hydrocarbyl containing one or more heteroatoms and/or one or more carbocyclic or heterocyclic moieties;
   (f) C₁-C₂₅ hydrocarbyl containing one or more heteroatoms and/or one or more carbocyclic or heterocyclic moieties and substituted by one or more functional groups as defined in (c) and (d) above;
   (g) C₁-C₂₅ hydrocarbyl substituted by a residue of an amino acid, a peptide, a protein, a monosaccharide, an oligosaccharide, or a polysaccharide; or
   (h) a residue of an amino acid, a peptide, a protein, a monosaccharide, an oligosaccharide, or a polysaccharide;
Rs may further be H⁺ or a cation R⁺₁₀ when R₁, R'₂ and R₆ each independently is Y-R₈;
R⁺₁₀ is a metal cation, an ammonium group or an organic cation;
A is a physiologically acceptable anion;
m is 0 or 1; and
the dotted line at positions 7-8 represents an optional double bond; and
pharmaceutically acceptable salts.

In certain embodiments, the dotted line at positions 7-8 represents a double bond and the photosensitizer is a chlorophyll of the formula II.

The tetracyclic compound of formula II (three pyrroles and one pyrroline coupled through four methine linkages) wherein M is absent, R₁ at position 17³ is a propionic acid, R'₂ at position 15 is -CH₂COOH, R₆ at position 13² is -COOH, R₄ at position 3 is vinyl, the dotted line at positions 7-8 represents a double bond, R'₄ is methyl at position 7 and R₄ is ethyl at position 8, is chlorin, and its derivatives will have different metal atoms and/or different substituents R₁, R'₂, R₄, R'₄ and/or R₆.

In certain other embodiments, the positions 7-8 are hydrogenated and the photosensitizer is a bacteriochlorophyll of the formula II.

The tetracyclic (two pyrroles and two pyrroline coupled through four methine linkages) compounds of formula II wherein M is absent, methyl groups at positions 2, 7, 12, 18, R₁ at position 17³ is propionic acid, R'₂ at position 13 is COOCH₃, R₄ at position 3 is acetyl and at position 8 is ethyl, and the dotted line at positions 7-8 is absent, is bacteriochlorin a. When a vinyl group is attached at positions 3, the compound is rhodobacteriochlorins ((3¹-vinyl)-bacteriochlorin a). Derivatives of bacteriochlorin a and rhodobacteriochlorin will have different metal atom and/or different substituents R₁, R'₂, R₄ at position 3 and/or R₆.

As used herein, the term "hydrocarbyl" means any straight or branched, saturated or unsaturated, acyclic or cyclic, including aromatic, hydrocarbyl radicals, of 1-25 carbon atoms, preferably of 1 to 20 or 1 to 10, more preferably 1 to 6, most preferably 2-3 carbon atoms. The hydrocarbyl may be a lower alkyl radical of 1-6, preferably of 1-4 carbon atoms, e.g. methyl, ethyl, propyl, isopropyl, butyl, isobutyl, tert-butyl, or alkenyl, alkynyl or cycloalkyl, or at the position 17 of the compounds of formula II, the hydrocarbyl is a radical derived from natural Chl and Bchl compounds, e.g. geranylgeranyl (2,6-dimethyl-2,6-octadienyl) or phytyl (2,6,10,14-tetramethyl-hexadec-14-en-16-yl).

In one embodiment, the alkyl group has 10 carbon atoms or more, e.g. -C₁₀H₂₁, -C₁₅H₃₁, -C₁₆H₃₃, -C₁₇H₃₅, -C₁₈H₃₇, -C₂₀H₄₁, and the like.

In another embodiment, the C₁-C₂₅ hydrocarbyl is a straight or branched C₂-C₂₅ alkenyl or alkynyl radical, preferably of 2-6 carbon atoms, e.g. vinyl, prop-2-en-1-yl, but-3-en-1-yl, pent-4-en-1-yl, hex-5-en-1-yl, ethynyl, propargyl, and the like.

In yet another embodiment, the C₁-C₂₅ hydrocarbyl is a C₃-C₂₅ monocyclic or polycyclic cycloalkyl or partially unsaturated cycloalkyl, preferably C₃-C₁₄, more preferably C₃-C₇ cycloalkyl, such as cyclopropyl, cyclobutyl, cyclopentyl, cyclohexyl and cycloheptyl.

The hydrocarbyl may further be aryl or aralkyl, wherein the term "aryl" as used herein refers to a "C₆-C₁₄" aromatic carbocyclic group having 6 to 14 carbon atoms, preferably 6 to 10 carbon atoms, consisting of a single, bicyclic or tricyclyc ring system, such as phenyl, naphthyl, carbazolyl, anthryl, phenanthryl and the like, and the term "aralkyl" refers to a radical derived from an arylalkyl compound wherein the aryl moiety is preferably a C₆-C₁₄, more preferably a C₆-C₁₀ aryl such as benzyl, phenanthryl and the like.

The term "heterocyclic ring" or "heterocyclyl" means a radical derived from a saturated, partially unsaturated, optionally substituted, monocyclic, bicyclic or tricyclic heterocycle of 3-12, preferably 5-10, more preferably 5-6 members in the ring containing 1 to 3 heteroatoms selected from O, S and/or N. Particular examples are dihydrofuryl, tetrahydrofuryl, pyrrolynyl, pyrrolydinyl, dihydrothienyl, dihydropyridyl, piperidinyl, quinolinyl, piperazinyl, morpholino or 1,3-dioxanyl.

The terms "heteroaryl" or "heteroaromatic moiety" refer to a mono- or polycyclic heteroaromatic ring that may comprise both carbocyclic and heterocyclic rings, containing 1 to 3 heteroatoms selected from O, S and/or N and optionally substituted. Particular examples are, without being limited to, pyrrolyl, furyl, thienyl, pyrazolyl, imidazolyl, oxazolyl, thiazolyl, pyridyl, quinolinyl, isoquinolinyl, pyridazinyl, pyrimidinyl, pyrazinyl, 1,3,4-triazinyl, 1,2,3-triazinyl, 1,3,5-triazinyl, benzofuryl, isobenzofuryl, indolyl, imidazo[1,2-a]pyridyl, benzimidazolyl, benzthiazolyl, benzoxazolyl, benzodiazepinyl, and other radicals derived from further polycyclic heteroaromatic rings.

Any "carbocyclyl", "heterocyclyl", "aryl" or "heteroaryl" may be substituted by one or more radicals such as halogen, C₆-C₁₄ aryl, C₁-C₂₅ alkyl, nitro, OR, SR, -COR, -COOR, COOR", -SO₃R, -SO₃R", -SO₂R, -NHSO₂R, -NRR', -(CH₂)ₙ-NR-COR', and -(CH₂)ₙ-CO-NRR', wherein n, R, R' and R" are as defined above. It is to be understood that when a polycyclic heteroaromatic ring is substituted, the substitutions may be in any of the carbocyclic and/or heterocyclic rings.

The term "alkoxy" as used herein refers to a group (C₁-C₂₅)alkyl-O-, wherein C₁-C₂₅ alkyl is as defined above. Examples of alkoxy are methoxy, ethoxy, n-propoxy, isopropoxy, butoxy, isobutoxy, tert-butoxy, pentoxy, hexoxy, -OC₁₂H₂₅, -OC₁₅H₃₁, -OC₁₆H₃₃, -OC₁₇H₃₅, -OC₁₈H₃₇, and the like. The term "aryloxy" as used herein refers to a group (C₆-C₁₈)aryl-O-, wherein C₆-C₁₈ aryl is as defined above, for example, phenoxy and naphthoxy.

The term "halogen", as used herein, refers to fluoro, chloro, bromo or iodo.

The hydrocarbon chain of Rs, R₉, R'₉ and/or R"₉ may optionally contain one or more heteroatoms such as O, S and/or NH, and/or one or more carbocyclic rings or heterocyclic ring moieties, wherein "carbocyclic" as used herein encompasses cycloalkyl and aryl as defined herein.. In one embodiment, the hydrocarbyl chain contains one or more O atoms and has a OH end group as represented by an oligooxyethyleneglycol residue of 4 to 10 carbon atoms, preferably pentaoxyethyleneglycol. In other embodiments, the hydrocarbyl contains phenyl or pyridyl.

Rs, R₉, R'₉ and/or R"₉ may also be hydrocarbyl substituted by one or more functional groups such as halogen, for example Cl, Br, F or I, nitro, oxo, alkoxy (OR), SR, epoxy, epithio, - CONRR', -COR, COOR", COSR, -OSO₃R, -SO₃R", -SO₂R, -NHSO₂R, -SO₂NRR' -NRR', =NOR, =N-NRR', -C(=NR)-NRR', -NR-NRR', -(R)N-C(=NR)-NRR', O←NR-, >C=NR, -(CH₂)ₙ-NR-COR', -(CH₂)ₙ-CO-NRR', -O-(CH₂)ₙ-OR, -O-(CH₂)ₙ-O-(CH₂)ₙ-R, -PRR', -OPO₃RR', - PO₂HR and -PO₃R"R", wherein n is an integer of 1 to 10 and R and R' each independently is H, hydrocarbyl, or heterocyclyl, or R and R' together with the N atom to which they are attached form a 3-7 membered saturated ring, optionally containing one or more heteroatoms selected from the group consisting of O, S or N, and optionally further substituted at the additional N atom, and and R" is H, a cation, hydrocarbyl, or heterocyclyl.

In certain embodiments, some of the functional groups above are acidic groups that may convert to negatively charged groups under physiological pH, for example COOH, COSH, SO₃H, PO₃H₂, or the functional groups are negatively charged group such as COO⁻, COS⁻, SO₃⁻, or PO₃²⁻ . The negatively charged group and the acidic group may be an end group or a group within the hydrocarbyl chain. In most preferred embodiments, the hydrocarbyl has 2 or 3 carbon atoms and an end group selected from COO⁻, PO₃²⁻, or, most preferably, SO₃⁻.

As used herein, "physiological conditions" refers to the conditions in different tissues and cell compartments of the body.

In certain embodiments, R₈, R₉, R'₉ and/or R"₉ may by substituted by at least one positively charged group and/or at least one basic group that is converted to a positively charged group under physiological conditions. In a preferred embodiment, the at least one positively charged group may be a cation derived from a N-containing group such as, but not limited to, an ammonium - N⁺(RR'R"), hydrazinium -(R)N-N⁺(R'R"), ammoniumoxy O←N⁺(RR')-, iminium >C=N⁺(RR'), amidinium -C(=RN)-N⁻R'R" or guanidinium -(R)N-C(=NR)-N⁺R'R" group, wherein R, R' and R" are as defined above. It is to be understood that the positively charged N-containing group may be an end group, a group within the hydrocarbyl chain, or part of a saturated ring in which the N is protonated, as defined hereinafter. In addition, the at least one positively charged group may also be a cation derived from a N-containing heteroaromatic radical, as defined hereinafter.

In one preferred embodiment, the hydrocarbyl chain is substituted by an ammonium group of the formula -N⁺(RR'R"), wherein each of R, R' and R" independently is H, hydrocarbyl, preferably C₁-C₂₅ alkyl, more preferably C₁-C₁₀ or C₁-C₆ alkyl, or heterocyclyl. When one of R, R' or R" is OH, the group is a hydroxyl ammonium group. Preferably, the ammonium group is a quaternary ammonium group wherein R, R' and R" each independently is C₁-C₆ alkyl such as methyl, ethyl, propyl, butyl, pentyl or hexyl.

In certain embodiments, the ammonium group of the formula -N⁺(RR'R") is a cyclic group, wherein two of R, R' and R" together with the N atom form a 3-7 membered saturated ring, optionally containing a further heteroatom selected from the group consisting of O, S and N atom, and optionally further substituted at the additional N atom, as defined hereinafter. Examples of such cyclic ammonium groups include aziridinium, pyrrolidinium, piperidinium, piperazinium, morpholinium, thiomorpholinium, azepinium, and the like.

In certain embodiments, the positively charged group is a cation derived from a N-heteroaromatic compound that may be a mono- or polycyclic compound that may further contain O, S or additional N atoms. The ring from which the cation is derived should contain at least one N atom and be aromatic, but the other ring(s), if any, can be partially saturated. Examples of N-heteroaromatic cations include pyrazolium, imidazolium, oxazolium, thiazolium, pyridinium, pyrimidinium, quinolinium, isoquinolinium, 1,2,4-triazinium, 1,3,5-triazinium and purinium.

The cation may also be an onium group not containing N such as, but not limited to, a phosphonium [-P⁺(RR'R")], arsonium [-As⁺(RR'R")], oxonium [-O⁺(RR')], sulfonium [-S⁺(RR')], selenonium [-Se⁺(RR')], telluronium [-Te⁺(RR')], stibonium [-Sb⁺(RR'R")], or bismuthonium [-Bi⁺(RR'R")] group, wherein R, R' and R" are as defined above. In preferred embodiments, R, R' and R" are H, C₁-C₆ alkyl such as methyl, ethyl, propyl, isopropyl, n-butyl, isobutyl, sec-butyl, pentyl or hexyl, an aryl group, preferably, phenyl, or an aralkyl group, such as benzyl and phenethyl.

In certain other embodiments, R₈, R₉, R'₉ and/or R"₉ are substituted by at least one basic group that is converted to a positively charged group under physiological conditions. As defined herein, "a basic group that is converted to a positively charged group under physiological conditions" is, at least theoretically, any basic group that will generate under physiological conditions a positively charged group as defined herein, wherein the physiological conditions, as used herein, do not refer solely to the serum, but to different tissues and cell compartments in the body.

In certain embodiments, the basic group is a N-containing group. Examples of such N-containing basic groups include, without being limited to, any amino group that will generate an ammonium group, any imine group that will generate an iminium group, any hydrazine group that will generate a hydrazinium group, any aminooxy group that will generate an ammoniumoxy group, any amidine group that will generate an amidinium group, any guanidine group that will generate a guanidinium group, all as defined herein. Other examples include phosphino and mercapto groups.

Thus, Rs, R₉, R'₉ and/or R"₉ may be substituted by at least one basic group such as -NRR', -C(=NR)-NR'R", -NR-NR'R", -(R)N-C(=NR)-NR'R", O←NR-, or >C=NR, wherein R, R' and R" are as defined above but preferably C₁-C₂₅ alkyl, more preferably C₁-C₁₀ or C₁-C₆ alkyl, or heterocyclyl. In preferred embodiments, the basic group is an amino group -NRR', an end group or a group within the hydrocarbyl chain, that may be a secondary amino, wherein only one of R and R' is H, or a tertiary amino wherein none of R and R' is H, or it may be a cyclic amino wherein R and R' together with the N atom form a 3-7 membered saturated ring, optionally containing a further heteroatom selected from the group consisting of O, S and N atom, and optionally further substituted at the additional N atom.

The C₁-C₂₅ hydrocarbyl defined for R₈, R₉, R'₉ and/or R"₉ may also be substituted by the residue of a mono-, oligo- or polysaccharide such as glycosyl, or of an amino acid, peptide or protein. In addition, R₈, R₉, R'₉ and R"₉ each may independently be is a moiety of an oligosaccharide, or a polysaccharide, preferably a monosaccharide such as glucosamine, and/or the residue of an amino acid, a peptide or a protein. In one preferred embodiment, R₈, R₉, R'₉ or R"₉ at any of the positions, but preferably at position 17³, is the residue of an amino acid, a peptide or a protein. The amino acid, peptide or protein may be negatively charged if they contain a free terminal carboxyl group and/or a residue of an amino acid containing a non-terminal free carboxylic group, e.g. aspartic or glutamic acid.

In one embodiment, R₈, R₉, R'₉ or R"₉ is the residue of an amino acid containing a hydroxy group, such as serine, threonine and tyrosine or a derivative thereof selected from esters such as alkyl, preferably methyl, esters, and N-protected derivatives wherein the N-protecting group is for example tert-butyloxy, carbobenzoxy or trityl, or peptides (oligopeptide or polypeptide) containing such amino acid and/or amino acid derivatives. The hydroxylated amino acid or peptide is linked to the COO⁻ group, preferably at position 17³, of the (bactrio)chlorophyll derivative through its hydroxy group. Examples of such amino acid derivatives are serine methyl ester, N-tert-butyloxycarbonyl-serine, N-trityl-serine methyl ester, tyrosine methyl ester, and N-tert-butoxy-tyrosine methyl ester, and an example of a peptide containing said amino acid derivative is N-carbobenzoxy-seryl serine methyl ester, all of which are prepared as described in the EP 0584552.

In certain embodiments, R₈, R₉, R'₉ or R"₉ is the residue of a cell-specific or tissue-specific ligand selected from peptides and proteins, which are exemplified by, but not limited to, hormone peptides and antibodies, e.g. immunoglobulins. The peptide or protein may be linked directly to the -CO group via an ester, thioester or amide bond, or it may be linked via an ester or amide bond to an end functional group of the C₁-C₂₅ hydrocarbyl radical selected from OH, COOH and NH₂.

In certain embodiments, the (bacterio)chlorophyll derivatives used in accordance with the invention contain COOH, COSH, COO⁻, and/or COS group derived from R₁, R'₂ and R₆ being OH or SH, O⁻R₁₀⁺ or S⁻R₁₀⁺, respectively, i.e., when a carboxylic or thiocarboxylic group or a carboxylate or thiocarboxylate anion is present at the position 13¹, 15¹ (m is 0), 15² (m is 1), and/or 17³.

In certain embodiments R₁, R'₂ and/or R₆ is a basic group -NR₉R'₉ or an ammonium group -N⁺R₉R'₉R"₉, or R₅ is N-R₉ or N⁺R₉R'₉.

In certain embodiments, R₁, R'₂ and/or R₆ is Y-R₈ wherein Y is O and R₈ is a residue of an amino acid or peptide (oligo or polypeptide) linked through an amide bond via a free -NH₂ group.

The cation R₁₀⁺ may be a monovalent or divalent cation derived from an alkaline or alkaline earth metal such as K⁺, Na⁺, Li⁺, Ca⁺, more preferably K⁺; or R₁₀⁺ is an organic cation such as herein defined for "a cation derived from a N-containing group". Preferably, the organic cation is derived from an amine, e.g., NH₄⁺.

As defined herein, A⁻ is a physiologically acceptable anion such as chloride, bromide, iodide, perchlorate, sulfate, phosphate or an organic anion such as acetate, benzoate, caprylate, citrate, lactate, malonate, mandelate, mesylate, oxalate, propionate, succinate, tosylate, and the like.

As defined herein, "a 3-7 membered saturated ring" formed by R and R' together with the N atom to which they are attached may be a ring containing only N such as aziridine, pyrrolidine, piperidine, piperazine or azepine, or it may contain a further heteroatom selected from O and S such as morpholine or thiomorpholine. The further N atom in the piperazine ring may be optionally substituted by alkyl, e.g. C₁-C₆ alkyl, that may be substituted by halogen, OH or amino. The onium groups derived from said saturated rings include aziridinium, pyrrolidinium, piperidinium, piperazinium, morpholinium, thiomorpholinium and azepinium.

In certain embodiments, the (bacterio)chlorophyll derivative used in accordance with the present invention is unmetalated, namely M is 2H. In preferred embodiments, the photosensitizer is metalated and M is Mg, Pd, Pt, Zn, In, Gd, or Yb, more preferably Pd.

In certain embodiments, the ophthalmic composition for treatment of corneal thinning or scleral stretching comprises a derivative of chlorophyll or bacteriochlorophyll of formula II in which R₆ or both R₁ and R₆ are -NR₉R'₉. Preferably according to these embodiments, R₉ is H and R'₉ is a C₁-C₁₀ alkyl substituted by at least one group selected from a positively charged group, a negatively charged group, an acidic group that is converted to a negatively charged group under physiological conditions, or a basic group that is converted to a positively charged group under physiological conditions. In more particular embodiments R'₉ is a C₁-C₆ alkyl substituted by the acidic SO₃H group or an alkaline salt thereof, or by a basic group -NRR' or -NH-(CH₂)₂₋₆-NRR', wherein each of R and R' independently is H, C₁-C₆ alkyl optionally substituted by NH₂, or R and R' together with the N atom form a 5-6 membered saturated ring, optionally containing an O or N atom and optionally further substituted at the additional N atom by -(CH₂)₂₋₆-NH₂.

In certain embodiments, the ophthalmic composition of the invention comprises a bacteriochlorophyll derivative of formula II, wherein:
M is 2H, Mg, Pd, or Zn;
R₁ is selected from:
   (i) -O⁻R₁₀⁺;
   (ii) Y-R₈ wherein Y is O or S and R₈ is the residue of an amino acid, a peptide or a protein;
   (iii) -NH-CH₂-CH(OH)-CH₂OH;
   (iv) - NH-(CH₂)ₙ-OH;
   (v) - NH-CH(OH)-CH₃;
   (vi) - NH-(CH₂)ₙ-NR-(CH₂)ₙ-OH;
   (vii) glycosylamino; or
   (viii) NHR'₉ which is as defined for R₆;
R'₂ is C₁-C₆ alkoxy such as methoxy, ethoxy, propoxy or butoxy, more preferably methoxy;
R₄ is -C(=O)-CH₃, -CH=N-(CH₂)ₙ-SO₃⁻R₁₀⁺; -CH=N-(CH₂)ₙ-COO⁻R₁₀⁺; -CH=N-(CH₂)ₙ-PO₃²⁻(R₁₀⁺)₂; -CH₂-NH-(CH₂)ₙ-SO₃⁻R₁₀⁺; -NH-(CH₂)ₙ-COO⁻R₁₀⁺; -NH-(CH₂)ₙ-PO₃²⁻(R₁₀⁺)₂; or-C(CH₃)=NR₉, preferably C(CH₃)=N-(CH₂)ₙ-NH₂, or -C(CH₃)=N-(CH₂)ₙ-N(R)₃⁺A⁻;
R₆ is selected from (i) NHR'₉, selected from:
   (a) -NH-(CH₂)ₙ-SO₃⁻R₁₀⁺, preferably -NH-(CH₂)₂-SO₃R₁₀⁺ or -NH-(CH₂)₃-SO₃R₁₀⁺;
   (b) -NH-(CH₂)ₙ-COO⁻R₁₀⁺;
   (c) -NH-(CH₂)ₙ-PO₃²⁻(R₁₀⁺)₂;
   (d)-NH-(CH₂)ₙ-B:
   (e) 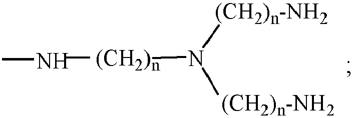
   (f) 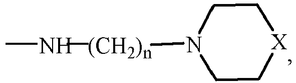 preferably -NH-(CH₂)₂-1-morpholino or -NH-(CH₂)₃-piperazino;
   (g) 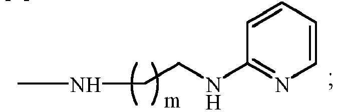
   (h) -NH-(CH₂)ₙ-N(R")-(CH₂)ₙ-NRR', preferably -NH-(CH2)3-NH-(CH2)3-NH2;
   (j) -NH-(CH₂)ₙ-NRR', preferably -NH-CH₂-CH₂-NRR';
R₁₀⁺ is H⁺, or a monovalent cation such as K⁺, Na⁺, Li⁺, or NH₄⁺, more preferably K⁺;
X is O, S or NH;
B is a positively charged group selected from ammonium -N⁺RR'R", preferably - N(CH₃)₃⁺A⁻, guanidinium, sulfonium, phosphonium, arsonium; or B is a basic group that is converted to a positively charged group under physiological conditions, selected from amino - NRR', preferably -NH₂, guanidino, phosphino, or arsino;
m is 1, n is an integer from 1 to 10, preferably 2 or 3; and
A⁻ is a physiologically acceptable anion;
wherein R, R' and R" each independently is H or C₁-C₆ alkyl.

In preferred embodiments, the pharmaceutical composition comprises bacteriochlorin or rhodobacteriochlorin derivative of formula II wherein only R₆ or both R₁ and R₆ are selected from -NH-(CH₂)₂-SO₃R₁₀⁺, -NH-(CH₂)₃-SO₃R₁₀⁺, -NH-(CH₂)₃-NH-(CH₂)₃-NH₂, -NH-(CH₂)₂-1-morpholino, or -NH-(CH₂)₃-piperazino-(CH₂)₃-NH₂, and R₁₀⁺ is a monovalent cation such as K⁺, Na⁺, Li⁺, NH₄⁺, preferably K⁺. In certain more preferred embodiments, R₁ and R₆ are both -NH-(CH₂)₂-SO₃R₁₀⁺ or -NH-(CH₂)₃-SO₃R₁₀⁺.

In other preferred embodiments, chlorin or (rhodo)bacteriochlorin derivatives of the formula II are used in accordance with the invention, having at least one negatively charged group and M is absent or is divalent Pd or Zn ion, preferably Pd. In these embodiments, R₁ is selected from -O⁻R₁₀⁺, -NH-(CH₂)ₙ-SO₃⁻R₁₀⁺, -NH-(CH₂)ₙ-COO⁻R₁₀⁺ or -NH-(CH₂)ₙ-PO₃²⁻ (R₁₀⁺)₂, or R₁ is Y-R₈ wherein Y is O, S and R₈ is the residue of an amino acid, a peptide or a protein; R'₂ is C₁-C₆ alkoxy such as methoxy, ethoxy, propoxy, butoxy, more preferably methoxy; R₄ is selected from -C(=O)-CH₃, -CH=N-(CH₂)ₙ-SO₃⁻R₁₀⁺, -CH=N-(CH₂)ₙ-COO⁻R₁₀⁺, -CH=N-(CH₂)ₙ-PO₃²⁻ (R₁₀⁺)₂, or -CH₂-NH-(CH₂)ₙ-SO₃⁻R₁₀⁺; R₆ is-NH-(CH₂)ₙ-SO₃⁻R₁₀⁺, -NH-(CH₂)ₙ-COO⁻R₁₀⁺, or - NH-(CH₂)ₙ-PO₃²⁻(R₁₀⁺)₂; R₁₀⁺ is a monovalent cation such as K⁺, Na⁺, Li⁺, or NH₄⁺, more preferably K⁺; m is 1, and n is an integer from 1 to 10, preferably 2 or 3.

In a more preferred embodiment, the negatively charged photosensitizer used in accordance with the invention is a bacteriochlorin derivative of the formula II wherein: M is Pd; R₁ is -O⁻R₁₀⁺, -NH-(CH₂)ₙ-SO₃⁻R₁₀⁺, or Y-R₈ wherein Y is O or S and R₈ is the residue of a protein, preferably immunoglobulin; R'₂ is C₁-C₆ alkoxy such as methoxy, ethoxy, propoxy, butoxy, more preferably methoxy; R₄ is -C(=O)-CH₃, -CH=N-(CH₂)ₙ-SO₃⁻R₁₀⁺ or -CH₂-NH-(CH₂)ₙ-SO₃⁻R₁₀⁺; R₆ is -NH-(CH₂)ₙ-SO₃⁻R₁₀⁺, NH-(CH₂)ₙ-COO⁻R₁₀⁺, or NH-(CH₂)ₙ-PO₃²⁻(R₈⁺)₂; R₁₀⁺ is a monovalent cation such as K⁺, Na⁺, Li⁺ or NH₄⁺, more preferably K⁺; m is 1, and n is 2 or 3, more preferably 2.

Examples of rhodobacteriochlorin derivatives of formula II having one, two or three negatively charged groups at positions 3, 13 and/or 17 are:
Palladium 3¹-oxo-15-methoxycarbomylmethyl-rhodobacteriochlorin 13¹-(2-sulfoethyl)amide dipotassium salt (herein designated WST11);
Palladium 3¹-oxo-15-methoxycarbomylmethyl-rhodobacteriochlorin 13¹-(-sulfopropyl)amide dipotassium salt (compound **1**);
Palladium 3¹-oxo-15-methoxycarbomylmethyl-rhodobacteriochlorin 13¹,17³-di-(2-sulfoethyl)amide dipotassium salt (compound **2**);
Palladium 3¹-oxo-15-methoxycarbomylmethyl-rhodobacteriochlorin 13¹,17³-di-(3-sulfopropyl)amide dipotassium salt (compound **3**);
3¹-oxo-15-methoxycarbonylmethyl-rhodobacteriochlorin 13¹-(2-sulfoethyl) amide dipotassium salt (compound **4**);
3¹-oxo-15-methoxycarbomylmethyl-rhodobacteriochlorin 13¹-(-sulfopropyl)amide dipotassium salt (compound **5**);
3¹-oxo-15-methoxycarbomylmethyl-rhodobacteriochlorin 13¹,17³-di-(2-sulfoethyl)amide dipotassium salt (compound **6**);
3¹-oxo-15-methoxycarbomylmethyl-rhodobacteriochlorin 13¹,17³-di-(3-sulfopropyl)amide dipotassium salt (compound **7**);
Zinc 3¹-oxo-15-methoxycarbonylmethyl-rhodobacteriochlorin 13¹-(2-sulfoethyl) amide dipotassium salt;
Palladium 3¹-oxo-15-methoxycarbonylmethyl-rhodobacteriochlorin 13¹-(2-sulfoethyl)amide, 17³-(N-immunoglobulin G)amide potassium salt;
Palladium 3¹-oxo-15-methoxycarbonylmethyl-rhodobacteriochlorin 13¹-(2-carboxyethyl)amide dipotassium salt;
Palladium 3¹-oxo-15-methoxycarbonylmethyl-rhodobacteriochlorin 13¹-(3-phosphopropyl)amide tripotassium salt
Palladium 3¹-(3-sulfopropylimino)-15-methoxycarbonylmethyl-rhodobacteriochlorin 13¹,17³-di(3-sulfopropyl)amide tripotassium salt;
Palladium 3¹-(3-sulfopropylamino)-15-methoxycarbonylmethyl-rhodobacteriochlorin 13¹,17³-di(3-sulfopropyl)amide tripotassium salt.
Palladium 3¹-oxo-15-methoxycarbonylmethyl-rhodobacteriochlorin 13¹,17³-di(3-propionyl)amide salt;
Palladium 3¹-oxo-15-methoxycarbonylmethyl-rhodobacteriochlorin 13¹,17³-di[2-(3-propionylamino)-sulfoethyl]amide dipotassium salt;
Palladium 3¹-oxo-15-methoxycarbonylmethyl-rhodobacteriochlorin 13¹,17³-di[2-(3-propionylamino)-phosphoethyl]amide dipotassium salt;
Palladium 3¹-oxo-15-methoxycarbonylmethyl-rhodobacteriochlorin 13¹,17³-di(3-thiopropionyl)amide dipotassium salt 3¹-hydroxy-3¹-deoxo-bacteriopheophorbide a;
3¹-(Pyridin-4-ylmethoxy)-3¹-deoxo-bacteriopheophorbide a;
Bacteriopheophorbide a 17³-[(2,6-dichloro-4-methoxyphenyl)(2,4-dichlorophenyl)]methyl ester;
3¹-Hydroxy-3¹-deoxo-bacteriopheophorbide a 17³-[(2,6-dichloro-4-methoxyphenyl)(2,4-dichlorophenyl)]methyl ester;
3¹-trifluoroacetoxy-3¹-deoxo-bacteriopheophorbide a 17³-[(2,6-dichloro-4-methoxyphenyl)(2,4-dichlorophenyl)]methyl ester;
3¹-Bromo-3¹-deoxo-bacteriopheophorbide a;
3-Vinyl-3-deacetyl-bacteriopheophorbide;
3¹-(2-Hydroxyethoxy)-3¹-deoxo-bacteriopheophorbide a;
3¹-(2,2,2-Trifluoroethoxy)-3¹-deoxo-bacteriopheophorbide a;
3¹-(2-Mercaptoethylsulfanyl)-3¹-deoxo-bacteriopheophorbide a;
3¹-(2-Hydroxyethylamino)-3¹-deoxo-bacteriopheophorbide a;
Cobalt(III) 3¹-oxo-15-methoxycarbonylmethyl-rhodobacteriochlorin 13¹-(2-sulfoethyl)amide dipotassium salt;
Iron(III) 3¹-oxo-15-methoxycarbonylmethyl-rhodobacteriochlorin 13¹-(2-sulfoethyl)amide dipotassium salt; and
Nickel(II) Bacteriopheophorbide a;
Platinum(II) Bacteriopheophorbide a.

In a preferred embodiment, the compound used in accordance with the present invention are pharmaceutically acceptable salts of taurinated or homotaurinated bacteriochlorin derivative with a monovalent or divalent alkaline or alkaline earth metal cation, or with NH₄⁺ selected from:
Palladium 3¹-oxo-15-methoxycarbonylmethyl-rhodobacteriochlorin 13¹-(2-sulfoethyl) amide;
Palladium 3¹-oxo-15-methoxycarbomylmethyl-rhodobacteriochlorin 13¹-(-sulfopropyl)amide;
Palladium 3¹-oxo-15-methoxycarbomylmethyl-rhodobacteriochlorin 13¹,17³-di-(2-sulfoethyl)amide;
Palladium 3¹-oxo-15-methoxycarbomylmethyl-rhodobacteriochlorin 13¹,17³-di-(3-sulfopropyl)amide;
3¹-oxo-15-methoxycarbonylmethyl-rhodobacteriochlorin 13¹-(2-sulfoethyl) amide;
3¹-oxo-15-methoxycarbomylmethyl-rhodobacteriochlorin 13¹-(-sulfopropyl)amide;
3¹-oxo-15-methoxycarbomylmethyl-rhodobacteriochlorin 13¹,17³-di-(2-sulfoethyl) amide; and
3¹-oxo-15-methoxycarbomylmethyl-rhodobacteriochlorin 13¹,17³-di-(3-sulfopropyl)amide.

Most preferred compounds are WST11 and compounds 1-7.

The bacteriochlophyll derivatives used in the invention can be prepared by the methods described in US 7,947,672 or in WO 2005/120573. For the preparation of compounds wherein Rs is the residue of an amino acid, peptide or protein, the methods described in EP 0584552 may be applied. For the preparation of negatively charged bacteriochlorin derivatives wherein R₈ is a residue of amino acid, the method disclosed in EP 0584552 may be combined with the method described in Scheme 1 of US 7,947,672.

Method for the preparation of negatively-charged compounds of formula II are disclosed in US 7,947,672 mentioned above. For example, preparation of a bacteriochlorin compound wherein R₁ is -O⁻R₁₀⁺; R'₂ is -OCH₃; R₃ is acetyl; R₆ is a group -NH-(CH₂)ₙ-SO₃⁻R₁₀⁺, -NH-(CH₂)ₙ-COO⁻ R₁₀⁺, or -NH-(CH₂)ₙ-PO₃²⁻(R₁₀⁺)₂; R₁₀⁺ is a monovalent cation; m is 1 and n is 1 to 10, comprises: (i) reacting the corresponding metalated bacteriopheophorbide (M-bacteriopheophorbide) of formula I herein wherein R₁ is OH with an aminosulfonic acid of the formula H₂N-(CH₂)ₙ-SO₃H (e.g., taurine H₂N-(CH₂)₂-SO₃H or homotaurine H₂N-(CH₂)₃-SO₃H) aminocarboxylic acid of the formula H₂N-(CH₂)ₙ-COOH or aminophosphonic acid of the formula H₂N-(CH₂)ₙ-PO₃H₂, respectively, in a R₁₀⁺-buffer; and (ii) isolating the desired compound of formula II.

Bacteriochlorins of formula II having the same negatively charged groups mentioned above at positions 13 and 17 may be prepared by reacting the corresponding M-bacteriopheophorbide with an excess of the reagent aminosulfonic, aminocarboxylic or aminophosphonic acid as described above, and isolation of the desired 13,17-disubstituted derivative, or a different route can be followed as disclosed in US 7,947,672. For example, a bacteriochlorin of the formula II wherein R₁ and R₆ are each a group -NH-(CH₂)ₙ-SO₃⁻R₁₀⁺; R₂ is -OCH₃; R₃ is acetyl; R₁₀⁺ is a monovalent cation; m is 1 and n is 1 to 10, are prepared by: (i) coupling the corresponding M-bacteriopheophorbide of formula I wherein R₁ is OH with N-hydroxy-sulfosuccinimide (sulfo NHS) in the presence of 1-ethyl-3-(3-dimethylaminopropyl)-carbodiimide (EDC); (ii) reacting the resulting M-bacteriopheophorbide-17³-N-hydroxysulfosuccinimide ester with an excess of an aminosulfonic acid of the formula H₂N-(CH₂)ₙ-SO₃H in a R₈⁺-buffer, thus obtaining a compound of formula I having a sole negatively charged group at position 17; (iii) reacting this product with an excess of H₂N-(CH₂)ₙ-SO₃H in a R₈⁺-buffer; and isolating the desired bacteriochlorin of formula II. When the aminosulfonic acid is replaced by aminocarboxylic or aminophosphonic acid, the corresponding carboxylate and phosphonate derivatives are obtained.

In certain embodiments, the pharmaceutical compositions used in accordance with the invention comprise a chlorophyll or bacteriochlorophyll derivative of the formula II containing at least one positively charged group and/or at least one basic group that is converted to a positively charged group at the physiological pH, wherein the positively charged groups and basic group are as defined above.

In certain embodiments, the photosensitizer is a chlorophyll or (rhodo)bacteriochlorophyll derivative of formula II and R₆ is -NR₉R'₉, R₉ is H and R'₉ is HOCH₂-CH(OH)-CH₂-.

In certain preferred embodiments, the pharmaceutical composition used according to the invention comprises a bacteriochlorin or rhodobacteriochlorin derivative of the formula II wherein R₁ is selected from OH, -NR₉R'₉, or -NR₉-CH₂-CH(OH)-CH₂OH; and R₆ is selected from - NR₉R'₉ or -NR₉-CH₂-CH(OH)-CH₂OH, wherein R₉ is H or C₁-C₆ alkyl; and R'₉ is C₁-C₂₅ hydrocarbyl substituted by at least one positively charged group and/or at least one basic group that is converted to a positively charged group under physiological conditions. In preferred embodiments, R'₉ is C₁-C₂₅ alkyl, preferably C₁-C₁₀, more preferably C₁-C₆ alkyl, substituted by at least one positively charged group, more preferably -N⁺RR'R", or by at least one basic group, preferably -NRR', and optionally interrupted by a -N(R")- group, wherein R and R' each independently is H, C₁-C₆ alkyl optionally substituted by NR"R", or heterocyclyl such as pyridyl, or R and R' together with the N atom form a 6-membered ring further containing an O, S or N atom, and R" is H or C₁-C₆ alkyl.

Particular compounds used in accordance with these preferred embodiments are positively charged bacteriochlorophyll derivatives wherein M is absent or is Pd; R'₂ is -OR₈ wherein R₈ is C₁-C₆ alkyl; R₄ is -COCH₃; and wherein:
(a) R₁ is OH and R₆ is -NHR'₉;
(b) R₁ and R₆ are both the same -NHR'₉ group;
(c) R₁ is -NH-CH₂-CH(OH)-CH₂OH and R₆ is a -NHR'₉ group;
(d) R₁ is a NHR'₉ group and R₆ is -NH-CH₂-CH(OH)-CH₂OH; and
(e) R₆ is -NH-CH₂-CH₂-NRR'; and R₁ is selected from the group consisting of
   - NH-(CH₂)ₙ-OH;
   - NH-CH(OH)-CH₃;
   - NH-(CH₂)ₙ-NR-(CH₂)ₙ-OH; or
   - glycosylamino.

The -NHR'₉ group is selected from:
(i) -NH-(CH₂)ₙ-NRR' or -NH-(CH₂)ₙ-N⁺RR'R";
(ii) -NH-(CH₂)ₙ-N(R")-(CH₂)ₙ-NRR';
(iii) 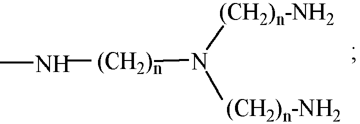
(iv) 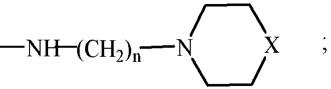 and
(v) 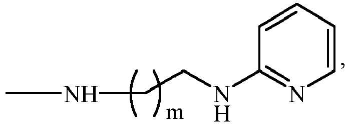 wherein
   X is O, S or NR;
   R, R' and R" are as defined above, but preferably each independently is H or C₁-C₆ alkyl, more preferably methyl or ethyl;
   n is an integer from 1 to 10, preferably 2 to 6, more preferably 2 or 3; and
   m is an integer from 1 to 6, preferably 1 to 3.

Particular examples of such positively charged compounds or compounds that become positively charged under physiological pH are compounds include the herein designated compounds **4'-7', 8**- **12** and **24-75:**
Palladium 3¹-oxo-15-methoxycarbonylmethyl-rhodobacteriochlorin-13¹-(2-N³-trimethylammoniumethyl)amide chloride salt (compound **12**);
Palladium 3¹-oxo-15-methoxycarbonylmethyl-rhodobacteriochlorin-13¹-(2-N³-(trimethylammoniumethyl)amide acetate salt (compound **24**);
Palladium 3¹-oxo-15-methoxycarbonylmethyl-rhodobacteriochlorin-13¹-(2-N²-dimethylaminoethyl)amide (compound **25**);
Palladium 3¹-oxo-15-methoxycarbonylmethyl-rhodobacteriochlorin-13¹-(3-N²-dimethylaminopropyl)amide (compound **26**);
Palladium 3¹-oxo-15-methoxycarbonylmethyl-rhodobacteriochlorin-13¹-(2-[(2-aminoethyl)amino]ethyl)amide (compound **27**);
Palladium 3¹-oxo-15-methoxycarbonylmethyl-rhodobacteriochlorin-13¹-([2-bis(2-aminoethyl)amino]ethyl)amide (compound **28**);
Palladium 3¹-oxo-15-methoxycarbonylmethyl-rhodobacteriochlorin-13¹-(2-morpholino-N-ethyl)amide (compound **29**);
Palladium 3¹-oxo-15-methoxycarbonylmethyl-rhodobacteriochlorin-13¹-(2-piperazino-N-ethyl)amide (compound **30**);
Palladium 3¹-oxo-15-methoxycarbonylmethyl-rhodobacteriochlorin-13¹-(2-[(2-N²-diethylaminoethyl)amino]ethyl)amide (compound **31**);
Palladium 3¹-oxo-15-methoxycarbonylmethyl-rhodobacteriochlorin-13¹-(3-[(3-aminopropyl)amino]propyl)amide (compound **32**);
3¹-oxo-15-methoxycarbonylmethyl-rhodobacteriochlorin-13¹,17³-di(2-aminoethyl)amide (compound **4'**);
3¹-oxo-15-methoxycarbonylmethyl-rhodobacteriochlorin-13¹,17³-di(2-N³-trimethylammoniumethyl)amide dicitrate salt (compound **5'**);
3¹-oxo-15-methoxycarbonylmethyl-rhodobacteriochlorin-13¹,17³-di(3-aminopropyl)amide (compound **6'**);
3¹-oxo-15-methoxycarbonylmethyl-rhodobacteriochlorin-13¹,17³-di(3-N³-trimethylammoniumpropyl)amide dicitrate salt (compound **7'**);
3¹-oxo-15-methoxycarbonylmethyl-rhodobacteriochlorin-13¹,17³-di(6-aminohexyl)amide (compound **8**);
3¹-oxo-15-methoxycarbonylmethyl-rhodobacteriochlorin-13¹,17³-di(6-N³-trimethylammoniumhexyl)amide dicitrate salt (compound **9**);
Palladium 3¹-oxo-15-methoxycarbonylmethyl-rhodobacteriochlorin 13¹,17³-di(2-aminoethyl)amide (compound **10**);
Palladium 3¹-oxo-15-methoxycarbonylmethyl-rhodobacteriochlorin 13¹,17³-di(2-N³-trimethylammoniumethyl)amide diphosphate salt (compound **11**);
Palladium 3¹-oxo-15-methoxycarbonylmethyl-rhodobacteriochlorin-13¹,17³-di(2-N³-trimethylammoniumethyl)amide diacetate salt (compound **33**);
Palladium 3¹-oxo-15-methoxycarbonylmethyl-rhodobacteriochlorin-13¹,17³-di(3-aminopropyl)amide (compound **34**);
Palladium 3¹-oxo-15-methoxycarbonylmethyl-rhodobacteriochlorin-13¹,17³-di(4-aminobutyl)amide (compound **35**);
Palladium 3¹-oxo-15-methoxycarbonylmethyl-rhodobacteriochlorin-13¹,17³-di(2-N²-dimethylaminoethyl)amide (compound **36**);
Palladium 3¹-oxo-15-methoxycarbonylmethyl-rhodobacteriochlorin-13¹,17³-di(3-N²-dimethylaminopropyl)amide (compound **37**);
Palladium 3¹-oxo-15-methoxycarbonylmethyl-rhodobacteriochlorin-13¹,17³-di-(2-[(2-aminoethyl)amino]ethyl)amide (compound **38**);
Palladium 3¹-oxo-15-methoxycarbonylmethyl-rhodobacteriochlorin-13¹,17³-di-(2-[(2-N²-diethylaminoethyl)amino]ethyl)amide (compound **39**);
Palladium 3¹-oxo-15-methoxycarbonylmethyl-rhodobacteriochlorin-13¹,17³-di(2-morpholino-N-ethyl)amide (compound **40**);
Palladium 3¹-oxo-15-methoxycarbonylmethyl-rhodobacteriochlorin-13¹,17³-di(2-piperazino-N-ethyl)amide (compound **41**);
Palladium 3¹-oxo-15-methoxycarbonylmethyl-rhodobacteriochlorin-13¹,17³-di-(3-[(3-aminopropyl)amino]propyl)amide (compound **42**);
Palladium 3¹-oxo-15-methoxycarbonylmethyl-rhodobacteriochlorin-13¹,17³-di([2-bis(2-aminoethyl)amino]ethyl)amide (compound **43**);
Palladium 3¹-oxo-15-methoxycarbonylmethyl-rhodobacteriochlorin-13¹,17³-di(2-N-(2'-pyridyl)aminoethyl)amide (compound **44**);
Palladium 3¹-oxo-15-methoxycarbonylmethyl-rhodobacteriochlorin-13¹,17³-di(2-N²-diethylaminoethyl)amide (compound **45**);
Palladium 3¹-oxo-15-methoxycarbonylmethyl-rhodobacteriochlorin-13¹-(2-aminoethyl)amide-17³-(2,3-dihydroxypropyl)amide (compound **48**);
Palladium 3¹-oxo-15-methoxycarbonylmethyl-rhodobacteriochlorin-13¹-(2-N²-dimethylaminoethyl)amide-17³-(2,3-dihydroxypropyl)amide (compound **50**);
Palladium 3¹-oxo-15-methoxycarbonylmethyl-rhodobacteriochlorin-13¹-(2-[(2-aminoethyl)amino]ethyl)amide-17³-(2,3-dihydroxypropyl)amide (compound **55**);
Palladium 3¹-oxo-15-methoxycarbonylmethyl-rhodobacteriochlorin-13¹-(2-N-(2'-pyridyl)aminoethyl)amide-17³-(2,3-dihydroxypropyl)amide (compound **57**);
Palladium 3¹-oxo-15-methoxycarbonylmethyl-rhodobacteriochlorin-13¹-([2-bis(2-aminoethyl)amine]ethyl)amide-17³-(2,3-dihydroxypropyl)amide (compound **59**);
Palladium 3¹-oxo-15-methoxycarbonylmethyl-rhodobacteriochlorin-13¹-(3-aminopropyl)amide-17³-(2,3-dihydroxypropyl)amide (compound **60**);
Palladium 3¹-oxo-15-methoxycarbonylmethyl-rhodobacteriochlorin-13¹-(4-aminobutyl)amide-17³-(2,3-dihydroxypropyl)amide (compound **61**);
Palladium 3¹-oxo-15-methoxycarbonylmethyl-rhodobacteriochlorin-13¹-(2-N²-diethylaminoethyl)amide-17³-(2,3-dihydroxy propyl)amide (compound **62**);
Palladium 3¹-oxo-15-methoxycarbonylmethyl-rhodobacteriochlorin-13¹-(2-N-ethylaminoethyl)amide-17³-(2,3-dihydroxy propyl)amide (compound **63**);
Palladium 3¹-oxo-15-methoxycarbonylmethyl-rhodobacteriochlorin-13¹-(3-N-methylaminopropyl)amide-17³-(2,3-dihydroxypropyl)amide (compound **64**);
Palladium 3¹-oxo-15-methoxycarbonylmethyl-rhodobacteriochlorin-13¹-(3-N-(2'-pyridyl)aminopropyl)amide-17³-(2,3-dihydroxypropyl)amide (compound **71**);
Palladium 3¹-oxo-15-methoxycarbonylmethyl-rhodobacteriochlorin-13¹-(4-N-(2'-pyridyl)aminobutyl)amide-17³-(2,3-dihydroxypropyl)amide (compound **72**);
Palladium 3¹-oxo-15-methoxycarbonylmethyl-rhodobacteriochlorin-13¹-(2,3-dihydroxypropyl)amide-17³-(2-trimethylammoniumethyl)amide (compound **46**);
Palladium 3¹-oxo-15-methoxycarbonylmethyl-rhodobacteriochlorin-13¹-(2,3-dihydroxypropyl)amide-17³-(2-aminoethyl)amide (compound **47**);
Palladium 3¹-oxo-15-methoxycarbonylmethyl-rhodobacteriochlorin-13¹-(2,3-dihydroxypropyl)amide-17³-(2-N²-dimethyl aminoethyl)amide (compound **49**);
Palladium 3¹-oxo-15-methoxycarbonylmethyl-rhodobacteriochlorin-13¹-(2,3-dihydroxypropyl)amide-17³-(2-[(2-aminoethyl)amino]ethyl)amide (compound **51**);
Palladium 3¹-oxo-15-methoxycarbonylmethyl-rhodobacteriochlorin-13¹-(2,3-dihydroxypropyl)amide-17³-(2-[(2-N²-diethyl aminoethyl)amino]ethyl)amide (compound **52**);
Palladium 3¹-oxo-15-methoxycarbonylmethyl-rhodobacteriochlorin-13¹-(2,3-dihydroxypropyl)amide-17³-(2-morpholino-N-ethyl)amide (compound **53**);
Palladium 3¹-oxo-15-methoxycarbonylmethyl-rhodobacteriochlorin-13¹-(2,3-dihydroxypropyl)amide-17³-(2-piperazino-N-ethyl)amide (compound **54**);
Palladium 3¹-oxo-15-methoxycarbonylmethyl-rhodobacteriochlorin-13¹-(2,3-dihydroxypropyl)amide-17³-(2-N-(2'-pyridyl)aminoethyl)amide (compound **56**);
Palladium 3¹-oxo-15-methoxycarbonylmethyl-rhodobacteriochlorin-13¹-(2,3-dihydroxypropyl)amide-17³-([2-bis(2-aminoethyl)amino]ethyl)amide (compound **58**);
Palladium 3¹-oxo-15-methoxycarbonylmethyl-rhodobacteriochlorin-13¹-(2,3-dihydroxypropyl)amide-17³-(3-N-(2'-pyridyl)aminopropyl)amide (compound **73**);
Palladium 3¹-oxo-15-methoxycarbonylmethyl-rhodobacteriochlorin-13¹-(2,3-dihydroxypropyl)amide-17³-(4-N-(2'-pyridyl)aminobutyl)amide (compound **74**);
Palladium 3¹-oxo-15-methoxycarbonylmethyl-rhodobacteriochlorin-13¹-(2-N²-dimethylaminoethyl)amide-17³-(2-hydroxy ethyl)amide (compound **65**);
Palladium 3¹-oxo-15-methoxycarbonylmethyl-rhodobacteriochlorin-13¹-(2-N²-dimethylaminoethyl)amide-17³-(3-hydroxy propyl)amide (compound **66**);
Palladium 3¹-oxo-15-methoxycarbonylmethyl-rhodobacteriochlorin-13¹-(2-N²-dimethylaminoethyl)amide-17³-(2-hydroxy propyl)amide (compound **67**);
Palladium 3¹-oxo-15-methoxycarbonylmethyl-rhodobacteriochlorin-13¹-(2-N²-dimethylaminoethyl)amide-17³-((R)-2-hydroxypropyl)amide (compound **68**);
Palladium 3¹-oxo-15-methoxycarbonylmethyl-rhodobacteriochlorin-13¹-(2-N²-dimethylaminoethyl)amide-17³-((S)-2-hydroxypropyl)amide (compound **69**);
Palladium 3¹-oxo-15-methoxycarbonylmethyl-Rhodobacteriochlorin-13¹-(2-N²-dimethylaminoethyl)amide-17³-(2-(2-hydroxyethylamino)ethyl)amide (compound **70**); and
Palladium 3¹-oxo-15-methoxycarbonylmethyl-rhodobacteriochlorin-13¹-(2-N²-dimethylaminoethyl)amide-17³-(glycosyl)amide (compound **75**).

The compounds listed above can be prepared e.g., as described in details in WO 2005/120573 mentioned above.

Other positively charged bacteriochlorin derivatives and basic bacteriochlorin derivatives that become positively charged under physiological pH are compounds of the formula II, wherein M is Pd, R'₂ is -OR₈ wherein R₈ is C₁-C₆ alkyl, preferably methyl, R₄ is -COCH₃, and R₁ and/or R₆ are -NR₉R'₉, wherein R₉ is H and R'₉ is C₁-C₂₅ hydrocarbyl, preferably C₁-C₂₅ alkyl, more preferably C₁-C₁₀ alkyl, substituted by: (i) a guanidino or guanidinium group; (ii) a sulfonium group; (iii) a phosphino or phosphonium group; (iv) an arsino or arsonium group.

In a more preferred embodiment, R₁ and R₆ are both the same group selected from:
(i) - NH-(CH₂)ₙ-C(=NH)-NH₂ or -NH-(CH₂)ₙ-C(=NH)-N⁺(R)₃A⁻, more preferably, -NH-(CH₂)ₙ-C(=NH)-N(CH₃)₃⁺A⁻;
(ii) -NH-(CH₂)ₙ-S⁺(R)₂A⁻, more preferably, -NH-(CH₂)ₙ-S(CH₃)₂⁺A⁻;
(iii) -NH-(CH₂)ₙ-P(R)₂, more preferably, -NH-(CH₂)ₙ-P(CH₃)₂ or NH-(CH₂)ₙ-P⁺(R)₃A⁻, more preferably, -NH-(CH₂)ₙ-P⁺(CH₃)₃A⁻; or
(iv) -NH-(CH₂)ₙ-As(R)₂, more preferably, -NH-(CH₂)ₙ-As(CH₃)₂ or NH-(CH₂)ₙ-As⁺(R)₃ A⁻, more preferably, -NH-(CH₂)ₙ-As⁺(CH₃)₃A⁻, wherein n is an integer from 1 to 10, preferably 2, 3 or 6.

Examples of such compounds are the compounds herein designated compounds **14, 14a, 15-19:**
Palladium 3¹-oxo-15-methoxycarbonylmethyl-rhodobacteriochlorin 13¹,17³-di(2-guanidinoethyl)amide (compound **14**);
Palladium 3¹-oxo-15-methoxycarbonylmethyl-rhodobacteriochlorin 13¹,17³⁻di(2-trimethylguanidiniumethyl)amide (compound **14a**);
Palladium 3¹-oxo-15-methoxycarbonylmethyl-rhodobacteriochlorin-13¹-(2-S²-dimethylsulfoniumethyl)amide citrate salt (compound **15**);
3¹-oxo-15-methoxycarbonylmethyl-rhodobacteriochlorin-13¹,17³-di(2-P³-trimethylphosphoniumethyl)amide dicitrate salt (compound **17**);
3¹-Oxo-15-methoxycarbonylmethyl-rhodobacteriochlorin-13¹,17³-di(2-dimethylphosphinoethyl)amide (compound **18**); and
3¹-Oxo-15-methoxycarbonylmethyl-rhodobacteriochlorin-13¹,17³-di(2-As³-trimethylarsoniumethyl)amide dicitrate salt (compound **19**).

The above compounds may be prepared as taught in WO 2005/120573.

In still further preferred embodiments, the Bchl derivative used in accordance with the invention is a bacteriochlorin derivative of the formula II, wherein M is 2H or Pd, R'₂ is -OR₈ wherein R₈ is C₁-C₆ alkyl, preferably methyl, R₄ is -C(CH₃)=NR₉, and R₁ and/or R₆ are -NR'₉R"₉, wherein R'₉ is H and R₉ and R"₉ are C₁-C₂₅ hydrocarbyl, preferably C₁-C₂₅ alkyl, more preferably C₁-C₁₀ alkyl, substituted by at least one amino end group or a positively charged group, more preferably an ammonium end group of the formula -N⁺(RR'R")A⁻, wherein R, R' and R" are preferably the same C₁-C₆ alkyl, preferably methyl, and A⁻ is an anion. In a more preferred embodiment of the invention, R₄ is a group of the formula -C(CH₃)=N-(CH₂)ₙ-NH₂ or - C(CH₃)=N-(CH₂)ₙ-N(R)₃⁺A⁻, most preferably -C(CH₃)=N-(CH₂)ₙ-N(CH₃)₃⁺A⁻, and R₁ and R₆ are a group of the formula -NH-(CH₂)ₙ-NH₂ or NH-(CH₂)ₙ-N(R)₃⁺A⁻, more preferably, -NH-(CH₂)ₙ-N(CH₃)₃⁺A⁻, wherein n is an integer from 1 to 10, but preferably 2, 3 or 6. Examples of such compounds are the herein designated compounds **20-23:**
3¹-(aminoethylimino)-15-methoxycarbonylmethyl-rhodobacteriochlorin-13¹,17³-di(2-aminoethyl)amide (compound **20**);
Palladium 3¹-(aminoethylimino)-15-methoxycarbonylmethyl-rhodobacteriochlorin 13¹,17³-di(2-aminoethyl)amide (compound **21**);
3¹-(trimethylammoniumethylimino)-15-methoxycarbonylmethyl-rhodobacteriochlorin 13¹,17³-di(2-trimethylammoniumethyl)amide (compound **22**); and
Palladium 3¹-(trimethylammoniumethylimino)-15-methoxycarbonylmethyl-rhodobacteriochlorin 13¹,17³-di(2-trimethylammoniumethyl)amide (compound **23**).

The above compounds may be prepared as described in WO 2005/120573.

In accordance with the present invention, pharmaceutically acceptable salts of the (bacterio)chlorophyll compounds of formula II are used for PDT treatment of the eye, both salts formed by any carboxy groups present in the molecule, and a base as well as acid addition salts.

Pharmaceutically acceptable salts are formed with metals or amines cations, such as cations of alkali and alkaline earth metals or organic amines. Examples of metals used as cations are sodium, potassium, magnesium, calcium, and the like. Examples of suitable amines are N,N'-dibenzylethylenediamine, chloroprocaine, choline, diethanolamine, ethylenediamine, N-methylglucamine, and procaine (see, for example, Berge S. M., et al., "Pharmaceutical Salts," (1977) J. of Pharmaceutical Science, 66:1-19).

Acid addition salts of basic photosensitizers include salts derived from inorganic acids such as hydrochloric, nitric, phosphoric, sulfuric, hydrobromic, hydriodic, phosphorous, and the like, as well as salts derived from organic acids such as aliphatic mono- and dicarboxylic acids, phenyl-substituted alkanoic acids, hydroxy alkanoic acids, alkanedioic acids, aromatic acids, aliphatic and aromatic sulfonic acids, etc. Such salts thus include sulfate, pyrosulfate, bisulfate, sulfite, bisulfite, nitrate, phosphate, monohydrogenphosphate, dihydrogenphosphate, metaphosphate, pyrophosphate, chloride, bromide, iodide, acetate, propionate, caprylate, isobutyrate, oxalate, malonate, succinate, suberate, sebacate, fumarate, maleate, mandelate, benzoate, chlorobenzoate, methylbenzoate, dinitrobenzoate, phthalate, benzenesulfonate, toluenesulfonate, phenylacetate, citrate, lactate, maleate, tartrate, methanesulfonate, and the like. Also contemplated are salts of amino acids such as arginate and the like and gluconate or galacturonate (see, for example, Berge S. M., et al., "Pharmaceutical Salts," (1977) J. of Pharmaceutical Science, 66:1-19).

The acid addition salts of said basic compounds are prepared by contacting the free base form with a sufficient amount of the desired acid to produce the salt in the conventional manner. The free base form may be regenerated by contacting the salt form with a base and isolating the free base in the conventional manner. The free base forms differ from their respective salt forms somewhat in certain physical properties such as solubility in polar solvents, but otherwise the salts are equivalent to their respective free base for purposes of the present invention.

The base addition salts of acidic photosensitizers used in accordance with the invention are prepared by contacting the free acid form with a sufficient amount of the desired base to produce the salt in the conventional manner. The free acid form may be regenerated by contacting the salt form with an acid and isolating the free acid in the conventional manner. The free acid forms differ from their respective salt forms somewhat in certain physical properties such as solubility in polar solvents, but otherwise the salts are equivalent to their respective free acid for purposes of the present invention.

The negatively charged and the positively charges chlorophyll and bacteriochlorophyll compounds and pharmaceutically acceptable salts thereof used in accordance with the invention are highly water soluble and enable the preparation of aqueous formulation with no added surfactants.

The pharmaceutical composition used in accordance with the invention is an ophthalmic compositions, preferably a liquid e.g., a solution, suspension, and emulsion, or a gel that is topically applied to the eye of the patient and allowed to penetrate the cornea or the sclera before PDT is applied. The ophthalmic composition usually comprises inert pharmaceutically acceptable carriers and excipients that are well known in the art. The pharmaceutical composition may further contain one or more functional excipients, for example, for the purpose of mediating the depth of active agent penetration. Limiting the depth of photosensitizer penetration may be advantageous as it confines the treatment to the region of interest thereby minimizing adverse effects to adjacent tissues caused by the reactive oxygen species (ROS) formed upon illumination of the photosensitizer.

For preparation of pharmaceutical compositions, the photosensitizers may be lyophilized, for example, with mannitol, and the dry powder is directly solubilized in saline or any other pharmaceutically acceptable aqueous solution for topical application to a patient or for application on a sample *in vitro* target. The preparation of the compositions is carried out by techniques well-known in the art, for example as summarized in Remington: The Science and Practice of Pharmacy, Mack Publishing Co., Easton, Pa, 1990.

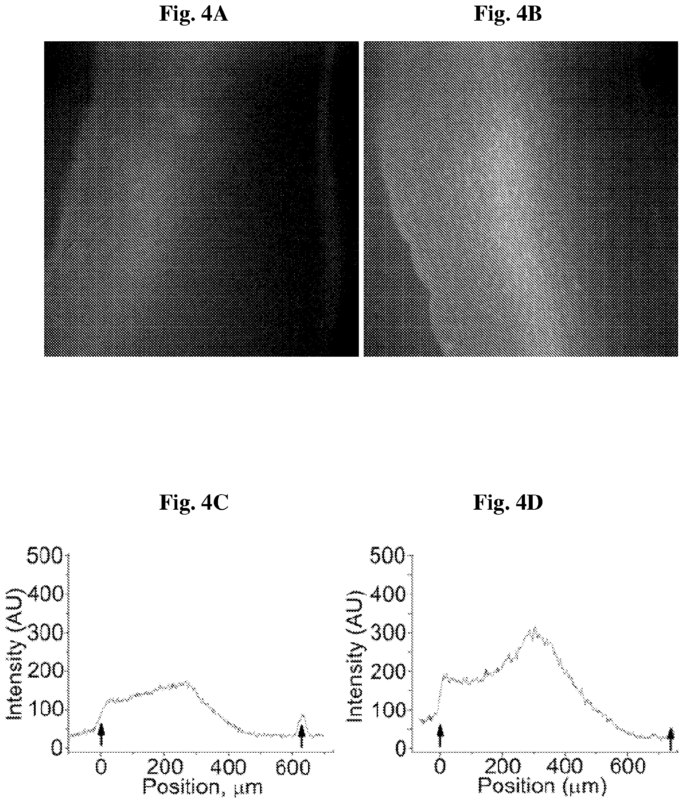

The present inventors found that penetration of several bactriochlorophyll derivatives into rabbit corneas and sclera that were exposed to the photosensitizer for 10 or 30 min was significantly deeper when the photosensitizer was applied without dextran-500 then when applied with the excipient. Fluorescence microscope measurements of rabbit cornea presented in **Figs. 4A-4H** indicate that the negatively charged bacteriochlorin derivative WST11 crosses half of the corneal stroma after 10 minutes of incubation and the entire stroma depth after 30 min of incubation in the absence of dextran. In contrast, application of WST11 with dextran-500 limited the penetration depth to the outer 1/3 of the corneal stroma after 10 minutes and to ∼50% after 30 minutes of incubation. Similar results were obtained when the penetration depth of other sulfonated Bchls derivative, namely compounds 1-7, was measured in the presence of dextran in the formulation.

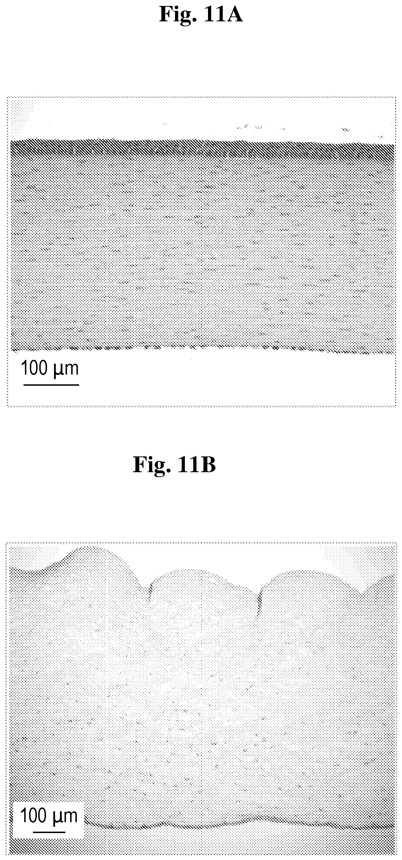

Treatment with WST11 (or with compounds 1-7) mixed with dextran-500 did not alter the stiffening effect as compared to WST11 without dextran. However, it greatly reduced the treatment's adverse effects. Clinically, the duration and extent of corneal edema and epithelial healing was significantly shortened. Also, epithelial haze that formed in some treated corneas, appeared only in the absence of dextran. However, the most important effect appears to be the protection of the endothelial layer and the reduced impairment of keratocytes in the treated corneas. This is clearly demonstrated in the histologic sections of rabbit eyes presented in **Figs. 11A-11C** herein. Histology tests after treatment with WST11 (or with compounds **1**-**7**) admixed with dextran 500, followed by NIR illumination showed reduction in keratocyte population in the anterior cornea with no damage to the endothelium.

This protection as well as the attenuated edema and shortened time of healing are probably related to the reduced depth of WST11 penetration and subsequently, the spatially limited effect of the photogenerated radicals. Surprisingly, while WST11 in saline penetrated half of the corneal stroma after 10 minutes of incubation, it produced less stiffening than WST11 applied with dextran after penetrating to a similar depth in the cornea. This observation may qualitatively correlate with the accumulation of WST11 at the cornea central layer and the possibility to generate a higher ROS concentration in this domain compared to the more diffused distribution of WST11 in the absence of dextran.

The protective role of dextran is probably maintained in the rivoflavin (RF)/UVA treatment known in the art for stiffening cornea. Although formulation of RF with dextran-500 has been claimed to help maintaining solution isoosmolarity (Letko et al., 2011), it was recently reported that the stromal penetration of RF-dextran solution (RF-D) is limited to the anterior 200 µm, even under prolonged exposure time (Søndergaard et al., 2010). This finding corroborates with data obtained by the present inventors and substantiates the conclusion that dextran limits the anterior penetration of the photosensitizer into the cornea and thereby protects the endothelium from phototoxic effect. The molecular nature of this effect is yet to be determind but it emerges that the polysachcaride forms together with the natural colagen a matrix, which acts as a barrier that prevents migration of the photosensitizer into deeper layers.

Dextran is a high molecular weight polymer of glucose, digestible and degradable, which accounts for its wide use as an excipient for ophthalmic formulations, for example, artificial tears and eye drops. Dextran fractions are derived from the partial acid hydrolysis of native dextran, they have various properties, and uses, and are supplied in molecular weights ranging from 1000 to 2 million. The molecular weight of the fraction is in most cases a key property. The designation dextran 5, dextran 10 and the like represents the mean molecular weight divided by 1000. Thus, Dextran 10 corresponds to a mean molecular weight of 10,000. In certain embodiments, the dextran fractions are selected from dextran 50, dextran 70, dextran 100, dextran 200, dextran 500, dextran 1000 and dextran 2000. The most preferable dextran fraction used for the preparation of the ophthalmic composition of the invention is dextran 500.

When a 20% dextran 500 is added to the formulation it increases the viscosity of the formulation to 179cP. An alternative approach to explain the reduced penetration depth of the photosensitizer when co-administered with dextran is increased viscosity of the formulation. The present inventors tested the effect of co-administering the photosensitizer with a solution of 65% glucose or 92% glycerol having a viscosity of 200cP, comparable to that of 20% dextran solution. It turned out that a solution of 65% glucose or 92% glycerol limited the penetration depth of the photosensitizer to the same extent as dextran.

Thus, in more preferred embodiments, the present invention provides a viscous ophthalmic composition for treatment of corneal thinning. According to these embodiments, the ophthalmic composition comprises a viscous agent as an excipient that enhances the viscosity of the formulation and thereby restricts or delays the active agent penetration into the cornea. Any viscous agent used in ophthalmic formulations is suitable for the purpose of the invention. Preferred functional excipients that function as viscous agents are biopolymers, preferably polysaccharides, more preferably natural polysaccharides that are highly biodegradable in the body as a result of natural biological processes. Natural polysaccharides are advantageous also due to their unique physico-chemical properties, relatively low cost and since they can be chemically modified to suit specific needs. Examples of polymeric viscous agents include dextran, scleroglucan and derivatives thereof, Gellan gum, Guar gum, methylcellulose (MC), polyvinyl alcohol, polyvinyl pyrrolidone, propylene glycol, polyethylene glycol, gelatin, carbomers and the like. Preferred viscous agents are dextran, and the exopolysaccharide hydrogels scleroglucan, particularly carboxylated derivatives thereof, Gellan gum and Guar gum.

Non-polymeric viscous agents that may be used in the ophthalmic formulation of the invention include glucose and glycerol.

Alternatively, the composition may be co-administered with a viscous, inert solution, such as, but not limited to, a solution of 20% dextran or 65% glucose or 92% glycerol.

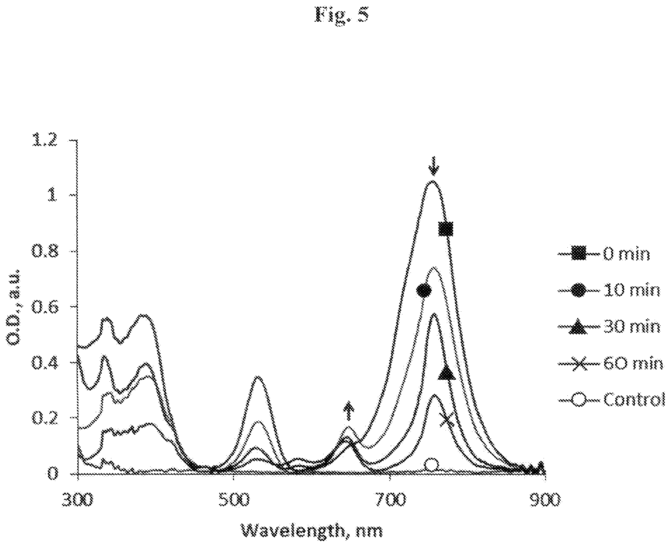

The optical application of the bacteriochlorophyll derivatives in rabbit cornea and sclera followed by illumination at 600-900 nm, and the trapping of photogenerated oxygen radicals in the cornea and sclera, induced photochemical reactions, probably polymerization reactions, that resulted in consistent corneal and scleral stiffening *ex vivo* and *in vivo.* The photochemical reactions of the bacteriochlorophyll derivatives applied to the eyes of rabbits could be obsereved as a continueous bleaching and spectral modifications during illumination (the bleaching is shown herein in **Fig. 5**).

For rabbit cornea, *ex vivo* incubation with WST11 for 30 minutes prior to illumination, increased the Young's modulus and the ultimate stress by 369% and 267%, respectively. Treatment with the same parameters performed *in vivo,* raised the Young's modulus and ultimate stress by 174% and 111%, respectively. The Young's modulus increased with the incubation time for the cornea treated in vivo.

For rabbit sclera, *ex vivo* incubation with WST11 for 30 minutes prior to illumination, increased the Young's modulus and the ultimate stress by 300% and 274%, respectively.

Thus, PDT of cornea and sclera pre-treated with water soluble (bactrio)chlorophyll derivatives enhanced the ultimate stress and the Young's modulus by at least a factor of 2, with no damage to endothelial cells. This treatment appeared safe in the animal models and may therefore be considered for clinical trials in keratoconus and corneal ectasia after refractive surgery.

Thus, the present disclosure provides a method for photodynamic therapy of eye diseases, disorders and conditions associated with corneal thinning or scleral stretching, comprising the steps of: (a) administering to an individual afflicted with corneal thinning or sclera stretching a photosensitizer which is a (bacterio)chlorophyll of the formula II as defined above or a pharmaceutical composition comprising same; and (b) irradiating the eye with light at the red or near infrared (NIR) wavelength.

The method is advantageous over RF/UVA treatment. UVA irradiation was shown by several reports to be toxic to corneal endothelial cells (Wollensak et al., 2004(a); Wollensak et al., 2004(b); Wollensak 2010(a)) and to result in a complete loss of keratocytes (Spoerl et al, 2007; Hafezi et al., 2009; Wollensak et al., 2010(b)) even when the penetration depth of the applied RF was limited.

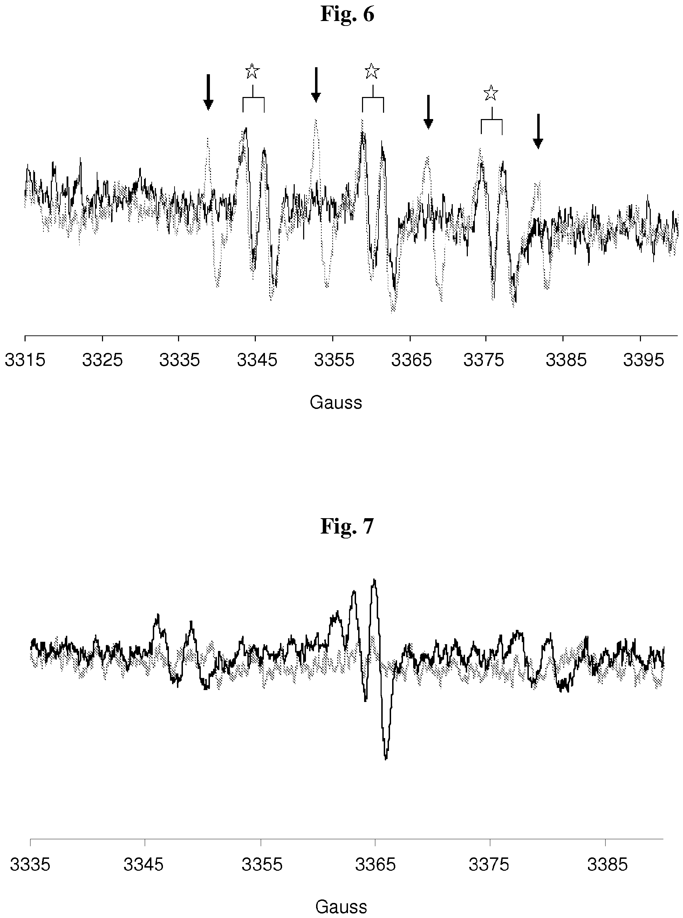

The mechanism involved in corneal stiffening by bacteriochlorophyll/NIR treatment may differ from that imposed by RF/UVA. Photoexcited water soluble bacteriochlorophyll derivatives such as WST11 generate superoxide and hydroxyl radicals with minimal traces of singlet oxygen which is the major ROS product of RF/UVA treatment (see **Fig. 6**).

In one embodiment, of the invention the corneal thinning diseases, disorders and conditions are selected from the group consisting of keratoconus, corneal ectasia caused by trauma, for example, post-laser-assisted in situ keratomileusis (LASIK) ectasia, post-photorefractive keratectomy (PRK) ectasia, post-infection ectasia, peripheral ectasia, atrophy, raised intraocular pressure or as a complication of photorefractive surgery in which the corneal stroma has been left thinner than 250 µm.

Other diseases and conditions associated with visual loss as a result of scleral and/or cornea anomalies that can be treated include rheumatoid condition of the cornea, degenerative myopia, regular myopia, scleral staphyloma, ocular hypertension glaucoma, low tension glaucoma and combinations thereof.

The combined treatment of (bacterio)chlorophyll photosensitizer and red or NIR irradiation of the eye may be used for preventing expected scleral weakening or corneal thinning in patients that intend to, or already underwent interventional procedures.

The amount of (bacterio)chlorophyll derivative to be administered for PDT therapy will be established by the skilled physician according to the experience accumulated with other Bchl derivatives used in PDT, and will vary depending on the choice of the derivative used as active ingredient, the condition to be treated, the mode of administration, the age and condition of the patient, and the judgement of the physician.

### EXAMPLES

### Materials and methods

***(i)*** ***Compounds.*** Palladium 3¹-oxo-15-methoxycarbomylmethyl-rhodobacteriochlorin 13¹-(2-sulfoethyl)amide monovalent salt (e.g. the dipotassium salt WST11) was supplied by STEBA Laboratories, Israel. Palladium 3¹-oxo-15-methoxycarbomylmethyl-rhodobacteriochlorin 13¹-(-sulfopropyl)amide divalent salt (e.g., the dipotassium salt compound 1); palladium 3¹-oxo-15-methoxycarbomylmethyl-rhodobacteriochlorin 13¹,17³-di-(2-sulfoethyl)amide divalent salt (e.g., the dipotassium salt compound **2**); palladium 3¹-oxo-15-methoxycarbomylmethyl-rhodobacteriochlorin 13¹,17³-di-(3-sulfopropyl)amide salt (e.g., the dipotassium salt compound 3); 3¹-oxo-15-methoxycarbomylmethyl-rhodobacteriochlorin 13¹-(2-sulfoethyl)amide salt (e.g., the dipotassium salt compound **4**); 3¹-oxo-15-methoxycarbomylmethyl-rhodobacteriochlorin 13¹-(-sulfopropyl)amide salt (e.g., the dipotassium salt compound **5**); 3¹-oxo-15-methoxycarbomylmethyl-rhodobacteriochlorin 13¹,17³-di-(2-sulfoethyl)amide divalent salt (e.g., the dipotassium salt compound 6) and 3¹-oxo-15-methoxycarbomylmethyl-rhodobacteriochlorin 13¹,17³-di-(3-sulfopropyl)amide divalent salt (e.g., the dipotassium salt compound **7**), were prepared as described in US 7,947,672. Other bacteriochlorophyll derivatives e.g., derivatives containing negatively charged groups or positively charged groups were prepared as described in US 7,947,672 and WO 2005/120573. Solutions of the bacteriochlorophyll derivatives were prepared in two forms: (a) in saline only, at a concentration of 2.5 mg/ml and pH adjusted to 7.3 (herein referred to as "Bchl-S solution" or "Bchl-S"); (b) 2.5 mg/ml of a bacteriochlorophyll derivative in saline with 20% dextran 500 (31392 Fluka, Switzerland) and pH adjusted to 7.3 (herein referred to as "Bchl-D solution" or "Bchl-D").
   Two forms of riboflavin (RF) solution were used: (a) 0.1% solution of riboflavin-5'-phosphate in saline, pH adjusted to 7.3 (herein referred to as "RF solution"); (b) commercial (Medio Cross, Germany) 0.1% solution of riboflavin-5'-phosphate in 20% dextran T-500, measured pH 6.8 (herein "RF-D solution" or RF-D").
***(ii)*** ***Light sources.*** (a) Diode laser with tunable output up to 1 W at 755 nm (CeramOptec, Germany); (b) LED system at 760 nm 2x18 mW (Roithner Lasertechnik, Austria); and (c) LED system at 370 nm 2x3 mW (Roithner Lasertechnik, Austria).
***(iii) Animal.*** New Zealand white (NZW) rabbits were housed and handled with ad libitum access to food and water at the Core Animal Facility of the Weizmann Institute of Science (Rehovot, Israel). All experimental procedures were approved by the Institutional Animal Care and Use Committee. All experiments were done in adherence to the ARVO Statement for the Use of Animals in Ophthalmic and Vision Research.

### Methods

### (a) Corneal studies

### Biomechanical testing of corneal stiffness

***(i)*** ***Sample preparation for ex vivo biomechanical studies of corneal stiffening.*** Eyes of rabbits (15-16 weeks old, weighting 3-4 Kg) were enucleated post mortem. Before enucleation, the 12 and 6 h position were marked on the sclera for preservation of subsequent cutting orientation. The corneas were de-epithelialized mechanically using a PKR scraper (Becton Dickenson, USA). Usually, 10 rabbits were assigned to bacteriochlorophyll/NIR treatment, herein the "test group" (for example WST11/NIR), and 2 rabbits were treated by RF/UVA (herein the "RF/UVA group") for comparison. In the test group, 10 eyes of 10 rabbits were immersed upside down for 30 minutes in test compound saline solution (Bchl-S) followed by NIR illumination (600-900 nm) with a diode laser at 10 mW/cm² for 30 minutes. The untreated contralateral eyes served as controls. In the RF/UVA group, RF-D solution was topically applied on 2 eyes of 2 rabbits, every 10 minutes, for 30 minutes, followed by UVA irradiation at 370 nm by a double diode at 3 mW/cm², for 30 minutes. After treatment, the corneo-scleral rings were removed and placed on paraffin hemisphere buttons with a matching shape to establish accurate sectioning without tissue stretching. Corneal strips, 4±0.2 mm in width, were cut from the epithelial side in a superior-inferior orientation, with a self-constructed double-blade cutter. The strips included 2 mm of sclera on both ends. Central corneal thickness was measured by ultrasonic pachymetry (Humphrey ultrasonic pachometer, USA). The corneal strips were then transferred to the biomechanical tester.
***(ii) Sample preparation for in vivo studies***

Twelve to twenty five weeks old rabbits (2.5-3 Kg weight) were used. They were anesthetized by intramascular (i.m.) injection of 35 mg/kg ketamine (Rhone Merieux, Lyon, France) and 5 mg/kg xylazine (Vitamed, Benyamina, Israel). Usually, 16 rabbits were assigned for bacteriochlorophyll treatment. After de-epithelialization, one eye of each rabbit was treated topically with a test compound in saline for 10, 20 and 30 minutes using an eye cap (12 mm in diameter), followed by NIR illumination for 10, 20 or 30 minutes (755 nm, 10 mW/cm²). The other eye served as untreated control. To prevent exposure of the limbal stem cells, the illuminated area was restricted by an aluminum foil mask with an 8 mm diameter central opening. To determine the role of dextran, 12 rabbits were divided into 4 treatment groups of 3 rabbits each: groups 1, the Bchl-S group, was treated with a saline solution of a bacteriochlorophyll derivative; group 2, the Bchl-D group, treated with a bacteriochlorophyll-dextran solution. Both groups were incubated with the test compound for 20-min, followed by NIR illumination (755 nm, 10 mW/cm²) for 30 minutes. Group 3, the RF group, was treated with rivboflavin without dextran, and group 4, the RF-D group was treated with RF-dextran solution (RF-D). Both group were incubated with RF or RF-D solutions for 30-minute, followed by UVA illumination (370 nm, 3 mW/cm²) for 30 minutes. An ophthalmic ointment containing dexamethasone 0.1%, neomycine and polymixin B (Maxitrol®, Alcon, Belgium) was applied on the treated eyes once daily for two weeks. Four weeks after the treatment the rabbits were sacrificed, and the corneoscleral rings were removed and placed on paraffin hemisphere buttons of matching shape to assure accurate sectioning without stretching the tissue. Corneal strips, 4±0.2 mm in width, were cut from the epithelial side, as described above in the *ex-vivo* section. The corneal strips were transferred to the biomechanical tester without delay.

### Drug accumulation, penetration and photobleaching

### (i) Photosensitizer overall accumulation

Eyes of rabbits were de-epithelialised mechanically immediately post mortem. For most studies, 6 rabbits were used, wherein 6 eyes of 6 rabbits were exposed to the bactriochlorophyll test compound for 10, 20 and 30 minutes (2 eyes for each test) using an eye cap, while the contralateral eyes were left untreated and served as controls. The corneas were removed and the central buttons of 8-mm diameter were punched out with a round trephine and placed onto the outer side of a polymethylmetacrylate cuvette in the area of light beem passage. Absorption spectra were recorded, and optical density (OD) at 600-900 nm (in most cases at 755 nm) was measured using V-570 spectrophotometer (Jasco, Japan).

### (ii) depth penetration studies

Eyes of rabbits were enucleated and deepithelialised mechanically post mortem. The eyes were exposed to Bchl-S solution using an eye cap for 5, 10, and 30 minutes and to Bchl-D solution for 10 and 30 min, in darkness. Controls: untreated eyes, and one eye treated with dextran-500 only for 30 min in darkness. Following this pretreatment, the corneas were briefly rinsed with saline and the central 8-mm buttons were trephined, removed, wrapped in aluminum foil and frozen on dry ice until further use. Central serial corneal sagittal slices (12 µm) were cut with a cryomicrotome, mounted on a microscope glass slide and stored frozen in -70°C until use. For measurement of fluorescence, the individual slices were placed on the microscope base and a photographic record was immediately taken. Fluorescence intensity of Bchl-S or Bchl-D treated corneas from 3 serial cryo sections was recorded at 760 nm, upon excitation at 740 nm, using a fluorescence microscope (BX61 Olympus, Japan) equipped with CCD camera (Cascade 512B, Roper Sci., USA) and a long pass filter of above 760 nm. The digital data was analyzed, using ImageJ software (NIH, USA).

### (iii) Photobleaching of photosensitizer

Corneas of eyes from euthanized rabbits were de-epithelialized. Usually, 5 rabitts were tested, and 8 of the 10 eyes were pretreated for 20 minutes with a bacteriochlorophyll derivative in saline (Bchl-S), using an eye cap. The solution excess was carefully tipped off with filter paper. The eyes were irradiated for the specified time duration: 0, 10, 30, and 60 min (two corneas per time frame). Additional two eyes were used as control. The corneas were removed, and the central buttons of 8-mm diameter were punched out with a round trephine. Absorption spectra were recorded using V-570 spectrophotometer (Jasco, Japan).

### Fluorescence spectroscopy following Bchl/NIR and RF/UVA treatment

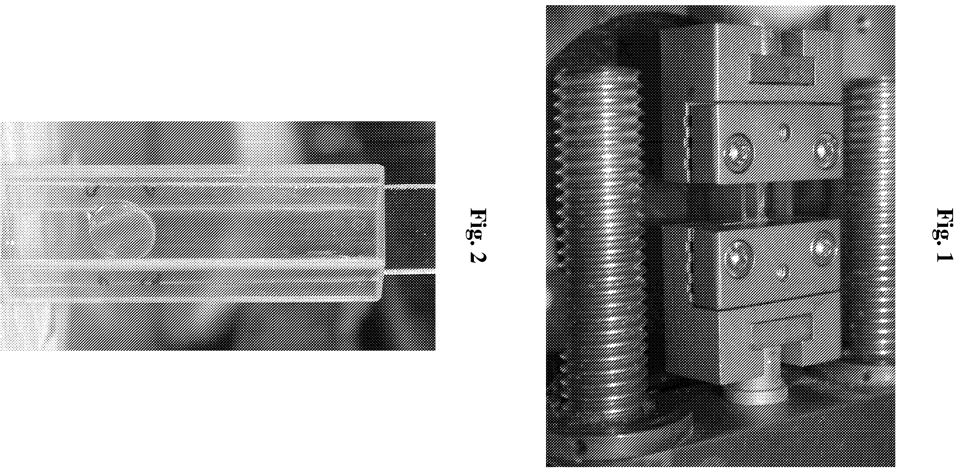

Rabbit eyes were enucleated post mortem. The corneal epithelium was de-epithelialised with a scraper. Usually, two eyes of two rabbits were immersed in Bchl-S or Bchl-D solutions for 30 min, followed by NIR irradiation (30 min). RF-D solution was applied on two eyes for 30 min, followed by UVA irradiation (30 min). Two eyes served as controls. The corneas were removed and the central 8-mm buttons were punched out with a round trephine. The buttons were mounted on a glass slide that was placed obliquely at 45° (Fig. 2) in a spectrofluorimeter (Varian-Cary Eclipse, USA). Excitation beam was set at 295, 315, 320, 328, 335 nm and corresponding emission spectra were read at 360 to 480 nm. Other readings were performed with excitation at 350 nm and emission at 380-480 nm, and excitation at 370 nm and emission at 400-700 nm. Excitation slit was 10 nm, emission slit ∼ 10 nm.

### Histology

Forthy eight hours or 1 weed after treatment with Bchl-S, Bchl-D, RF or RF-D, rabits were euthanized, and the eyes were immediately enucleated and fixated in Davidson's fixative (48 hour post treatment) or in 4% formaldehyde (1 week post treatment). Six-µm sections were prepared from either the whole eye, or corneas and stained with hematoxylin-eosin (H&E). The pathology of the corneal and retinal sections was examined. The sections were photographed with a digital video camera (Nikon DS-Ri1, Japan), mounted on a light microscope (E-800 Leica, Germany). Keratocyte and endothelial cell densities were counted in 2 areas of 2 central histological sections and calculated, using Image-Pro software (MediaCybernetics, MD, USA).

***Staining for apoptosis.*** For apoptosis detection, rabbits were euthanized one day post Bchl-S or Bchl-D treatment. Their corneas were removed, fixated in 4% formaldehyde and embedded in paraffin. Six-µm central sections were prepared from the corneas, placed on microscope slides and deparaffinized. Peroxidase-based terminal deoxyribonucleotidyl transferase-mediated dUTP-digoxigenin nick and labeling (TUNEL) assay was performed according to the manufacturer's instructions (Apop-Tag assay, Chemicon, Merck Millipore, Darmstadt, Germany).

***Endothelial staining.*** Rabbits underwent analysis of corneal endothelial damage 1 day post treatment, using alizarine red S and trypan blue staining as described in Spence and Peyman, 1976. The treatment included either a 20-minute pretreatment with Bchl-S or Bchl-D solutions, followed by 30-minute irradiation at 755nm (2 rabbits), or only a 30-minute irradiation at 760 nm (2 rabbits). Contralateral non-treated eyes served as controls.

### (b) Scleral studies

### Sample preparation for ex vivo biomechanical studies of scleral stiffening

Rabbit eyes were enucleated immediately post mortem and treated externally by applying an eye cap with a 2.5 mg/ml of a test compound in saline (Bchl-S) for 30 minutes on the superior or inferior sclera, followed by irradiation with NIR at 10 mW/cm² for 30 minutes. The opposite side of the sclera served as control. Scleral strips, 4 mm in width, were cut at the equatorial treated area and the opposite sclera, and stress-strain measurements were performed using a biomaterial tester.

### Impregnation measurements

### Ex-vivo impregnation:

Eyes of euthanized rabbits were enucleated, and the superior equatorial sclera (or inferior equatorial) was exposed to a test compound (Bchl-S) solution (2.5 mg/ml) for 10 minutes and 30 minutes using an eye cap of 10 mm diameter filled with the test compound. The inferior equatorial sclera (or the superior equatorial sclera, if impregnation was applied to the inferior equator) served as control.

### In situ impregnation:

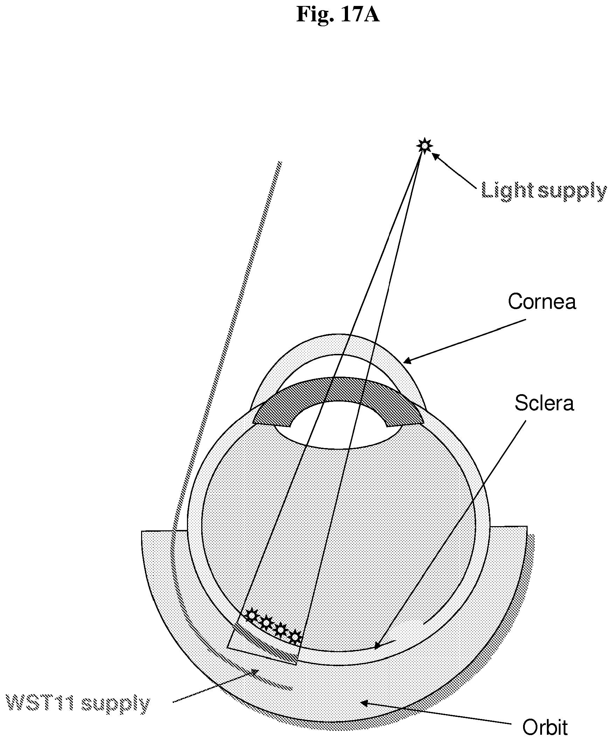

Test compound solution was applied in the supero-temporal or infero-nasal (alternating) quadrant of the rabbit by a curved plastic or metal glide with attached Merocel® sponge connected by a tube that ran along the glide outside. The glide was inserted through a conjunctival opening at the limbus (see **Figs. 17A-17B**). After placing the glide attached to the sclera, the tube was connected to a syringe or pump, and the test compound reservoir was injected to impregnate the sclera. Application of the photosensitizer was continued for 10 or 30 minutes. Afterwards, the glide with the Merocel® was withdrawn.

The following technique was performed after ex-vivo or in-situ impregnation of photosensitizer:
The treated scleral area and the opposing control sclera were trephined with a round skin trephine of 8-mm diameter and scissors and immediately frozen. Sagittal slices of 20 microns were dissected with a cryomicrotome at the central area of the scleral button. The fluorescence intensity at 755 nm was recorded upon excitation at 740 nm using a fluorescence microscope (Olympus, Japan).

### Illumination in vivo through the anterior segment.

NIR illumination was performed through the cornea and lens by a diode laser with a fundus contact lens (Volk pan-fundoscopic) or by mounting the optic fiber to an indirect ophthalmoscope, and using a +20 dioper lens applying the light on the sclera through the retina and pigment epithelium. The illumination intensity was adjusted accordingly due to the attenuation of the light.

After illumination, the rabbits were euthanized, their eyes were enucleated, and the treated scleral area and the opposing control sclera were trephined with a round skin trephine of 8-mm diameter and scissors. The scleral buttons were frozen immediately. Sagittal slices of 50 microns were performed with a cryomicrotome at the central area of the scleral button. Fluorescence spectroscopy was performed using a fluorescence microscope (Olympus, Japan), and fluorescence intensity at 755 nm was recorded upon excitation at 740 nm.

### (c) Biomechanical testing

The corneal and scleral strips were clamped horizontally, at a distance of 6 mm, between the jaws of a microcomputer-controlled biomaterial tester (Minimat, Germany) with a 200 Newton force cell (**Fig. 1**). Controlled tightening of screws was performed with a calibrated screwdriver at a preset torque of 9 cN.m (Torqueleader, England). The strain was increased linearly at a rate of 1.0 mm/min and was measured up to tissue rupture. Young's modulus was calculated by the testing machine. Ultimate stress and Young's modulus were expressed in Mega Pascal (MPa) units.

### (d) Electron Spin Resonance (ESR) spectroscopy

All ESR measurements were carried out using a Magnettech ESR Miniscope MS 100 Spectrometer (Germany), with a Microwave X-band Bridge. The ESR spectrometer operates at 9.3-9.55 GHz, 20 mW microwave power. ESR measurements were carried out at room temperature in glass capillaries or flat cells.

Samples of aqueous solutions of bacteriochlorophyll derivatives without or with dextran (Bchl-S and Bchl-D, respectively), and of riboflavin without or with dextran (RF and RF-D, respectively), were illuminated at 755 nm as described in Ashur et al, 2009. To each solution, the spin-trap α-(4-pyridyl *N*-oxide)-*N*-*tert*-butylnitrone (4-POBN, 65 mM) and ethanol (8%) were added. Controls contained illuminated 4-POBN in saline with/without dextran, and non-illuminated Bchl-S, Bchl-D, RF and RF-D solutions with/without 4-POBN.

***Ex-vivo ESR measurements of rabit corneas.*** Eyes of rabbits were enucleated post mortem. The corneas were de-epithelialized mechanically and corneal strips, 5 mm in width, were cut with a self-constructed double-blade cutter. The strips were immediately immersed for 30 min in solutions containing 4-POBN (65 mM) and ethanol (8%), and either Bchl-S or RF-D. Next, the strips were washed with saline and put in the flat-cells (0.5x5 mm³) for NIR/UVA illumination followed by ESR measurements with the aforementioned Miniscope.

### Example 1. WST11 uptake by rabbit corneal tissue

### (i) Overall accumulation of WST11 by the rabbit cornea

Six eyes of 3 euthanized rabbits were de-epithelialized mechanically immediately post mortem. Five of these eyes were exposed to WST11 in saline (WST11-S solution) for 10, 20 and 30 min, using an eye cap, and the optical absorption at 755 nm of the washed corneas was determined as described in Materials and Methods. One eye served as untreated control.

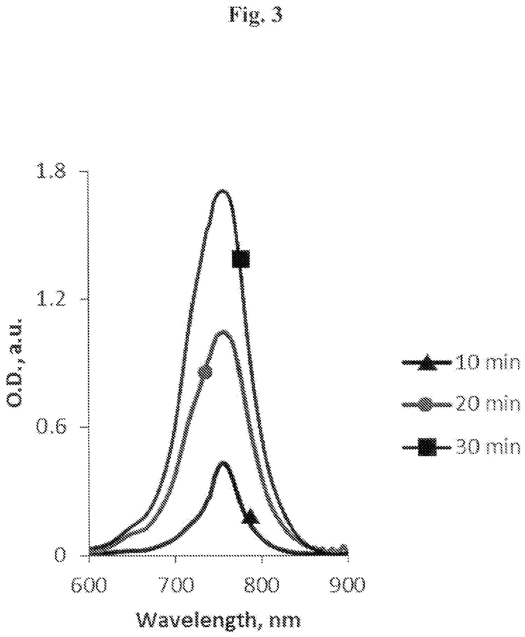

As shown in **Fig. 3****,** the optical density of the impregnated corneas increased with time up to a valuen of 1.71 OD units, without interference with the native spectrum of WST11.

### (ii) WST11 depth of penetration into the rabbit cornea

Upon topical application, the penetration and the depth of photochemical impact on the corneal tissue was determined for several bacteriochlorophyll derivatives. Results are provided herein for measurements with WST11.

The pentration depth of the photosensitizer deterimines the extent of cornea's tissue exposure to the photodynamic effect. In paticular, the deeper the penetration, the higher is the probability for endothelial impairment. To resolve the penetration depth of test componds following different times of incubation, the digitized fluorescence of dissected corneal discs was monitored using fluorescence microscopy as described in Materials and Methods.

The penetration depth of WST11 into the de-epithelized cornea, following various incubation times, was performed in the darkness in the absence or presence of dextran, using saline or dextran solutions of WST11 (WST11-S or WST11-D solutions, respectively) in 20 eyes of euthanized rabbits. Sagittal frozen sections mounted on glass slides were subjected to flourescence microscopy. The distribution of WST11 across the cornea was recorded photographically by fluorescence microscopy (excitation/emission 740/760 nm). The controls were 4 untreated eyes and 1 eye treated only with dextran-T 500 for 30 minutes.

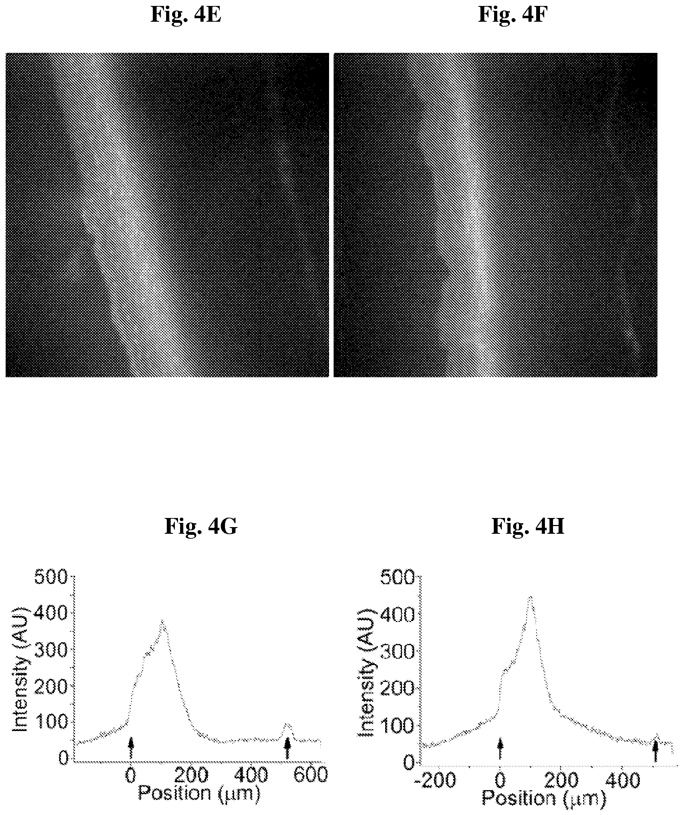

Following 10 minutes of exposure (of 2 eyes) to WST11-S solution, WST11 was evident through the entire outer half of the stroma (**Figs. 4A, 4C**). Further exposure (30 minutes; 5 eyes) resulted in diffused fluorescence all the way to Descemet's membrane (**Figs. 4B, 4D**). This treatment increased the stromal thickness from 360-380 µm to 600-730 µm. In contrast, application of WST11-D solution limited the penetration depth to the outer 1/3 (200 out of 520 µm) of the corneal stroma, at both 10 and 30 minutes exposure times (3 eyes and 5 eyes were tested, respectively) as shown in **Figs. 4E, 4G** and **Figs. 4F, 4H****,** respectively. Importantly, the fluorescence of WST11-D in the cornea, showed a relatively sharp front of drug migration that did not exceed the center of the stroma. However, at the depth of 200 µm the level of fluorescence was still higher for corneas following 30 minute exposure to WST11-D suggesting a higher accumulation level.

Comparable results for corneal immpregnation and penetration depth were obtained with saline and dextran solutions of compound **1-7.** (data not shown).

### Example 2. Photochemistry of WST11 in the cornea

### (i) Photobleaching of WST11 in ex-vivo treated corneas

It was previously described by the present inventors that generation of oxygen radicals upon illumination of certain bacteriochlorophyll derivatives such as WST11 in the absence of serum albumin, is accompanied by bleaching of the NIR tranisition and absorption increase at ∼645 nm due to the photochemical generation of a chlorophyll-like molecules (Ashur et al. 2009). Such changes are therfore important markers for the photochemical activity of such bacteriochlorophyll derivatives in situ. Hence, the changes in the optical absorption of several sulfonated bacteriochlorophyll derivatives during cornea illumination were monitored in de-epithelialized corneas of rabbits. Results obtianed for 10 corneas of 5 euthanized rabbits treated with WST11 in saline are presented herein.

Eight de-epithelialized eyes were retreated with WST11-S for 20 min using an eye cap, and the remaining 2 eys served as control. As shown in **Fig. 5****,** the 755-nm absorption of corneal WST11 diminished by 30, 50 and 75% after NIR illumination for 10, 30 and 60 min, respectively (downwarded arrow). In paralell, the absorption increased at 645 nm (upward arrow), typical to the corresponding chlorin derivative formation by the photochemical interaction of WST11 with molecular oxygen (Ashur et al., 2009)

### (ii) Oxygen radical formation by photoexcited WST11 in the cornea deduced from Electron Spin Resonance (ESR) Spectroscopy

Comparison of the ESR spectra of α-(4-pyridyl *N*-oxide)-*N*-*tert*-butylnitrone (4-POBN) in aqueous solution following photoproduction of reactive oxygen species (ROS) by WST11/NIR (black) and RF/UVA (light blue) and spin trapping by 4-POBN, are presented in **Fig. 6****.** The observed quartet is due to singlet oxygen trapping is present only in the RF/UVA spectra, while the sextet represents superoxide and hydroxyl radical formation (stars) in both WST11/NIR and RF/UVA.

ESR measurements of ROS trapped in the rabbit cornea treated by WST11/NIR is shown in **Fig. 7****.** The signals are similar to those observed in aqueous solutions with no traces of singlet oxygen. The signals generated by RF/UVA were at the noise level.

### Example 3. Corneal stiffening in response to WST11-S/NIR treatment

### Stress-strain measurements of ex-vivo treated eyes

Stress-strain measurements were conducted in 12 rabbits as described in Materials and Methods. Briefly, 10 eyes of 10 rabbits were treated by WST11/NIR, and 2 eyes of 2 rabbits were treated by RF-D/UVA. The contralateral eyes served as control.

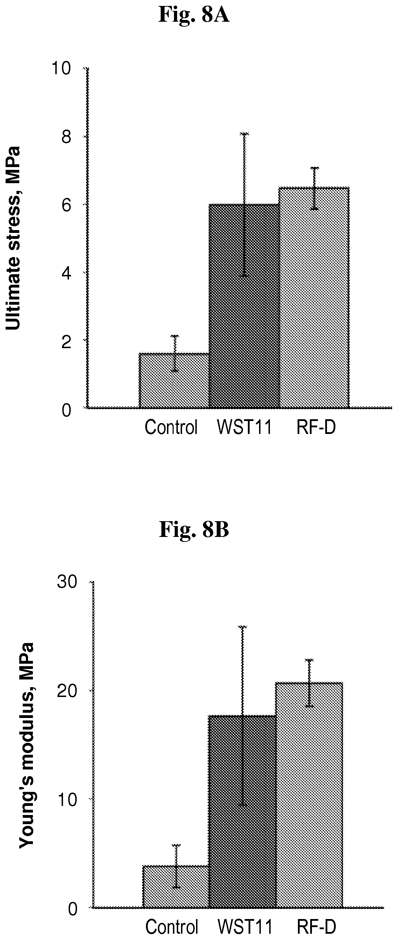

The stress-strain measurements following 30 min pre-incubation with WST11-S and then NIR illumination, showed a nearly 3-fold increase in the corneal stiffness (of all 10 eyes) as compared to the untreated control eyes (**Figs. 8A-8B**). There was a maximal increase of 267% in the ultimate stress (P < 0.0001) from a mean of 1.63 MegaPascal (MPa) without treatment to 5.98 MPa after treatment and an increase of 369% in Young's modulus (P < 0.0001), from a mean of 3.76 MPa without treatment to 17.65 MPa after treatment. The results are depicted in Table 1.

The mean ultimate stress in the two RF-D/UVA-treated corneas increased from 1.44 MPa without treatment to 6.46 MPa after treatment and in the mean Young's modulus from 3.28 MPa without treatment to 20.72 MPa after treatment.

Thus, stiffening due to WST11/NIR treatment appeared similar to that observed in the two RF-D/UVA treated corneas.

### Stress-strain measurements of in-vivo treated eyes

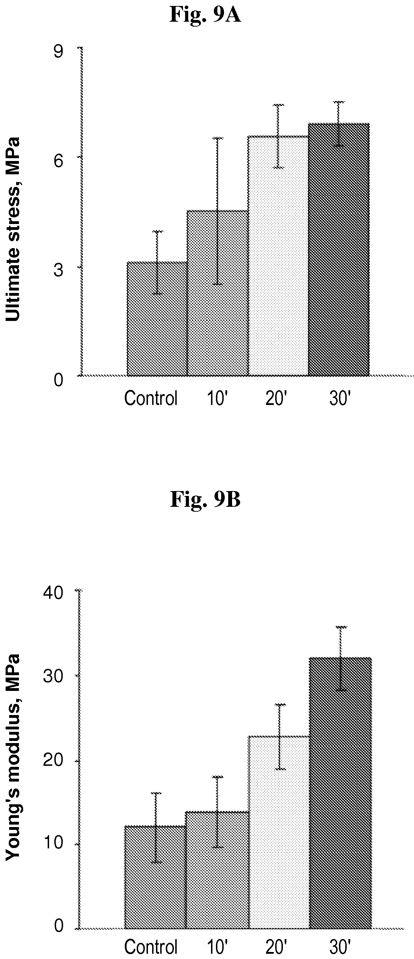

Corneas of 16 live rabbits were pretreated for 10 (n=4), 20 (n=6) and 30 (n=6) minutes with WST11-S (2.5 mg/ml). NIR illumination (755 nm, 10 mW/cm²) was then delivered for 30 minutes. The eyes were allowed to heal, and one month later the ultimate stress of the treated corneas was measured (as described in Material and Methods), and found to increase by 45, 113, and 126%, respectively, as compared to the non-treated eyes. The mean Young's modulus in WST11-S/NIR-treated corneas showed a 10, 79 and 173% increase for the same treatment times. Results are shown in **Figs. 9A-9B****,** and in Tables 1 and 2.

Corneas treated by WST11-S/NIR developed edema for one week after treatment. The corneal epithelial defect healed gradually after 10-14 days. After epithelial healing, the corneas regained transparency, with some corneas demonstrating epithelial haze.

**Table 1. rabbit cornea stiffening following WST11-S/NIR treatment**

| **Setting** | **Sample** | **Mean ultimate stress** | | **Mean Young's Modulus** | |
|---|---|---|---|---|---|
| | | **Intensity (MPa)** | **Increase % (*P* value)** | **Intensity (MPa)** | **Increase % (*P* Value)** |
| **Ex vivo** | Control: untreated fellow eyes n=10 | 1.63 ± 0.58 | | 3.76± 1.96 | |
| | WST11/NIR treated eyes n=10 | 5.98 ± 2.11 | 286 (<0.0001) | 17.65 ± 8.25 | 410 (<0.0001) |
| **In vivo 30 days** | Control: untreated fellow eyes n=6 | 3.06 ± 0.67 | | 11.7 ± 2.6 | |
| | WST11/NIR treated eyes n=6 | 6.91 ± 0.53 | 111 (0.0049) | 32 ± 3.6 | 174 (0.0035) |

**Table 2. Effect of WST11 incubation time on rabbit corneas stiffening**

| | **Sample** | **Mean ultimate stress** | | **Mean Young's Modulus** | |
|---|---|---|---|---|---|
| | | **Intensity (MPa)** | **Increase % (*P* value)** | **Intensity (MPa)** | **Increase % (*P* Value)** |
| **IIn vivo** | Control: untreated fellow eyes n=10 | 3.12 ± 0.73 | | 12.69 ± 3.31 | |
| | WST11 10 min treated eyes n=4 | 4.51 ± 1.85 | 45 (0.1446) | 13.9 ± 3.9 | 9 (0.183) |
| | WST11 20 min treated eyes n=6 | 6.66 ± 0.67 | 113 (<0.0001) | 22.7 ± 3.82 | 79 (0.0028 |

Notably, the in-vivo rabbits were 12 weeks old at the time of treatment, and 16 weeks old when sacrificed, while the ex-vivo group rabbits were sacrificed at the age of 12 weeks. The aging resulted in a higher baseline Young's modulus and ultimate stress values of the control eyes **(Table 1)** that probably accounts for the gap between the parameters achieved in the two settings (Knox et al., 2010; Elsheikh et al., 2007).

Stress-strain measurements of rabbit cornea where conducted, both ex vivo and in vivo as described above, but exposing the eyes to saline solutions of Compound **1-3** for the indicated times, followed by NIR illumination. The ultimate stress and Young's modulus values obtained for these compounds were similar to those obtained with WST11-S (data not shown).

### Example 4. In vivo cornea treatment by WST11-D/NIR

To determine the role of dextran, the photochemical treatment with a formulation of WST11 2.5 mg/ml containing 20% dextran T-500 (WST11-D) was examined. Six rabbits were examined: 3 rabbits were pretreated for 20 min with WST11-S solution and 3 with WST11-D solution, followed by NIR illumination for 30 minutes. Additional 4 rabbits were treated with RF (n=2) or RF-D (n=2) for 30 minutes, followed by 30-min UVA irradiation (370 nm, 3mW/cm²).

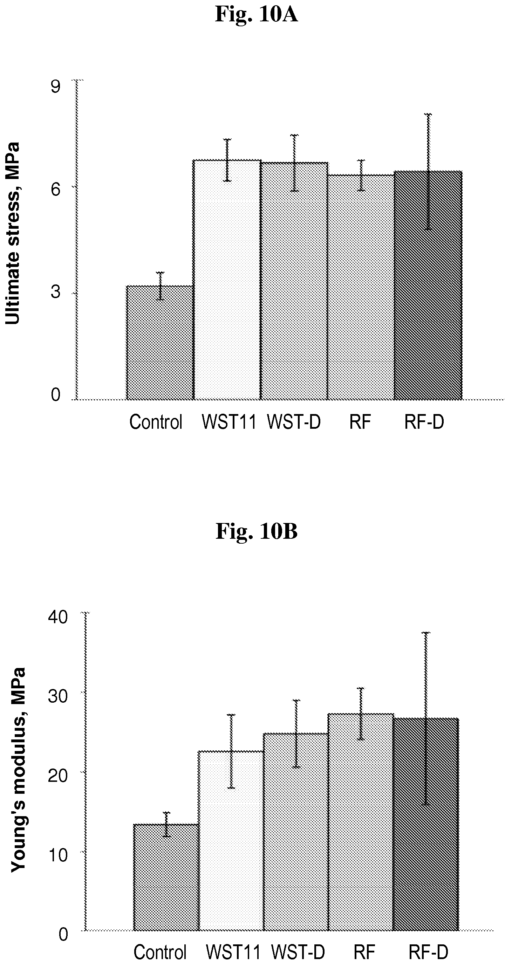

As shown in **Figs. 10A-10B**, application of WST11-S or WST11-D did not appear to affect the approximately two-fold increase in both the mean ultimate stress and Young's modulus, as compared with untreated corneas of fellow eyes.

However, the important finding is that treatment with WST11 significantly reduced the extent and duration of the edema and epithelial defect compared to RF-D/UVA treatment. Corneal edema cleared after 5 days, and the epithelium healed within 7-9 days without haze development. In the RF and RF-D treated corneas, the epithelial defect healed after 4 days. In the RF-D group the edema resolved after 4 days with recovery of transparency, whereas in the RF group the edema persisted for 6 days, followed by 2 days of central epithelial haze.

### Example 5. Endothelial and keratocyte response to WST11-S/NIR or WST11-D/NIR treatment

The endothelial and keratocyte response of rabbits' eyes to incubation with saline or dextran solutions of WST11 and Compouns **1-7,** followed by NIR illumonation were studied. The effects of these various bactriochlorophyll solutions were practically the same. Detailed results are provided herein for measurments with WST11.

One rabbit underwent treatment with WST11 in saline (WST11-S; 20-minute incubation, 30-minute irradiation at 755 nm). Six rabbits underwent treament with WST11-D (n=4, 20-minute incubation, 30-minute irradiation at 755 nm), or with RF-D (n=2, 30-minute incubation, 30-minute irradiation at 370 nm) as described above (*In-vivo studies* section). Contralateral eyes were used as control.

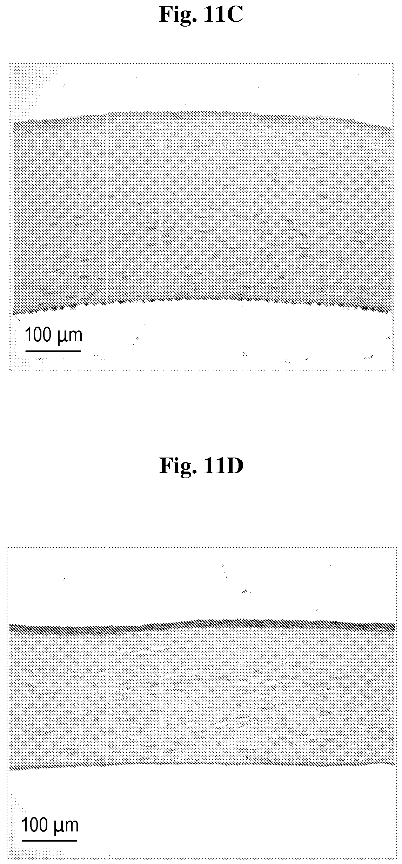

Histological examination of the corneas two days after WST11-S/NIR treatment showed marked edema (cornea swelling to 890 µm), and a reduced number of keratocytes throughout the stroma, more pronounced in the anterior half (see **Fig 1B**). A honeycomb-like lacunar hydration pattern, containing keratocytes or keratocyte debris, was present. However, the endothelial cell layer appeared intact and did not differ from the control **(****Fig. 11A****).** There was no statistical difference in the endothelial counts between treatment and control (P=0.47). In contrast, corneas treated with WST11-D showed minimal corneal edema, absence of the epithelium in the central area and a statistically significant reduction in the number of keratocytes (481±121 cells/mm² in the treated corneas, as compared to 1060±210 cells/mm² in controls, P<0.0001) limited to the outer half of the stroma **(****Fig. 11C****).** There was no evidence of damage to the endothelium in comparison to the control, both after vital (data not shown) and H&E staining **(****Fig. 11A****).** One week after treatment, the histological sections showed shrinkage of the anterior stroma to 250 µm (in addition to the stromal compaction to 340 µm that occurred following formaldehyde fixation of control samples), loss of keratocytes in the anterior 1/3 of the stroma (80µm) with epithelial healing, but no endothelial damage **(****Fig. 11D****).** H&E staining of the retina two days after WST11-D/NIR treatment did not show any morphological changes compared with control (data not shown).

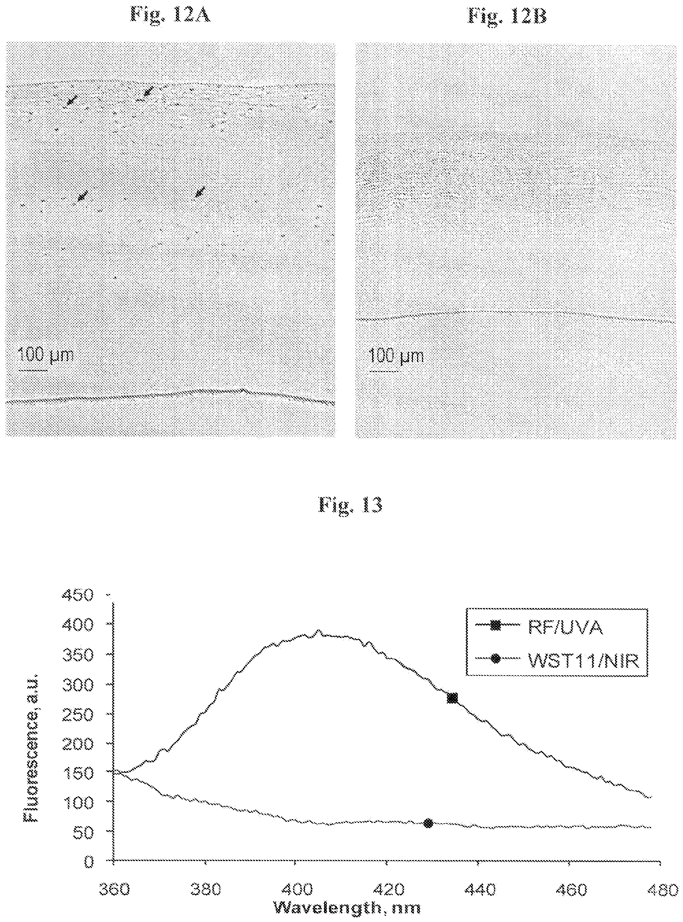

Apoptosis was examined using peroxidase-based terminal deoxyribonucleotidyl transferase-mediated dUTP-digoxigenin nick and labeling (TUNEL) assay. One day postoperatively, TUNEL-positive keratocytes were detected in the outer ½ of anterior stroma of treated corneas, as shown in **Fig. 12B**. No staining for TUNEL was observed in the posterior stroma the endothelium was absent and there was edema of the central stoma, as in the control corneas **(****Fig. 12A****).**

### Example 6. Fluorescence spectroscopy of rabbit corneas

Eight raqbbit eyes were enucleated post mortem and corneal endothelium was deepithlialized. Three eyes were immersed in SWT11-S solution and 3 eyes in SWT11-D solution for 30 nimutes, followed by NIR illumination. Two eyes were treated with RF-D solution for 30 minues, followed by UVA irradiation. Contralateral eyes served as control. Fluorescence of segments of the corneas was measured as described in Materials and Methods.

Excitation of the RF-D/UVA treated corneas at 320 nm, generated a clear emission signal at 405 nm that probably corresponded to the fluorescence of dityrosine, a known signature of cross linking (Kato et al., 1994). Such emission was absent in the WST11-S/NIR and WST11-D/NIR treated corneas, and in the controls as shown in **Fig. 13****.** Excitation at any other wavelength did not provide any significant change in the cornea emission profile.

### Example 7. Palladium based measurement of systemic absorption of topically applied WST11-D

Six rabbits were anesthetized as described in the Materials and Methods. After deepithelialization, one cornea of each rabbit was treated with WST11-D 2.5 mg/ml (n=3) and 10 mg/ml (n=3) for 20 minutes using an eye cap. Blood samples (∼0.5 ml) were taken from the ear vein before application (time 0), and at 10, 20, 40 and 60 minutes after application began, placed in pre-weighed polyethylene 1.5 ml test tubes, weighted and lyophilized. The dry samples were digested with nitric acid, and Pd concentrations were determined by inductively-coupled plasma mass spectrometry (ICP-MS) using a set of Pd standards (High-purity Standards, USA) as previously described (Mazor et al, 2005).

ICP-MS measurements of blood samples drawn from rabbits during and after topical application of WST11-D could not detect any significant levels of Pd⁺², as an evidence for no penetration of the drug into the circulation of the treated animals at all measured time points.

### Example 8. Thermographic analysis of the corneal surface

Three rabbits were treated with WST11-D for 20 minutes, followed by NIR illumination (755 nm, 10 mW/cm²) for 30 minutes. Temperature measurements on the corneal surface were performed during WST11-D/NIR treatment using an IR thermocamera (Thermal imager InfRec R300, NEC Avio Infrared Technologies Co., Ltd., Tokyo, Japan) with thermal resolution 0.05°C, temperature accuracy of ±1°C and a spatial resolution of 120 µm. Images were recorded before irradiation, every 7 minutes during irradiation and at the conclusion (last seconds) of irradiation. Selected thermographic images were processed with InfReC Analyzer NS9500 Lite (NEC Avio Infrared Technologies Co., Ltd., Tokyo, Japan).

A constant temperature gradient from T=32°C at the corneal center to T=37.5°C at the limbal periphery was measured before, during and after treatment. Deviations of less than 1°C were observed throughout the whole procedure (data not shown).

### Example 9. Transepithelial delivery of WST11 into rabbit cornea.

Permeability of the cornea to drugs is clinically important because it is the major factor determining the efficacy of topically applied ophthalmic formulations. To study the transepithelial penetration of the ophthalmic formulations of the invention, the delivery of SWT11 was assessed in the presence of a known transepithelial permeability enhancing excipient comprising benzalkonium chloride solution containing NaCl.

Two rabbits were anesthetized and treated as follows:
In one eye of each rabbit, the epithelium was removed, and the central corneal area was incubated with a 2.5 mg/ml solution of WST11 in 0.9% sodium chloride, pH 7.3, using eye cap during 20 minutes. In the contralateral eye the epithelium was left intact and the central corneal area was incubated with a 2.5 mg/ml solution of WST11 in 0.44% sodium chloride containing 0.02% benzalkonium chloride, pH 7.3, using eye cap, during 20 minutes, and then the epithelium was removed.

Following incubation with the photosensitizer, the animals were euthanized, central corneal discs of 8 mm in diameter were removed, and photosensitizer's overall accumulation was estimated by recording absorption spectra and measurement of OD at 755 nm as described in Materials and Methods.

Accumulation of WST11 in the de-epithelialized corneas resulted in optical density (OD) of 0.86 in one rabbit and 0.75 in the second rabbit. transepithelial corneal accumulation resulted in OD of 0.12 in the first rabbit which is 14% of WST11 accumulated in the de-epithelialized cornea, and 0.16 in the second rabbit, which is 21% of the photosensitizer that accumulated in the de-epithelialized cornea.

Thus, under the condition applied above, the amount of photosensitizer delivered by transepithelial corneal incubation can be ∼1/6 of the amount accumulated in de-epithelized cornea.

### Example 10. Penetration depth in cornea of WST11 in aqueous 65% sucrose solution

Aqueous 65% sucrose solution has similar viscosity as 20% dextran T-500 (∼175 cP). The penetration depth of topically applied photosensitizer in a formulation comprising 65% sucrose was tested in corneas of rabbits. The results described herein were obtained for WST11 in 65% sucrose solution.

Two rabbits were anesthetized, their corneas were de-epithelized and one eye of each rabbit was treated with a 2.5 mg/ml solution of WST11 in aqueous 65% sucrose solution, pH 7.3, using eye cap, during 10 minutes, and the contralateral eye was similarly treated but for 30 minutes. Then, the animals were euthanized, central corneal discs of 8 mm in diameter were removed, deep frozen and cut for sagittal slices as described in Materials and Methods.

After 10-min. incubation the photosensitizer penetrated to ∼1/3 of the outer stroma, and after 30-min. incubation to ∼1/2 of the outer stroma, in both animals (data not shown). Thus, a 65% sucrose solution can be applied as effectively as dextran for limiting photosensitizer penetration into cornea.

### Example 11. WST11 depth of penetration into the rabbit sclera

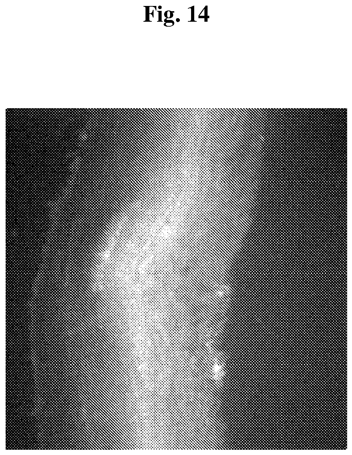

The depth of penetration of WST11 into rabbit sclera was assessed by fluorescence microscopy as described in Materials and Methods. The results shown in **Fig. 14** indicate that WST11 applied ex vivo using eye cap and in-situ using Merocel® sponge penetrated into sclera tissue.

### Example 12. Biomechanical testing of rabbit sclera treated ex vivo with WST11-S and external NIR illumination

Stress-strain tests of sclera of enucleated rabbits' eyes were performed following treatment with WST11 (2.5 mg/ml) as described in Materials and Methods. After photosensitizer impregnation, the sclera was illuminated by applying the laser beam directly onto the treated area (external illumination), using a flat optical fiber that run along a curved plastic or metal glide as shown in **Fig 17A** . Strips of the sclera were cut and tested.

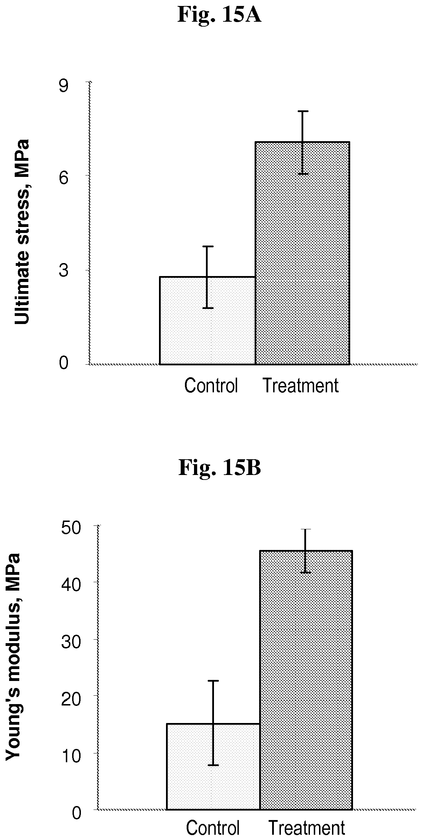

The measurements demonstrated a significant increase in the stiffness of the treated sclera. The mean maximal stress in the control sclera was 2.77 ± 0.99 MPa. The mean maximal stress in WST11/NIR treated sclera was 7.6 ± 0.99 MPa (174% increase). The mean Young's modulus in control sclera was 15.2 ± 7.5 MPa. The mean Young's modulus in WST11/NIR treated sclera was 45.6 ± 3.9 MPa (200% increase). The results are shown in **Figs. 15A-15B****.**

### Example 13. Biomechanical testing of rabbit sclera treated ex vivo with WST11-S and NIR illumination through the anterior eye segment.

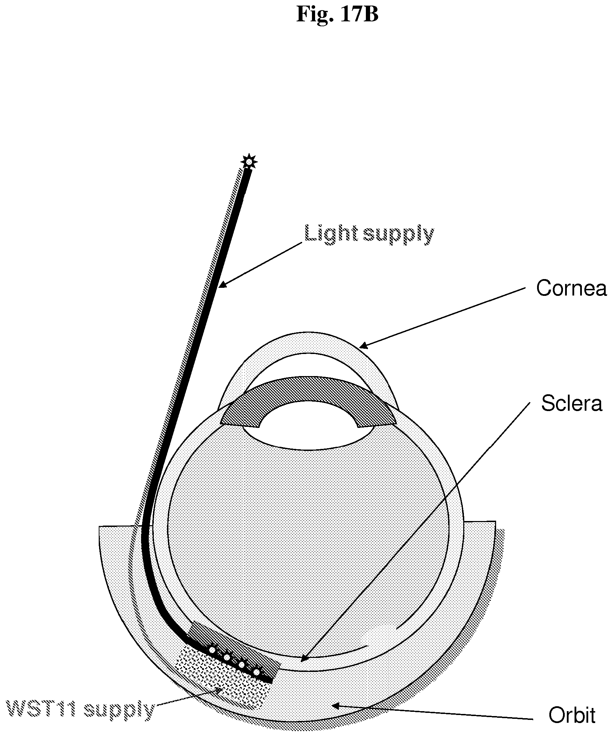

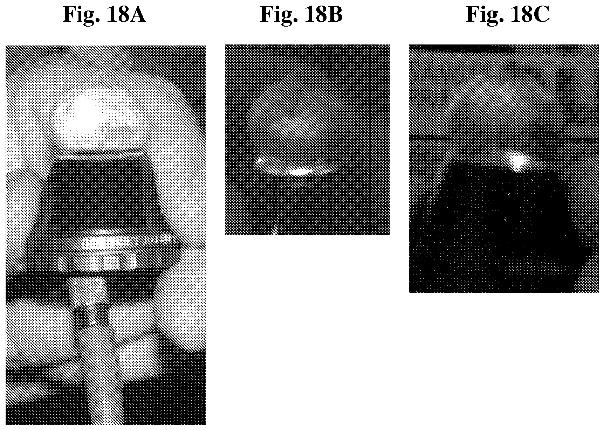

The inventors hypothesized that since tissues are fairly transparent to illumination at 755-820 nm, it should be possible to deliver sufficient illumination to the posterior sclera by NIR illumination through the cornea and anterior segment of the eye using either the three mirror fundus lens apparatus shown in **Figs. 18A-18C****,** or by with an illuminating the cornea through an indirect ophthalmoscope using the apparatus described in **Fig. 17B****.**

Six eyes of three rabbits were enucleated. The posterior eye area up to the equatorial line was incubated using an eye cap with 2.5 mg/ml solution of WST11 in saline pH 7.3 for 20 minutes and then illuminated by NIR illumination through the anterior cornea using a diode laser (755 nm, 0.5 W) and three mirror fundus lens **(****Figs. 18A-18C****)** for 30 minutes. Strips of sclera were cut and tested as described in Materials and Methods.

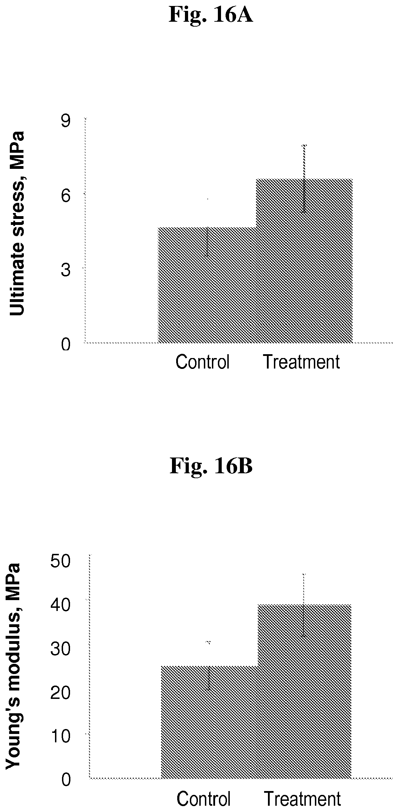

The measurements demonstrated an increase in the stiffness of the treated sclera. The mean maximal stress in control sclera was 4.65±1.15 MPa. The mean maximal stress in WST11/NIR treated corneas was 6.59±1.32 MPa (42% increase). The mean Young's modulus in control sclera was 25.25±5.30 MPa. The mean Young's modulus in WST11/NIR treated sclera was 38.83±6.89 MPa (54% increase). The results are shown in **Figs. 16A-16B**

### Example 14. In vivo sclera stiffening with illumination through the anterior segment

*In vivo* scleral stiffening while avoiding retinal and eye orbit toxicity is achieved as follows:
Impregnation of the photosensitizer (a Bchl derivative) into the sclera is performed by inserting a sub-tenon glide through openings in the limbal conjunctiva in 4 quadrants. The inner distal 10 mm of the glide is porous and contains a reservoir of the test compound. This reservoir is connected by a tube that runs outside along the glide. After placing the glide attached to the sclera, the tube is connected to a syringe or pump, and the test compound reservoir is injected to impregnate the sclera. The sensitizer concentration and time of exposure is *a priori* optimized via *ex-vivo* measurements of enucleated rabbit eyes using fluorescence spectroscopy of frozen histological sclera sections as described in Example 11 above. If required, dextran or sucrose solutions are used to optimize penetration depth.

The retina safety depends on the traversing light energy. In preclinical and Phase II clinical trials of vascular targeted PDT with WST11, the inventors have shown that illumination of retina by 50-70 J (82-120 sec at 600 mW/cm²) proved safe. Consequently, the treatment energy is set at the range of 5-12 J (10-20 mW/cm² for 20-10 min) for which minimal morbidity of the retina is expected.

Using the optimized parameters Bchl-S or Bchl-D is applied as described above followed by 755-nm frontal illumination at an optimized laser power output in the range of 20-250 mW to deliver an *a priori* optimized light intensity at the posterior segment of the eye. The illumination is performed using a NIR laser with He-Ne aiming beam through Goldmann 3 mirror fundus lens or NIR laser attached to an indirect ophthalmoscope with He -Ne aiming beam. One month after the treatment the rabbits are euthanized and the eyes are enucleated. Treatment success is assessed by strain-stress measurements of scleral strips excised from the superior and the inferior sclera of the treated eyes compared to the matching sclera of non-treated fellow eyes.

### Example 15. In vivo sclera stiffening with illumination through the anterior segment

*In vivo* scleral stiffening while avoiding retinal and eye orbit toxicity is achieved as described in Example 14, but the photosensitizer delivered to the sclera via a Merocel® sponge attached to the glide inserted through openings in the limbal conjunctiva, instead of using a porous glide to deliver the photosensitizer.

### Example 16. In vivo sclera stiffening with illumination through the anterior segment

*In vivo* scleral stiffening is achieved as described in Example 14, but illumination is applied externally by insertion of a glide connected to optical fiber through the limbal conjunctiva openings as illustrated in **(****Fig. 17B****).** This glide is designed to spread a diffuse light directed towards the sclera; it is opaque in its orbital aspect. The illumination glide is connected to a NIR laser or LED source to deliver the NIR light. Both impregnation and illumination glides are marked with a millimetric ruler to verify insertion depth. Position of illumination glide can be monitored during light delivery by fundoscopy or fundus imaging.

An alternative glide that is used for *in vivo* scleral stiffening is a glide designed as a combined unit for both drug and illumination delivery.

### REFERENCES

Ashur I, et al. 2009, "Photocatalytic generation of oxygen radicals by the water-soluble bacteriochlorophyll derivative WST11, noncovalently bound to serum albumin", JPhys Chem., 113:8027-37.
Avila MY, Navia JL, 2010, "Effect of genipin collagen crosslinking on porcine corneas", J Cataract Refract Surg., 36:659-664.
Berdugo M et al. 2008, "Evaluation of the new photosensitizer stakel (WST-11) for photodynamic choroidal vessel occlusion in rabbit and rat eyes", Inv Ophthalmol Vis Sci. 49:1633-1644.
Bourges JL et al., 2006, "PDT of corneal neovessels using a new hydrosoluble photosensitizer (WST11)", Acta Ophthalmol Scand., 84(S 239:41): 352.
Brandis A, Mazor O, Neumark E, Rosenbach-Belkin V, Salomon Y, Scherz A. 2005, "Novel water-soluble bacteriochlorophyll derivatives for vascular-targeted photodynamic therapy: synthesis, solubility, phototoxicity, and the effect of serum proteins", Photochem Photobiol., 81:983-993.
Elsheikh A, Wang D, Brown M, Rama P, Campanelli M, Pye D, 2007, "Assessment of corneal biomechanical properties and their variation with age", Curr Eye Res., 32:11-19.
Hafezi F, Kanellopoulos J, Wiltfang R, Seiler T. 2007, "Corneal collagen crosslinking with riboflavin and ultraviolet A to treat induced keratoectasia after laser in situ keratomileusis" J Cataract Refract Surg., 33:2035-2040.
Hafezi F, Mrochen M, Iseli HP, Seiler T. 2009, "Collagen crosslinking with ultraviolet-A and hypoosmolar riboflavin solution in thin corneas", J Cataract Refract Surg., 35:621-624.
Kato Y, Uchida K, Kawakishi S. Aggregation of collagen exposed to UVA in the presence of riboflavin: a plausible role of tyrosine modification. Photochem Photobiol. 1994;59:343-349.
Knox Cartwright NE, Tyrer JR, Marshall J, 2010, "Age-related differences in the elasticity of the human cornea", Invest Ophthalmol Vis Sci., 52:4324-4329.
Lepor H. 2008, "Vascular targeted photodynamic therapy for localized prostate cancer", Rev Urol., 10:254-261.
Letko E, Majmudar PA, Forstot SL, Epstein RJ, Rubinfeld RS, 2011, "UVA-light and riboflavin-mediated corneal collagen cross-linking", Int ophthalmol clin., 51:63-76.
Liu K et al. 2004, "Superoxide, hydrogen peroxide and hydroxyl radical in D1/D2/cytochrome b-559 Photosystem II reaction center complex", Photosynthesis Research., 81:41-47.
Mazor O. et al. 2005, "WST11, A novel water-soluble bacteriochlorophyll derivative; cellular uptake, pharmacokinetics, biodistribution, and vascular targeted photodynamic activity against melanoma tumors", Photochem Photobiol., 81:342-345.
Moore CM, Pendse D, Emberton M. 2009, "Photodynamic therapy for prostate cancer-a review of current status and future promise", Nat Clin Pract Urol., 6:18-30.
Raiskup-Wolf F, Hoyer A, Spoerl E, Pillunat LE. 2008, "Collagen cross-linking with riboflavin and ultraviolet-A light in keratoconus: long-term results", J Cataract Refract Surg. 34:796-801
Søndergaard AP, Hjortdal J, Breitenbach T, Ivarsen A, 2010, "Corneal distribution of riboflavin prior to collagen cross-linking", Curr Eye Res., 116:121-135.
Spence DJ, Peyman GA. 1976, "A new technique for the vital staining of the corneal endothelium", Inv Ophthalmol Vis Sci., 15: 1000-1002.
Spoerl E, Mrochen M, Sliney D, Trokel S, Seiler T. 2007, "Safety of UVA-riboflavin cross-linking of the cornea", Cornea, 26:385-389.
Trachtenberg J et al. 2007, "Vascular targeted photodynamic therapy with palladium-bacteriopheophorbide photosensitizer for recurrent prostate cancer following definitive radiation therapy: assessment of safety and treatment response", J Urol., 178:1974-1979.
Vakrat-Haglili Y et al., 2005, "The microenvironment effect on the generation of reactive oxygen species by Pd-Bacteriopheophorbide", JAm Chem Soc., 127:6487-6497.
Wollensak G, 2010(a) "Histological changes in human cornea after cross-linking with riboflavin and ultraviolet A", Letter to the editor. Acta Ophthalmol. 88:e17-18.
Wollensak G, Aurich H, Wirbelauer C, Saadettin S. 2010(b), "Significance of the riboflavin film in corneal collagen crosslinking", J Cataract Refract Surg., 36:114-120.
Wollensak G, Spoerl E, Reber F, Pillunat L, Funk R. 2003(c), "Corneal endothelial cytoxicity of riboflavin/UVA treatment in vitro", Ophthalmic Res., 35:324-328.
Wollensak G, Spoerl E, Reber F, Seiler T. 2004(a) "Keratocyte cytotoxicity of riboflavin/UVA-treatment in vitro", Eye, 18:718-722.
Wollensak G, Spoerl E, Seiler T. 2003(a) "Riboflavin/ultraviolet-A-induced collagen crosslinking for the treatment of keratoconus", Am J Ophthalmol, 135:620-627.
Wollensak G, Spoerl E, Wilsch M, Seiler T. 2003(b) "Endothelial cell damage after riboflavin-ultraviolet-A treatment in the rabbit", J Cataract Refract Surg. 29:1786-1790.
Wollensak G, Spoerl E, Wilsch M, Seiler T. 2004(b) "Keratocyte apoptosis after corneal collagen cross-linking using riboflavin/UVA treatment", Cornea. 23:43-49.

## Claims

1. A chlorophyll or bacteriochlorophyll derivative or a pharmaceutical acceptable salt thereof for use in treating diseases, disorders and conditions associated with corneal or scleral anomaly selected from corneal thinning and scleral weakening by photodynamic therapy (PDT), wherein the chlorophyll or bacteriochlorophyll derivative has the formula II: 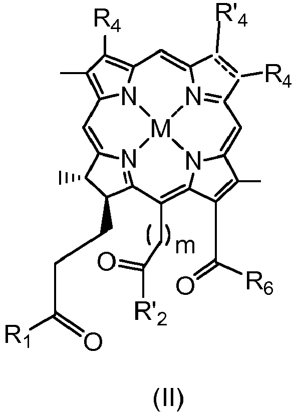 wherein
M represents 2H or an atom selected from the group consisting of Mg, Pd, Pt, Zn, In, Gd and Yb;
R₁, R'₂ and R₆ each independently is Y-R₈, -NR₉R'₉ or -N⁺R₉R'₉R"₉A⁻;
Y is O or S;
R₄ is -CH=CR₉R'₉, -CH=CR₉Hal, -CH=CH-CH₂-NR₉R'₉, -CH=CH-CH₂- N⁺R₉R'₉R"₉ A⁻, -CHO, -CH=NR₉, -CH=N⁺R₉R'₉A⁻ , -CH₂-OR₉, -CH₂-SR₉, -CH₂-Hal, -CH₂-R₉, -CH₂-NR₉R₉, -CH₂-N⁺R₉R'₉R"₉A⁻, -CH₂-CH₂R₉, -CH₂-CH₂Hal, -CH₂-CH₂OR₉, -CH₂-CH₂SR₉,-CH₂-CH₂-NR₉R'₉, -CH₂-CH₂-N⁺R₉R'₉R"₉A⁻, -COCH₃, C(CH₃)=CR₉R'₉, -C(CH₃)=CR₉Hal,-C(CH₃)=NR₉, -CH(CH₃)=N⁺R₉R'₉A⁻ , -CH(CH₃)-Hal, -CH(CH₃)-OR₉, -CH(CH₃)-SR₉,-CH(CH₃)-NR₉R'₉, -CH(CH₃)-N⁺R₉R'₉R"₉A⁻, or -C≡CR₉;
R'₄ is methyl or formyl;
Rs, R₉, R'₉ and R"₉ each independently is:
(a) H;
(b) C₁-C₂₅ hydrocarbyl;
(c) C₁-C₂₅ hydrocarbyl substituted by one or more functional groups selected from the group consisting of halogen, nitro, oxo, OR, SR, epoxy, epithio, -CONRR', -COR, COOR",-OSO₃R, -SO₃R", -SO₂R, -NHSO₂R, -SO₂NRR', -NRR', =N-OR, =N-NRR', -C(=NR)-NRR',-NR-NRR', -(R)N-C(=NR)-NRR', O←NR-, >C=NR, -(CH₂)ₙ-NR-COR', -(CH₂)ₙ-CO-NRR', -O-(CH₂)ₙ-OR, -O-(CH₂)ₙ-O-(CH₂)ₙ-R, -PRR', -OPO₃RR', -PO₂HR and -PO₃R"R", wherein n is an integer of 1 to 10 and R and R' each independently is H, hydrocarbyl or heterocyclyl, or R and R' together with the N atom to which they are attached form a 3-7 membered saturated ring optionally containing a further heteroatom selected from O, S and N, wherein the further N atom may be substituted, and R" is H, a cation, hydrocarbyl or heterocyclyl;
(d) C₁-C₂₅ hydrocarbyl substituted by one or more functional groups selected from the group consisting of positively charged groups, negatively charged groups, basic groups that are converted to positively charged groups under physiological conditions, and acidic groups that are converted to negatively charged groups under physiological conditions;
(e) C₁-C₂₅ hydrocarbyl containing one or more heteroatoms and/or one or more carbocyclic or heterocyclic moieties;
(f) C₁-C₂₅ hydrocarbyl containing one or more heteroatoms and/or one or more carbocyclic or heterocyclic moieties and substituted by one or more functional groups as defined in (c) and (d) above;
(g) C₁-C₂₅ hydrocarbyl substituted by a residue of an amino acid, a peptide, a protein, a monosaccharide, an oligosaccharide, or a polysaccharide; or
(h) a residue of an amino acid, a peptide, a protein, a monosaccharide, an oligosaccharide, or a polysaccharide;
Rs may further be H⁺ or a cation R⁺₁₀ when R₁, R'₂ and R₆ each independently is Y-R₈;
R⁺₁₀ is a metal cation, an ammonium group or an organic cation;
A is a physiologically acceptable anion;
m is 0 or 1; and
the dotted line at positions 7-8 represents an optional double bond.

2. The chlorophyll or bacteriochlorophyll derivative according to claim 1 for use according to claim 1, wherein the dotted line at positions 7-8 represents a double bond and the compound is a chlorophyll derivative of the formula II or the dotted line at positions 7-8 is absent and the compound is a bacteriochlorophyll derivative of the formula II .

3. The chlorophyll or bacteriochlorophyll derivative according to claim 2 for use according to claim 2, containing at least one group selected from:
(a) a negatively charged group selected from COO⁻, COS⁻, SO₃⁻, or PO₃²⁻;
(b) an acidic group that is converted to a negatively charged group at the physiological pH, selected from COOH, COSH, SO₃H, or PO₃H₂, or a salt thereof;
(c) a positively charged group, preferably an end group or a group located within an alkyl chain, selected from: (i) an onium group not containing a N atom selected from -O⁺(RR'), -S⁺(RR'), -Se⁺(RR'), -Te⁺(RR'), -P⁺(RR'R"), -As⁺(RR'R"), -Sb⁺(RR'R"), and -Bi⁺(RR'R"); (ii) a cation derived from a N-containing group selected from -N⁺(RR'R"), -(R)N-N⁺(RR'R"), O←N⁺(RR'R")-, >C=N⁺(RR'), -C(=NR)-N⁺RR'R" or -(R)N-C(=NR)-N⁺RR'R" group, preferably N^{I}(RR'R"); or (iii) a cation derived from a heteroaromatic compound containing one or more N atoms and optionally O or S atoms selected from pyrazolium, imidazolium, oxazolium, thiazolium, pyridinium, quinolinium, isoquinolinium, pyrimidinium, 1,2,4-triazinium, 1,3,5-triazinium or purinium; or
(d) a basic group that is converted to a positively charged group under physiological conditions, said basic group is an end group or a group located within an alkyl chain, selected from -NRR', -C(=NR)-NR'R", -NR-NR'R", -(R)N-C(=NR)-NR'R", O←NRR'-, or >C=NR, or the basic group is a N-containing heteroaromatic radical selected from pyrazolyl, imidazolyl, oxazolyl, thiazolyl, pyridyl, quinolinyl, isoquinolinyl, pyrimidyl, 1,2,4-triazinyl, 1,3,5-triazinyl or purinyl,
wherein R, R' and R" each independently is H, optionally substituted hydrocarbyl or heterocyclyl, or two of R, R' and R" together with the N atom to which they are attached form a 3-7 membered saturated ring, optionally containing one or more heteroatoms selected from O, S or N, and optionally further substituted at the additional N atom, said ring is selected from the group consisting of aziridine, pyrrolidine, piperidine, morpholine, thiomorpholine, azepine or piperazine optionally substituted at the additional N atom by C₁-C₆ alkyl optionally substituted by halo, hydroxyl or amino.

4. The chlorophyll or bacteriochlorophyll derivative according to claim 3 for use according to claim 3, wherein R₆ is -NR₉R'₉, wherein R₉ is H and R'₉ is a C₁-C₁₀ alkyl substituted by at least one group selected from: a positively charged group, a negatively charged group, an acidic group that is converted to a negatively charged group under physiological conditions, preferably SO₃H or an alkaline salt thereof, or a basic group that is converted to a positively charged group under physiological conditions, preferably -NRR' or -NH-(CH₂)₂₋₆-NRR', wherein each of R and R' independently is H, C₁-C₆ alkyl optionally substituted by NH₂, or R and R' together with the N atom form a 5-6 membered saturated ring, optionally containing an O or N atom and optionally further substituted at the additional N atom by -(CH₂)₂₋₆-NH₂.

5. The chlorophyll or bacteriochlorophyll derivative according to claim 4 for use according to claim 4, wherein the derivative is a bacteriochlorophyll of formula II, wherein:
M is 2H, Mg, Pd, or Zn, preferably 2H or Pd, more preferably Pd;
R₁ is selected from:
(i) -O⁻R₁₀⁺;
(ii) Y-R₈ wherein Y is O or S and Rs is the residue of an amino acid, a peptide or a protein;
(iii) -NH-CH₂-CH(OH)-CH₂OH;
(iv) - NH-(CH₂)ₙ-OH;
(v) - NH-CH(OH)-CH₃;
(vi) - NH-(CH₂)ₙ-NR-(CH₂)ₙ-OH;
(vii) glycosylamino; or
(viii) NHR'₉ which is as defined for R₆;
R'₂ is C₁-C₆ alkoxy selected from methoxy, ethoxy, propoxy or butoxy, more preferably methoxy;
R₄ is -C(=O)-CH₃, -CH=N-(CH₂)ₙ-SO₃⁻R₁₀⁺; -CH=N-(CH₂)ₙ-COO⁻R₁₀⁺; -CH=N-(CH₂)ₙ-PO₃²⁻(R₁₀⁺)₂; -CH₂-NH-(CH₂)ₙ-SO₃⁻R₁₀⁺; -NH-(CH₂)ₙ-COO⁻R₁₀⁺; -NH-(CH₂)ₙ-PO₃²⁻(R₁₀⁺)₂; or-C(CH₃)=NR₉, preferably C(CH₃)=N-(CH₂)ₙ-NH₂, or -C(CH₃)=N-(CH₂)ₙ-N(R)₃⁺A⁻;
R₆ is selected from (i) NHR'₉, selected from:
(a) -NH-(CH₂)ₙ-SO₃⁻R₁₀⁺, preferably -NH-(CH₂)₂-SO₃R₁₀⁺ or -NH-(CH₂)₃-SO₃R₁₀⁺;
(b) -NH-(CH₂)ₙ-COO⁻R₁₀⁺;
(c) -NH-(CH₂)ₙ-PO₃²⁻(R₁₀⁺)₂;
(d)-NH-(CH₂)ₙ-B:
(e) 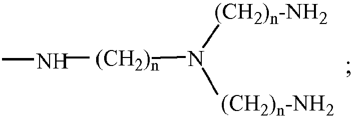
(f) 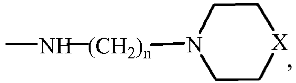 preferably -NH-(CH₂)₂-1-morpholino or -NH-(CH₂)₃-piperazino;
(g) 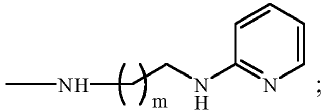
(h) -NH-(CH₂)ₙ-N(R")-(CH₂)ₙ-NRR', preferably -NH-(CH₂)₃-NH-(CH₂)₃-NH₂;
(j) -NH-(CH₂)ₙ-NRR', preferably -NH-CH₂-CH₂-NRR';
R₁₀⁺ is H⁺, or a monovalent cation selected from K⁺, Na⁺, Li⁺, or NH₄⁺, more preferably K⁺;
X is O, S or NH;
B is a positively charged group selected from ammonium -N⁺RR'R", preferably - N(CH₃)₃⁺A⁻, guanidinium, sulfonium, phosphonium, arsonium; or B is a basic group that is converted to a positively charged group under physiological conditions, selected from amino-NRR', preferably -NH₂, guanidino, phosphino, or arsino;
m is 1, n is an integer from 1 to 10, preferably 2 or 3; and
A⁻ is a physiologically acceptable anion;
wherein R, R' and R" each independently is H or C₁-C₆ alkyl.

6. The chlorophyll or bacteriochlorophyll derivative according to claim 5 for use according to claim 5, wherein the derivative is a bacteriochlorophyll of formula II selected from the pharmaceutically acceptable salts with a monovalent or divalent alkaline or alkaline earth metal cation, or with NH₄⁺, of the following compounds:
Palladium 3¹-oxo-15-methoxycarbonylmethyl-rhodobacteriochlorin 13¹-(2-sulfoethyl) amide;
Palladium 3¹-oxo-15-methoxycarbonylmethyl-rhodobacteriochlorin 13¹-(-sulfopropyl)amide;
Palladium 3¹-oxo-15-methoxycarbonylmethyl-rhodobacteriochlorin 13¹,17³-di-(2-sulfoethyl)amide;
Palladium 3¹-oxo-15-methoxycarbonylmethyl-rhodobacteriochlorin 13¹,17³-di-(3-sulfopropyl)amide;
3¹-oxo-15-methoxycarbonylmethyl-rhodobacteriochlorin 13¹-(2-sulfoethyl)amide;
3¹-oxo-15-methoxycarbonylmethyl-rhodobacteriochlorin 13¹-(3-sulfopropyl)amide;
3¹-oxo-15-methoxycarbonylmethyl-rhodobacteriochlorin 13¹,17³-di-(2-sulfoethyl) amide; and
3¹-oxo-15-methoxycarbonylmethyl-rhodobacteriochlorin 13¹,17³-di-(3-sulfopropyl)amide.

7. The chlorophyll or bacteriochlorophyll derivative according to claim 6 for use according to claim 6, selected from the salts consisting of:
Palladium 3¹-oxo-15-methoxycarbonylmethyl-rhodobacteriochlorin 13¹-(2-sulfoethyl) amide dipotassium salt (herein WST11);
Palladium 3¹-oxo-15-methoxycarbonylmethyl-rhodobacteriochlorin 13¹-(3-sulfopropyl)amide dipotassium salt (herein compound 1);
Palladium 3¹-oxo-15-methoxycarbonylmethyl-rhodobacteriochlorin 13¹,17³-di-(2-sulfoethyl)amide dipotassium salt (herein compound 2); and
Palladium 3¹-oxo-15-methoxycarbonylmethyl-rhodobacteriochlorin 13¹,17³-di-(3-sulfopropyl)amide dipotassium salt (herein compound 3).
3¹-oxo-15-methoxycarbonylmethyl-rhodobacteriochlorin 13¹-(2-sulfoethyl)amide dipotassium salt;
3¹-oxo-15-methoxycarbonylmethyl-rhodobacteriochlorin 13¹-(3-sulfopropyl)amide dipotassium salt;
3¹-oxo-15-methoxycarbonylmethyl-rhodobacteriochlorin 13¹,17³-di-(2-sulfoethyl)amide dipotassium salt; and
3¹-oxo-15-methoxycarbonylmethyl-rhodobacteriochlorin 13¹,17³-di-(3-sulfopropyl)amide dipotassium salt.

8. The chlorophyll or bacteriochlorophyll derivative according to claim 7 for use according to claim 7, wherein said bacteriochlorophyll derivative is Palladium 3¹-oxo-15-methoxycarbonylmethyl-rhodobacteriochlorin 13¹-(2-sulfoethyl)amide dipotassium salt (herein WST11).

9. A pharmaceutical composition comprising a pharmaceutically acceptable carrier and a chlorophyll or bacteriochlorophyll derivative according to any one of claims 1 to 8 for use in treating diseases, disorders and conditions associated with corneal or scleral anomaly selected from corneal thinning and scleral weakening by photodynamic therapy (PDT).

10. The pharmaceutical composition according to claim 9 for use according to claim 9, further comprising a viscous agent for enhancing the viscosity of the formulation and restricting penetration of the chlorophyll or bacteriochlorophyll derivative into the cornea.

11. The pharmaceutical composition according to claim 10 for use according to claim 10, wherein said viscous agent is a polymer, preferably a biopolymer selected from dextran, scleroglucan and derivatives thereof, Gellan gum, Guar gum or methylcellulose.

12. The pharmaceutical composition according to claim 11 for use according to claim 11, further comprising a transepithelial permeability enhancer for enhancing the penetration of the chlorophyll or bacteriochlorophyll derivative through the epithelium.

13. The pharmaceutical composition according to any one of claims 9 to 12 for use according to any one of claims 9 to 12, for use in topical application.

14. The pharmaceutical composition according to any one of claims 9 to 13 for use according to any one of claims 9 to 13, for use in the photodynamic therapy before local irradiation of the eye with light at a red or near infrared (NIR) wavelength.

15. The chlorophyll or bacteriochlorophyll derivative according to any one of claims 1 to 8 for use according to any one of claims 1 to 8 or the pharmaceutical composition according to any one of claims 9 to 14 for use according to any one of claims 9 to 14, wherein the diseases, disorders and conditions associated with corneal thinning and scleral weakening are selected from keratoconus, post-laser-assisted in situ keratomileusis (LASIK) ectasia, post-photorefractive keratectomy (PRK) ectasia, post-infection ectasia, peripheral ectasia, rheumatoid condition of the cornea, degenerative myopia, regular myopia, scleral staphyloma, ocular hypertension glaucoma, low tension glaucoma and combinations thereof.

16. The chlorophyll or bacteriochlorophyll derivative according to claim 8 for use according to claim 8 or the pharmaceutical composition according to any one of claims 9 to 15 for use according to any one of claims 9 to 15, wherein the disease, disorder and condition associated with corneal thinning is keratoconus.

## Patentansprüche

1. Chlorophyll- oder Bakteriochlorophyll-Derivat oder ein pharmazeutisch akzeptables Salz davon zur Verwendung bei der Behandlung von Krankheiten, Störungen und Leiden, die mit Hornhaut- oder Skleraanomalie verbunden sind, ausgewählt aus Hornhautausdünnung und Skleraschwächung, durch photodynamische Therapie (PDT), wobei das Chlorophyll- oder Bakteriochlorophyll-Derivat die Formel II aufweist: 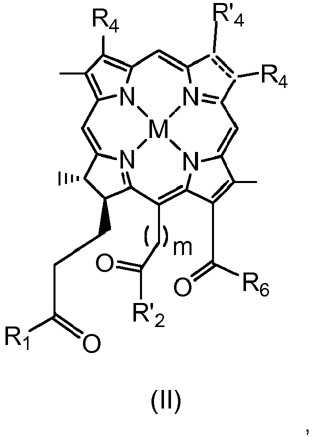 wobei
M 2H oder ein Atom, ausgewählt aus der Gruppe, bestehend aus Mg, Pd, Pt, Zn, In, Gd und Yb, darstellt;
R₁, R'₂ und R₆ jeweils unabhängig Y-R₈, -NR₉R'₉ oder -N⁺R₉R'₉R"₉A⁻ sind;
Y O oder S ist;
R₄ -CH=CR₉R'₉, -CH=CR₉Hal, -CH=CH-CH₂-NR₉R'₉, -CH=CH-CH₂-N⁺R₉R'₉R"₉A⁻, -CHO, -CH=NR₉, -CH=N⁺R₉R'₉A⁻, -CH₂-OR₉, -CH₂-SR₉, -CH₂-Hal, -CH₂-R₉, -CH₂-NR₉R'₉, -CH₂-N⁺R₉R'₉R"₉A⁻, -CH₂-CH₂R₉, -CH₂-CH₂Hal, -CH₂-CH₂OR₉, -CH₂-CH₂SR₉, -CH₂-CH₂-NR₉R'₉, -CH₂-CH₂-N⁺R₉R'₉R"₉A⁻, -COCH₃, C(CH₃)=CR₉R'₉, -C(CH₃)=CR₉Hal, -C(CH₃)=NR₉, -CH(CH₃)=N⁺R₉R'₉A⁻, -CH(CH₃)-Hal, -CH(CH₃)-OR₉, -CH(CH₃)-SR₉, -CH(CH₃)-NR₉R'₉, -CH(CH₃)-N⁺R₉R'₉R"₉A⁻ oder -C=CR₉ ist;
R'₄ Methyl oder Formyl ist;
R₈, R₉, R'₉ und R"₉ unabhängig voneinander:
(a) H;
(b) C₁-C₂₅-Hydrocarbyl;
(c) C₁-C₂₅-Hydrocarbyl, substituiert mit einer oder mehreren funktionellen Gruppen, ausgewählt aus der Gruppe, bestehend aus Halogen, Nitro, Oxo, OR, SR, Epoxy, Epithio, -CONRR', -COR, COOR", -OSO₃R, -SO₃R", -SO₂R, -NHSO₂R, -SO₂NRR', -NRR', =N-OR, =N-NRR', -C(=NR)-NRR', -NR-NRR', -(R)ₙ-C(=NR)-NRR', O←NR-, >C=NR, -(CH₂)ₙ-NR-COR', -(CH₂)ₙ-CO-NRR', -O-(CH₂)ₙ-OR, -O-(CH₂)ₙ-O-(CH₂)ₙ-R, -PRR', -OPO₃RR', -PO₂HR und -PO₃R"R", wobei n eine ganze Zahl von 1 bis 10 ist und R und R' jeweils unabhängig voneinander H, Hydrocarbyl oder Heterocyclyl sind oder R und R' zusammen mit dem N-Atom, an das sie gebunden sind, einen 3-7-gliedrigen, gesättigten Ring bilden, der gegebenenfalls ein weiteres Heteroatom, ausgewählt aus O, S und N, enthält, wobei das weitere N-Atom substituiert sein kann, und R" H, ein Kation, Hydrocarbyl oder Heterocyclyl ist;
(d) C₁-C₂₅-Hydrocarbyl, substituiert mit einer oder mehreren funktionellen Gruppen, ausgewählt aus der Gruppe, bestehend aus positiv geladenen Gruppen, negativ geladenen Gruppen, basischen Gruppen, die unter physiologischen Bedingungen in positiv geladene Gruppen umgewandelt werden, und sauren Gruppen, die unter physiologischen Bedingungen in negativ geladene Gruppen umgewandelt werden;
(e) C₁-C₂₅-Hydrocarbyl, enthaltend ein oder mehrere Heteroatome und/oder eine oder mehrere carbocyclische oder heterocyclische Einheiten;
(f) C₁-C₂₅-Hydrocarbyl, enthaltend ein oder mehrere Heteroatome und/oder eine oder mehrere carbocyclische oder heterocyclische Einheiten und substituiert mit einer oder mehreren funktionellen Gruppen, wie oben in (c) und (d) definiert;
(g) C₁-C₂₅-Hydrocarbyl, substituiert mit einem Rest einer Aminosäure, eines Peptids, eines Proteins, eines Monosaccharids, eines Oligosaccharids oder eines Polysaccharids; oder
(h) ein Rest einer Aminosäure, eines Peptids, eines Proteins, eines Monosaccharids, eines Oligosaccharids oder eines Polysaccharids
sind;
R₈ ferner H⁺ oder ein Kation R⁺₁₀ sein kann, wenn R₁, R'₂ und R₆ jeweils unabhängig voneinander Y-R₈ sind;
R⁺₁₀ ein Metallkation, eine Ammoniumgruppe oder ein organisches Kation ist;
A⁻ ein physiologisch akzeptables Anion ist;
m 0 oder 1 ist und
die punktierte Linie an den Positionen 7-8 eine optionale Doppelbindung darstellt.

2. Chlorophyll- oder Bakteriochlorophyll-Derivat nach Anspruch 1 zur Verwendung nach Anspruch 1, wobei die punktierte Linie an den Positionen 7-8 eine Doppelbindung darstellt und die Verbindung ein Chlorophyll-Derivat der Formel II ist oder
die punktierte Linie an den Positionen 7-8 abwesend ist und die Verbindung ein Bakteriochlorophyll-Derivat der Formel II ist.

3. Chlorophyll- oder Bakteriochlorophyll-Derivat nach Anspruch 2 zur Verwendung nach Anspruch 2, enthaltend mindestens eine Gruppe, ausgewählt aus:
(a) einer negativ geladenen Gruppe, ausgewählt aus COO⁻, COS⁻, SO₃⁻ oder PO₃²⁻;
(b) einer sauren Gruppe, die bei dem physiologischen pH in eine negativ geladene Gruppe umgewandelt wird, ausgewählt aus COOH, COSH, SO₃H oder PO₃H₂, oder ein Salz davon;
(c) einer positiv geladenen Gruppe, vorzugsweise einer Endgruppe oder einer Gruppe, die sich innerhalb einer Alkylkette befindet, ausgewählt aus: (i) einer Oniumgruppe, die kein N-Atom enthält, ausgewählt aus -O⁺(RR'), -S⁺(RR'), -Se⁺(RR'), -Te⁺(RR'),
-P⁺(RR'R"), -As⁺(RR'R"), -Sb⁺(RR'R") und -Bi+(RR'R"); (ii) einem Kation, abgeleitet von einer N-enthaltenden Gruppe, ausgewählt aus einer -N⁺(RR'R")-, -(R)N-N⁺(RR'R")-, O←N⁺(RR'R")-, >C=N⁺(RR')-, -C(=NR)-N⁺RR'R"- oder -(R)N-C(=NR)-N⁺RR'R"-Gruppe, vorzugsweise N⁺(RR'R"); oder (iii) einem Kation, abgeleitet von einer heteroaromatischen Verbindung, die ein oder mehrere N-Atome und gegebenenfalls O- oder S-Atome enthält, ausgewählt aus Pyrazolium, Imidazolium, Oxazolium, Thiazolium, Pyridinium, Chinolinium, Isochinolinium, Pyrimidinium, 1,2,4-Triazinium, 1,3,5-Triazinium oder Purinium; oder
(d) einer basischen Gruppe, die unter physiologischen Bedingungen in eine positiv geladene Gruppe umgewandelt wird, wobei die basische Gruppe eine Endgruppe oder eine Gruppe ist, die sich innerhalb einer Alkylkette befindet, ausgewählt aus -NRR', -C(=NR)-NR'R", -NR-NR'R", -(R)N-C(=NR)-NR'R", O←NRR' oder >C=NR, oder die basische Gruppe ein N-enthaltender heteroaromatischer Rest ist, ausgewählt aus Pyrazolyl, Imidazolyl, Oxazolyl, Thiazolyl, Pyridyl, Chinolinyl, Isochinolinyl, Pyrimidyl, 1,2,4-Triazinyl, 1,3,5-Triazinyl oder Purinyl,
wobei R, R' und R" unabhängig voneinander H, gegebenenfalls substituiertes Hydrocarbyl oder Heterocyclyl sind, oder zwei von R, R' und R" zusammen mit dem N-Atom, an das sie gebunden sind, einen 3-7-gliedrigen gesättigten Ring bilden, der gegebenenfalls ein oder mehrere Heteroatome enthält, ausgewählt aus O, S oder N, und gegebenenfalls ferner an dem zusätzlichen N-Atom substituiert ist, wobei der Ring ausgewählt ist aus der Gruppe, bestehend aus Aziridin, Pyrrolidin, Piperidin, Morpholin, Thiomorpholin, Azepin oder Piperazin, das gegebenenfalls an dem zusätzlichen N-Atom mit C₁-C₆-Alkyl substituiert ist, das gegebenenfalls mit Halogen, Hydroxyl oder Amino substituiert ist.

4. Chlorophyll- oder Bakteriochlorophyll-Derivat nach Anspruch 3 zur Verwendung nach Anspruch 3, wobei R₆ -NRgR'g ist, wobei Rg H ist und R'₉ ein C₁-C₁₀-Alkyl ist, substituiert mit mindestens einer Gruppe, ausgewählt aus: einer positiv geladenen Gruppe, einer negativ geladenen Gruppe, einer sauren Gruppe, die unter physiologischen Bedingungen in eine negativ geladene Gruppe umgewandelt wird, vorzugsweise SO₃H oder ein alkalisches Salz davon, oder einer basischen Gruppe, die unter physiologischen Bedingungen in eine positiv geladene Gruppe umgewandelt wird, vorzugsweise -NRR' oder -NH-(CH₂)₂₋₆-NRR', wobei jeder von R und R' unabhängig voneinander H, C₁-C₆-Alkyl, gegebenenfalls substituiert mit NH₂, ist, oder R und R' zusammen mit dem N-Atom einen 5-6-gliedrigen gesättigten Ring bilden, der gegebenenfalls ein O- oder N-Atom enthält und gegebenenfalls ferner an dem zusätzlichen N-Atom mit -(CH₂)₂₋₆-NH₂ substituiert ist.

5. Chlorophyll- oder Bakteriochlorophyll-Derivat nach Anspruch 4 zur Verwendung nach Anspruch 4, wobei das Derivat ein Bakteriochlorophyll der Formel II ist, wobei:
M 2H, Mg, Pd oder Zn, vorzugsweise 2H oder Pd, stärker bevorzugt Pd ist;
R₁ ausgewählt ist aus:
(i) -O-R₁₀⁺;
(ii) Y-R₈, wobei Y O oder S ist und R₈ der Rest einer Aminosäure, eines Peptids oder eines Proteins ist;
(iii) -NH-CH₂-CH(OH)-CH₂OH;
(iv) -NH-(CH₂)ₙ-OH;
(v) -NH-CH(OH)-CH₃;
(vi) NH-(CH₂)ₙ-NR-(CH₂)ₙ-OH;
(vii) Glycosylamino oder
(viii) NHR'₉, das wie für R₆ definiert ist;
R'₂ C₁-C₆-Alkoxy ist, ausgewählt aus Methoxy, Ethoxy, Propoxy oder Butoxy, stärker bevorzugt Methoxy;
R₄ -C(=O)-CH₃, -CH=N-(CH₂)ₙ-SO₃⁻R₁₀⁺; -CH=N-(CH₂)ₙ-COO⁻R₁₀⁺; -CH=N-(CH₂)ₙ-PO₃²-(R₁₀⁺)₂; -CH₂-NH-(CH₂)ₙ-SO₃⁻R₁₀⁺; -NH-(CH₂)ₙ-COO⁻R₁₀⁺; -NH-(CH₂)ₙ-PO₃²⁻ (R₁₀⁺)₂ oder -C(CH₃)=NR₉, vorzugsweise C(CH₃)=N-(CH₂)ₙ-NH₂, oder -C(CH₃)=N-(CH₂)ₙ-N(R)₃⁺A⁻ ist;
R₆ ausgewählt ist aus (i) NHR'₉, ausgewählt aus:
(a) -NH-(CH₂)ₙ-SO₃⁻R₁₀⁺, vorzugsweise -NH-(CH₂)₂-SO₃R₁₀⁺ oder -NH-(CH₂)₃-SO₃R₁₀+,
(b) -NH-(CH₂)ₙ-COO⁻R₁₀⁺
(c) -NH-(CH₂)ₙ-PO₃²⁻(R₁₀⁺)₂;
(d) -NH-(CH₂)ₙ⁻B:
(e) 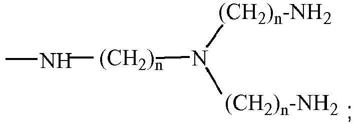
(f) 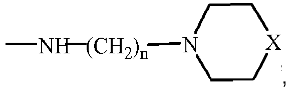 vorzugsweise -NH-(CH₂)₂-1-Morpholino oder -NH-(CH₂)₃-Piperazino;
(g) 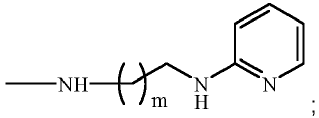
(h) -NH-(CH₂)ₙ-N(R")-(CH₂)ₙ-NRR', vorzugsweise -NH-(CH₂)₃-NH-(CH₂)₃-NH₂;
(j) -NH-(CH₂)ₙ-NRR', vorzugsweise -NH-CH₂-CH₂-NRR';
R₁₀⁺ H⁺ oder ein einwertiges Kation, ausgewählt aus K⁺, Na⁺, Li⁺ oder NH₄⁺, stärker bevorzugt K⁺ ist;
X O, S oder NH ist;
B eine positiv geladene Gruppe ist, ausgewählt aus Ammonium-N⁺RR'R", vorzugsweise -N(CH₃)₃⁺A⁻, Guanidinium, Sulfonium, Phosphonium, Arsonium; oder B eine basische Gruppe ist, die unter physiologischen Bedingungen in eine positiv geladene Gruppe umgewandelt wird, ausgewählt aus Amino-NRR', vorzugsweise -NH₂, Guanidino, Phosphino oder Arsino;
m 1 ist,
n eine ganze Zahl von 1 bis 10, vorzugsweise 2 oder 3 ist und
A⁻ ein physiologisch akzeptables Anion ist;
wobei R, R' und R" jeweils unabhängig voneinander H oder C₁-C₆-Alkyl sind.

6. Chlorophyll- oder Bakteriochlorophyll-Derivat nach Anspruch 5 zur Verwendung nach Anspruch 5, wobei das Derivat ein Bakteriochlorophyll der Formel II ist, ausgewählt aus den pharmazeutisch akzeptablen Salzen mit einem einwertigen oder zweiwertigen Alkali- oder Erdalkalimetallkation oder mit NH₄⁺ der folgenden Verbindungen:
Palladium-3¹-oxo-15-methoxycarbonylmethyl-rhodobacteriochlorin-13¹-(2-sulfoethyl)amid;
Palladium-3¹-oxo-15-methoxycarbonylmethyl-rhodobacteriochlorin-13¹-(-sulfopropyl)amid;
Palladium-3¹-oxo-15-methoxycarbonylmethyl-rhodobacteriochlorin-13¹,17³-di-(2-sulfoethyl)amid;
Palladium-3¹-oxo-15-methoxycarbonylmethyl-rhodobacteriochlorin-13¹,17³-di-(3-sulfopropyl)amid;
3¹-Oxo-15-methoxycarbonylmethyl-rhodobacteriochlorin-13¹-(2-sulfoethyl)amid;
3¹-Oxo-15-methoxycarbonylmethyl-rhodobacteriochlorin-13¹-(3-sulfopropyl)amid;
3¹-Oxo-15-methoxycarbonylmethyl-rhodobacteriochlorin-13¹,17³-di-(2-sulfoethyl)amid und
3¹-Oxo-15-methoxycarbonylmethyl-rhodobacteriochlorin-13¹,17³-di-(3-sulfopropyl)amid.

7. Chlorophyll- oder Bakteriochlorophyll-Derivat nach Anspruch 6 zur Verwendung nach Anspruch 6, ausgewählt aus den Salzen, bestehend aus:
Palladium-3¹-oxo-15-methoxycarbonylmethyl-rhodobacteriochlorin-13¹-(2-sulfoethyl)amid-Dikaliumsalz (hierin WST11);
Palladium-3¹-oxo-15-methoxycarbonylmethyl-rhodobacteriochlorin-13¹-(3-sulfopropyl)amid-Dikaliumsalz (hierin Verbindung 1);
Palladium-3¹-oxo-15-methoxycarbonylmethyl-rhodobacteriochlorin-13¹,17³-di-(2-sulfoethyl)amid-Dikaliumsalz (hierin Verbindung 2) und
Palladium-3¹-oxo-15-methoxycarbonylmethyl-rhodobacteriochlorin-13¹,17³-di-(3-sulfopropyl)amid-Dikaliumsalz (hierin Verbindung 3);
3¹-Oxo-15-methoxycarbonylmethyl-rhodobacteriochlorin-13¹-(2-sulfoethyl)-amid-Dikaliumsalz;
3¹-Oxo-15-methoxycarbonylmethyl-rhodobacteriochlorin-13¹-(3-sulfopropyl)-amid-Dikaliumsalz;
3¹-Oxo-15-methoxycarbonylmethyl-rhodobacteriochlorin-13¹,17³-di-(2-sulfoethyl)-amid-Dikaliumsalz und
3¹-Oxo-15-methoxycarbonylmethyl-rhodobacteriochlorin-13¹,17³-di-(3-sulfopropyl)amid-Dikaliumsalz.

8. Chlorophyll- oder Bakteriochlorophyll-Derivat nach Anspruch 7 zur Verwendung nach Anspruch 7, wobei das Bakteriochlorophyll-Derivat Palladium-3¹-oxo-15-methoxycarbonylmethyl-rhodobacteriochlorin-13¹-(2-sulfoethyl)amid-Dikaliumsalz (hierin WST11) ist.

9. Pharmazeutische Zusammensetzung, umfassend einen pharmazeutisch akzeptablen Träger und ein Chlorophyll- oder Bakteriochlorophyll-Derivat nach einem der Ansprüche 1 bis 8 zur Verwendung bei der Behandlung von Krankheiten, Störungen und Leiden, die mit Hornhaut- oder Skleraanomalie verbunden sind, ausgewählt aus Hornhautausdünnung und Skleraschwächung, durch photodynamische Therapie (PDT).

10. Pharmazeutische Zusammensetzung nach Anspruch 9 zur Verwendung nach Anspruch 9, ferner umfassend ein viskoses Mittel zur Erhöhung der Viskosität der Formulierung und Einschränkung des Eindringens des Chlorophyll- oder Bakteriochlorophyll-Derivats in die Hornhaut.

11. Pharmazeutische Zusammensetzung nach Anspruch 10 zur Verwendung nach Anspruch 10, wobei das viskose Mittel ein Polymer, vorzugsweise ein Biopolymer, ausgewählt aus Dextran, Scleroglucan und Derivaten davon, Gellangummi, Guargummi oder Methylcellulose ist.

12. Pharmazeutische Zusammensetzung nach Anspruch 11 zur Verwendung nach Anspruch 11, ferner umfassend einen Verstärker der transepithelialen Permeabilität zum Verstärken des Eindringens des Chlorophyll- oder Bakteriochlorophyll-Derivats durch das Epithel.

13. Pharmazeutische Zusammensetzung nach einem der Ansprüche 9 bis 12 zur Verwendung nach einem der Ansprüche 9 bis 12 zur Verwendung bei topischer Anwendung.

14. Pharmazeutische Zusammensetzung nach einem der Ansprüche 9 bis 13 zur Verwendung nach einem der Ansprüche 9 bis 13 zur Verwendung bei der photodynamischen Therapie vor einer lokalen Bestrahlung des Auges mit Licht bei einer Wellenlänge im Rot- oder nahen Infrarotbereich (NIR).

15. Chlorophyll- oder Bakteriochlorophyll-Derivat nach einem der Ansprüche 1 bis 8 zur Verwendung nach einem der Ansprüche 1 bis 8 oder pharmazeutische Zusammensetzung nach einem der Ansprüche 9 bis 14 zur Verwendung nach einem der Ansprüche 9 bis 14, wobei die Krankheiten, Störungen und Leiden, die mit Hornhautausdünnung und Skleraschwächung verbunden sind, aus Keratokonus, Ektasie nach laserassistierter In-situ-Keratomileusis (LASIK), Ektasie nach photorefraktiver Keratektomie (PRK), Ektasie nach einer Infektion, peripherer Ektasie, rheumaähnlichem Leiden der Hornhaut, degenerativer Myopie, regulärer Myopie, Sklerastaphylom, Augenhypertensionglaukom, Niedrigdruckglaukom und Kombinationen davon ausgewählt sind.

16. Chlorophyll- oder Bakteriochlorophyll-Derivat nach Anspruch 8 zur Verwendung nach Anspruch 8 oder pharmazeutische Zusammensetzung nach einem der Ansprüche 9 bis 15 zur Verwendung nach einem der Ansprüche 9 bis 15, wobei die Krankheit, die Störung und das Leiden, die mit Hornhautausdünnung verbunden sind, Keratokonus sind.

## Revendications

1. Dérivé de chlorophylle ou de bactériochlorophylle ou un sel pharmaceutiquement acceptable de celui-ci, destiné à être utilisé dans le traitement de maladies, de troubles et d'états associés à une anomalie cornéenne ou sclérale, choisis parmi l'amincissement de la cornée et l'affaiblissement de la sclère par thérapie photodynamique (PDT), dans lequel le dérivé de chlorophylle ou de bactériochlorophylle a la formule II : 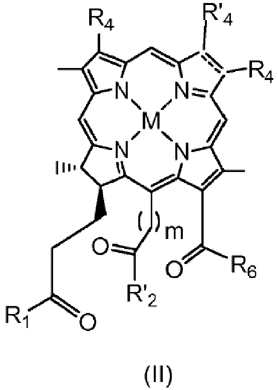 dans lequel
M représente 2H ou un atome choisi dans le groupe constitué de Mg, Pd, Pt, Zn, In, Gd et Yb ;
R₁, R'₂ et R₆ représentent chacun indépendamment Y-R₈, -NR₉R'₉ ou -N⁺R₉R'₉R"₉A⁻ ;
Y représente O ou S ;
R₄ représente -CH=CR₉R'₉, -CH=CR₉Hal, -CH=CH-CH₂-NR₉R'₉, -CH=CH-CH₂- N⁺R₉R'₉R"₉A⁻, -CHO, -CH=NR₉, -CH=N⁺R₉R'₉A⁻, -CH₂-OR₉, -CH₂-SR₉, -CH₂-Hal, -CH₂-R₉, -CH₂-NR₉R'₉, -CH₂-N⁺R₉R'₉R"₉A⁻, -CH₂-CH₂R₉, -CH₂-CH₂Hal, -CH₂-CH₂OR₉, -CH₂-CH₂SR₉, -CH₂-CH₂-NR₉R'₉, -CH₂-CH₂-N⁺R₉R'₉R"₉A⁻, -COCH₃, C(CH₃)=CR₉R'₉, -C(CH₃)=CR₉Hal, -C(CH₃)=NR₉, -CH(CH₃)=N⁺R₉R'₉A⁻, -CH(CH₃)-Hal, -CH(CH₃)-OR₉, -CH(CH₃)-SR₉, -CH(CH₃)-NR₉R'₉, -CH(CH₃)-N⁺R₉R'₉R"₉A⁻, ou -C≡CR₉ ;
R'₄ représente un méthyle ou formyle ;
R₈, R₉, R'₉ et R"₉ représentent chacun indépendamment :
(a) H;
(b) un hydrocarbyle en C₁-C₂₅ ;
(c) un hydrocarbyle en C₁-C₂₅ substitué par un ou plusieurs groupes fonctionnels choisis dans le groupe constitué de halogène, nitro, oxo, OR, SR, époxy, épithio, -CONRR', -COR, COOR", -OSO₃R, -SO₃R", -SO₂R, -NHSO₂R, -SO₂NRR', -NRR', =N-OR, =N-NRR', - C(=NR)-NRR', -NR-NRR', -(R)N-C(=NR)-NRR', O←NR-, >C=NR, -(CH₂)ₙ-NR-COR', -(CH₂)ₙ-CONRR', -O-(CH₂)ₙ-OR, -O-(CH₂)ₙ-O-(CH₂)ₙ-R, -PRR', - OPO₃RR', -PO₂HR et -PO₃R"R", où n est un nombre entier de 1 à 10 et R et R' représentent chacun indépendamment H, un hydrocarbyle ou un hétérocyclyle, ou R et R' forment avec l'atome de N auquel ils sont attachés un cycle saturé à 3-7 chaînons contenant éventuellement un autre hétéroatome choisi parmi O, S et N, où l'autre atome de N peut être substitué, et R" représente H, un cation, un hydrocarbyle ou un hétérocyclyle ;
(d) un hydrocarbyle en C₁-C₂₅ substitué par un ou plusieurs groupes fonctionnels choisis dans le groupe constitué de groupes chargés positivement, groupes chargés négativement, groupes basiques qui sont convertis en groupes chargés positivement dans des conditions physiologiques, et groupes acides qui sont convertis en groupes chargés négativement dans des conditions physiologiques ;
(e) hydrocarbyle en C₁-C₂₅ contenant un ou plusieurs hétéroatomes et/ou une ou plusieurs fractions carbocycliques ou hétérocycliques ;
(f) hydrocarbyle en C₁-C₂₅ contenant un ou plusieurs hétéroatomes et/ou une ou plusieurs fractions carbocycliques ou hétérocycliques et substitué par un ou plusieurs groupes fonctionnels tels que définis aux points (c) et (d) ci-dessus ;
(g) hydrocarbyle en C₁-C₂₅ substitué par un résidu d'un acide aminé, un peptide, une protéine, un monosaccharide, un oligosaccharide ou un polysaccharide ; ou
(h) un résidu d'un acide aminé, un peptide, une protéine, un monosaccharide, un oligosaccharide ou un polysaccharide ;
R₈ peut en outre représenter H⁺ ou un cation R⁺₁₀ lorsque R₁, R'₂ et R₆ représentent chacun indépendamment Y-R₈ ;
R⁺₁₀ représente un cation métallique, un groupe ammonium ou un cation organique ;
A⁻ représente un anion physiologiquement acceptable ;
m est égal à 0 ou 1 ; et
la ligne pointillée aux positions 7-8 représente une double liaison éventuelle.

2. Dérivé de chlorophylle ou de bactériochlorophylle selon la revendication 1 destiné à être utilisé selon la revendication 1, dans lequel la ligne pointillée aux positions 7-8 représente une double liaison et le composé est un dérivé de chlorophylle de formule Il ou
la ligne pointillée aux positions 7-8 est absente et le composé est un dérivé de bactériochlorophylle de formule II.

3. Dérivé de chlorophylle ou de bactériochlorophylle selon la revendication 2 destiné à être utilisé selon la revendication 2, contenant au moins un groupe choisi parmi :
(a) un groupe chargé négativement choisi parmi COO⁻, COS⁻, SO₃⁻, ou PO₃²⁻ ;
(b) un groupe acide qui est converti en un groupe chargé négativement au pH physiologique, choisi parmi COOH, COSH, SO₃H ou PO₃H₂, ou un sel de celui-ci ;
(c) un groupe chargé positivement, de préférence un groupe terminal ou un groupe situé à l'intérieur d'une chaîne alkyle, choisi parmi : (i) un groupe onium ne contenant pas d'atome de N tel que - O⁺(RR'), -S⁺(RR'), -Se⁺(RR'), -Te⁺(RR'), -P⁺(RR'R"), -As⁺(RR'R"), -Sb⁺(RR'R"), et -Bi⁺(RR'R") ; (ii) un cation dérivé d'un groupe contenant N choisi parmi les groupes -N⁺(RR'R"), -(R)N-N⁺(RR'R"), O←N⁺(RR'R")-, >C=N⁺(RR'), -C(=NR)-N⁺RR'R" ou -(R)N-C(=NR)-N⁺RR'R", de préférence N⁺(RR'R") ; ou (iii) un cation dérivé d'un composé hétéroaromatique contenant un ou plusieurs atomes de N et éventuellement des atomes de O ou de S choisis parmi pyrazolium, imidazolium, oxazolium, thiazolium, pyridinium, quinolinium, isoquinolinium, pyrimidinium, 1,2,4-triazinium, 1,3,5-triazinium ou purinium ; ou
(d) un groupe de base qui est converti en un groupe chargé positivement dans des conditions physiologiques, ledit groupe de base est un groupe terminal ou un groupe situé dans une chaîne alkyle, choisi parmi -NRR', -C(=NR)-NR'R", -NR-NR'R", -(R)N-C(=NR)-NR'R", ←O-NRR'-, ou >C=NR, ou le groupe de base est un radical hétéroaromatique contenant N, choisi parmi les radicaux pyrazolyle, imidazolyle, oxazolyle, thiazolyle, pyridyle, quinolinyle, isoquinolinyle, pyrimidyle, 1,2,4-triazinyle, 1,3,5-triazinyle ou purinyle,
dans lequel R, R' et R" représentent chacun indépendamment H, un hydrocarbyle ou hétérocyclyle éventuellement substitué, ou bien deux des R, R' et R" forment, conjointement avec l'atome de N auquel ils sont fixés, un cycle saturé à 3-7 chaînons, contenant éventuellement un ou plusieurs hétéroatomes choisis parmi O, S ou N, et éventuellement encore substitué sur l'atome de N supplémentaire, ledit cycle est choisi du groupe constitué de aziridine, pyrrolidine, pipéridine, morpholine, thiomorpholine, azépine ou pipérazine éventuellement substituée sur l'atome de N supplémentaire par un alkyle en C₁ à C₆ éventuellement substitué par un halogène, un hydroxyle ou un amino.

4. Dérivé de chlorophylle ou de bactériochlorophylle selon la revendication 3 destiné à être utilisé selon la revendication 3, dans lequel R₆ représente -NR₉R'₉, dans lequel R₉ représente H et R'₉ représente un alkyle en C₁-C₁₀ substitué par au moins un groupe choisi parmi : un groupe chargé positivement, un groupe chargé négativement, un groupe acide qui est converti en un groupe chargé négativement dans des conditions physiologiques, de préférence SO₃H ou un sel alcalin de celui-ci, ou un groupe basique qui est converti en un groupe chargé positivement dans des conditions physiologiques, de préférence -NRR' ou -NH-(CH₂)₂₋₆-NRR', où chacun de R et R' représentent indépendamment H, un alkyle en C₁-C₆ éventuellement substitué par NH₂, ou R et R' forment ensemble avec l'atome de N un cycle saturé à 5-6 chaînons, contenant éventuellement un atome de O ou de N et éventuellement encore substitué sur l'atome de N supplémentaire par -(CH₂)₂-₆-NH₂.

5. Dérivé de chlorophylle ou de bactériochlorophylle selon la revendication 4 destiné à être utilisé selon la revendication 4, dans lequel le dérivé est une bactériochlorophylle de formule II, dans lequel :
M représente 2H, Mg, Pd, ou Zn, de préférence 2H ou Pd, plus préférablement Pd ;
R₁ est choisi parmi :
(i) -O R₁₀⁺ ;
(ii) Y-R₈ où Y représente O ou S et R₈ représente le résidu d'un acide aminé, d'un peptide ou d'une protéine ;
(iii) -NH-CH₂-CH(OH)-CH₂OH ;
(iv) -NH-(CH₂)ₙ-OH ;
(v) -NH-CH(OH)-CH₃;
(vi) -NH-(CH₂)ₙ-NR-(CH₂)ₙ-OH ;
(vii) glycosylamino ; ou
(viii) NHR'₉ qui est tel que défini pour R₆ ;
R'₂ représente un alcoxy en C₁-C₆ tel que méthoxy, éthoxy, propoxy ou butoxy, plus préférablement méthoxy ;
R₄ représente -C(=O)-CH₃, -CH=N-(CH₂)ₙ-SO₃⁻R₁₀⁺; -CH=N-(CH₂)ₙ-COO⁻R₁₀⁺; -CH=N-(CH₂)ₙ-PO₃²-(R₁₀⁺)₂ ; -CH₂-NH-(CH₂)ₙ-SO₃⁻R₁₀⁺; -NH-(CH₂)ₙ-COO⁻R₁₀⁺; -NH-(CH₂)ₙ-PO₃²⁻(R₁₀⁺)₂; ou -C(CH₃)=NR₉, de préférence C(CH₃)=N-(CH₂)ₙ-NH₂, ou -C(CH₃)=N-(CH₂)ₙ-N(R)₃⁺A⁻" ;
R₆ est choisi parmi (i) NHR'₉, choisi parmi :
(a) -NH-(CH₂)ₙ-SO₃⁻R₁₀⁺, de préférence -NH-(CH₂)₂-SO₃R₁₀⁺ ou -NH-(CH₂)₃-SO₃R₁₀⁺" ;
(b) -NH-(CH₂)ₙ-COO⁻R₁₀⁺ ;
(c) -NH-(CH₂)ₙ-PO₃²⁻(R₁₀⁺)₂ ;
(d) -NH-(CH₂)ₙ-B :
(e) 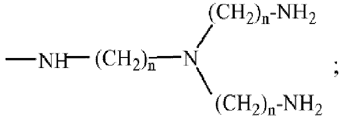
(f) 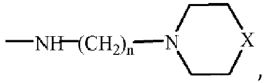 de préférence -NH-(CH₂)₂-1-morpholino ou -NH-(CH₂)₃-pipérazino ;
(g) 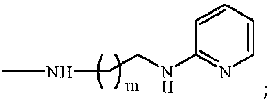
(h) -NH-(CH₂)ₙ-N(R")-(CH₂)ₙ-NRR', de préférence -NH-(CH₂)₃-NH-(CH₂)₃-NH₂ ;
(j) -NH-(CH₂)ₙ-NRR', de préférence -NH-CH₂-CH₂-NRR' ;
R₁₀⁺ représente H⁺, ou un cation monovalent choisi parmi K⁺, Na⁺, Li⁺, ou NH₄⁺, de préférence K⁺ ;
X représente O, S ou NH ;
B représente un groupe chargé positivement choisi parmi les groupes ammonium -N⁺RR'R", de préférence -N(CH₃)₃⁺A⁻, guanidinium, sulfonium, phosphonium, arsonium ; ou B représente un groupe basique qui est converti en un groupe chargé positivement dans des conditions physiologiques, choisi parmi des groupes amino--NRR', de préférence -NH₂, guanidino, phosphino, ou arsino ;
m est égal à 1, n est un nombre entier de 1 à 10, de préférence 2 ou 3 ; et
A⁻ est un anion physiologiquement acceptable ;
où R, R' et R" sont chacun indépendamment H ou un alkyle en C₁-C₆.

6. Dérivé de chlorophylle ou de bactériochlorophylle selon la revendication 5 destiné à être utilisé selon la revendication 5, dans lequel le dérivé est une bactériochlorophylle de formule II choisie parmi les sels pharmaceutiquement acceptables avec un cation de métal alcalin ou alcalino-terreux monovalent ou divalent, ou avec NH₄⁺, des composés suivants :
Palladium 3¹-oxo-15-méthoxycarbonylméthyl-rhodobactériochlore 13¹-(2-sulfoéthyl) amide ;
Palladium 3¹-oxo-15-méthoxycarbomylméthyl-rhodobactériochlore 13¹-(-sulfopropyl)amide ;
Palladium 3¹-oxo-15-méthoxycarbomylméthyl-rhodobactériochlore 13¹,17³-di-(2-sulfoéthyl)amide ;
Palladium 3¹-oxo-15-méthoxycarbomylméthyl-rhodobactériochlore 13¹,17³-di-(3-sulfopropyl)amide ;
3¹-oxo-15-méthoxycarbonylméthyl-rhodobactériochlore 13¹-(2-sulfoéthyl) amide ;
3¹-oxo-15-méthoxycarbomylméthyl-rhodobactériochlore 13¹-(3-sulfopropyl) amide ;
3¹-oxo-15-méthoxycarbomylméthyl-rhodobactériochlore 13¹,17³-di-(2-sulfoéthyl) amide ; et
3¹-oxo-15-methoxycarbomylmethyl-rhodobactériochlore 13¹,17³-di-(3-sulfopropyl) amide.

7. Dérivé de chlorophylle ou de bactériochlorophylle selon la revendication 6, destiné à être utilisé selon la revendication 6, choisi parmi les sels consistant de :
sel dipotassique de palladium 3¹-oxo-15-méthoxycarbonylméthyl-rhodobactériochlore 13¹-(2-sulfoéthyl)amide (ici WST11) ;
sel dipotassique de Palladium 3¹-oxo-15-méthoxycarbomylméthyl-rhodobactériochlore 13¹-(3-sulfopropyl)amide (ici composé 1) ;
sel dipotassique de Palladium 3¹-oxo-15-méthoxycarbomylméthyl-rhodobactériochlore 13¹,17³-di-(2-sulfoéthyl)amide (ci-après le composé **2**) ; et
sel dipotassique de Palladium 3¹-oxo-15-méthoxycarbomylméthyl-rhodobactériochlore 13¹,17³-di-(3-sulfopropyl)amide (ici composé **3**)**.**
sel dipotassique de 3¹-oxo-15-méthoxycarbonylméthyl-rhodobactériochlore 13¹-(2-sulfoéthyl) amide ;
sel dipotassique de 3¹-oxo-15-méthoxycarbomylméthyl-rhodobactériochlore 13¹-(3-sulfopropyl) amide ;
sel dipotassique de 3¹-oxo-15-méthoxycarbomylméthyl-rhodobactériochlore 13¹,17³-di-(2-sulfoéthyl)amide ; et
sel dipotassique de 3¹-oxo-15-methoxycarbonylmethyl-rhodobactériochlore 13¹,17³-di-(3-sulfopropyl)amide.

8. Dérivé de chlorophylle ou de bactériochlorophylle selon la revendication 7 destiné à être utilisé selon la revendication 7, dans lequel ledit dérivé de bactériochlorophylle est le sel dipotassique de Palladium 3¹-oxo-15-méthoxycarbonylméthyl-rhodobactériochlore 13¹-(2-sulfoéthyl)amide (ici WST11).

9. Composition pharmaceutique comprenant un support pharmaceutiquement acceptable et un dérivé de chlorophylle ou de bactériochlorophylle selon l'une quelconque des revendications 1 à 8, destinée à être utilisée dans le traitement de maladies, de troubles et d'états associés à une anomalie cornéenne ou sclérale, choisis parmi l'amincissement de la cornée et l'affaiblissement de la sclère par thérapie photodynamique (PDT).

10. Composition pharmaceutique selon la revendication 9 destinée à être utilisée selon la revendication 9, comprenant en outre un agent visqueux pour augmenter la viscosité de la formulation et restreindre la pénétration du dérivé de chlorophylle ou de bactériochlorophylle dans la cornée.

11. Composition pharmaceutique selon la revendication 10 destinée à être utilisée selon la revendication 10, dans laquelle ledit agent visqueux est un polymère, de préférence un biopolymère choisi parmi le dextrane, le scléroglucane et ses dérivés, la gomme Gellan, la gomme Guar ou la méthylcellulose.

12. Composition pharmaceutique selon la revendication 11 destinée à être utilisée selon la revendication 11, comprenant en outre un agent améliorant la perméabilité transepithéliale pour améliorer la pénétration du dérivé de chlorophylle ou de bactériochlorophylle à travers l'épithélium.

13. Composition pharmaceutique selon l'une quelconque des revendications 9 à 12 destinée à être utilisée selon l'une quelconque des revendications 9 à 12 destinée à être utilisée en application topique.

14. Composition pharmaceutique selon l'une quelconque des revendications 9 à 13 destinée à être utilisée selon l'une quelconque des revendications 9 à 13, destinée à être utilisée dans la thérapie photodynamique avant l'irradiation locale de l'œil avec une lumière à une longueur d'onde rouge ou proche infrarouge (NIR).

15. Dérivé de chlorophylle ou de bactériochlorophylle selon l'une quelconque des revendications 1 à 8 destinée à être utilisée selon l'une quelconque des revendications 1 à 8 ou composition pharmaceutique selon l'une quelconque des revendications 9 à 14 destinée à être utilisée selon l'une quelconque des revendications 9 à 14, dans lequel les maladies, troubles et états associés à l'amincissement de la cornée et à l'affaiblissement de la sclère sont choisis parmi le kératocône, ectasie post-laser assistée in situ keratomileusis (LASIK), ectasie kératectomie post-photoréfractive (PRK), ectasie post-infection, ectasie périphérique, état rhumatoïde de la cornée, myopie dégénérative, myopie régulière, staphylocoque scléral, glaucome de l'hypertension oculaire, glaucome à basse tension et combinaisons de ceux-ci.

16. Dérivé de chlorophylle ou de bactériochlorophylle selon la revendication 8 destinée à être utilisée selon la revendication 8 ou composition pharmaceutique selon l'une quelconque des revendications 9 à 15 destinée à être utilisée selon l'une quelconque des revendications 9 à 15, dans lequel la maladie, le trouble et l'état associés à l'amincissement de la cornée est le kératocône.
